# EUROPEAN PATENT APPLICATION

(11) **EP 2 108 702 A1**
(43) Date of publication of application: **14.10.2009**
(21) Application number: 08006915.6
(22) Date of filing: 07.04.2008
(51) Int. Cl.: C12P 7/64, C12N 1/18, A23L 1/30, A23K 1/16

(54) **Synthesis of polyunsaturated fatty acids in yeast**

(71) Applicant: Organo Balance GmbH, 13355 Berlin (DE)
(72) Inventor: The designation of the inventor has not yet been filed
(74) Representative: Vossius & Partner

(57) **Abstract**

Described is a method of providing an organism belonging to the genus Saccharomyces, which is capable of producing polyunsaturated fatty acids (PUFAs) comprising the steps of subjecting such an organism, which does not produce PUFAs, to a mutagenesis step and selecting a Saccharomyces organism, which is capable of accumulating at least 15% of the cellular dry weight as lipids; and genetically modifying the Saccharomyces organism selected in step (a) so that it is capable of synthesizing PUFAs, in particular eicosapentaenoic acid and/or docosahexaenoic acid. Also described are organisms obtained by such a method, as well as a method for the synthesis of PUFAs, in particular eicosapentaenoic acid and/or docosahexaenoic acid comprising culturing such an organism.

SEQUENCE LISTING

<110>  OrganoBalance GmbH

<120>  Synthesis of polyunsaturated fatty acids in yeast

<130>  P1022 EP S3

<160>  20

<170>  PatentIn version 3.3

<210>  1
<211>  1263
<212>  DNA
<213>  Pichia pastoris

<400>  1
atgtctgccg tcacagttac aggtaataat ggtgatgctt caagaagtaa caccaccacc      60

accacaaaac gtacaggcaa tgtttcctcc ttcagccaat ccaaaggttt gactgccata     120

gacacctggg gtaacgtctt caaagtccct gattttacaa tcaagcaaat cttggatgct     180

attccaaagc actgctatga acgcaggcta accacgtcgt tctactacgt gttcagggac     240

atattcctca ttggttgtac catgttcatg gggtcgttca ttcccatgat tgagaatgtt     300

ttccttagag gcgctgctta cgccgctttg gtttttctct tatctgttga gtacactggt     360

ttgtgggttt tggcccacga gtgcggtcat caagctttct ccgattatgg ttgggtcaac     420

gacaccgtgg gatggatttt gcattcttac ctgttagtcc catatttttc ttggaaatac     480

agtcatggta aacatcacaa ggctactggc cacttgacta gagacatggt gtttgtacct     540

gccacaaagg agaagttctt ggaaaagaga aacgccagca aacttggcga actgggagaa     600

gatgctccca ttttcacatt atatcagttg gtagcccaac aattgggagg ctggattttg     660

tatttgttca ccaacgttac tggtcaaccc taccccaaca cccctaaatg gatgcagaac     720

cactttgttc cctcatctcc aattttcgaa aaaaaggact actggtttat cattctgagt     780

gacctgggta tcttggcaca gttgatggtt ttgtatgtgt ggagacaaca aatgggaaac     840

tggaacttat ttatttactg gttcctgcct tatgttctca ccaaccattg gctggtgttc     900

atcacattcc tgcaacactc tgatccaacg atgcctcact acgaggctga acaatggacg     960

tttgccagag gagctgccgc aaccatcgac cgtgaatttg gattcattgg acctttcttc    1020

tttcacgata tcatcgaaac tcacgtcttg catcactatg tgagtagaat tcctttctac    1080

aacgcccgag aggccagtga aggtattaag aaagttatgg gggagcatta tcgctacagt    1140

ggtgaaaaca tgtgggtctc tctttggaag agtggacgtt catgtcagtt tgttgatgga    1200

gaaaacgggg tgaaaatgta ccgtaacatc aataactggg gtattggaac cggtgagaaa    1260

tag                                                                 1263

<210>  2
<211>  1263
<212>  DNA
<213>  Artificial sequence

<220>
<221>  source
<223>  /note="description of artificial sequence: Pichia pastoris"

<400>  2
atgtcagctg ttacagtcac aggcaacaat ggtgatgcta gtaggagtaa tactacaacc      60

actactaaaa gaaccggtaa tgtgagttcc ttttcacaat cgaagggatt aactgccata     120

gacacttggg ggaatgtttt taaagttcca gatttcacga ttaagcagat actagatgca     180

attcccaaac actgctatga gagaaggttg actacgtcat tctactatgt gtttcgagac     240

atctttttga ttggctgtac aatgtttatg gggtctttca taccaatgat tgaaaacgtg      300

tttttgagag gtgcagcgta tgccgctctt gttttccttt tatcagtcga gtacactggt     360

ttgtgggtat tagcccatga atgtggtcat caagctttca gcgactatgg ttgggtcaat     420

gatacagttg gttggattct gcattcctac ttgttagtac cctatttttc ctggaaatac     480

tctcatggta aacatcacaa agcaactggg catttgacaa gggatatggt ttttgttcct     540

gctacaaagg agaagttttt agagaagaga aatgccagca aactaggaga gttaggagaa     600

gatgctccta tctttactct gtatcagcta gttgcgcaac agttaggtgg ctggattctc     660

tacctgttta ccaacgtaac tggtcaacct taccctaata ccccgaaatg gatgcagaac     720

cactttgtac catcatctcc gatatttgag aagaaagact attggttcat catattgtct     780

gacttgggaa ttcttgctca actcatggtc ttatacgtat ggaggcaaca aatgggcaat     840

tggaacttat tcatctactg gtttctgcca tatgtcttga ccaatcattg gttggtgttc     900

attacgtttc tacaacattc ggatcctaca atgccacact atgaagcgga acaatggaca     960

tttgctagag gtgcagcagc tacgattgat cgtgaattcg ggttcattgg tccatttttc    1020

ttccatgata tcatcgaaac ccatgttctt catcactatg tatccagaat tccattctat    1080

aatgccagag aagcatctga aggcatcaag aaagtgatgg gtgaacacta ccgttattct    1140

ggagaaaaca tgtgggtttc tctatggaaa agtggaagaa gctgtcaatt tgtcgatggt    1200

gaaaatggcg ttaagatgta ccgcaacata aacaattggg gaataggtac cggtgaaaaa    1260

taa                                                                 1263

<210>  3
<211>  1371

<212>  DNA
<213>  Ostreococcus tauri

<400>  3
atgtgcgtgg agacggaaaa taacgatggg atccccacgg tggagatcgc gttcgacggt      60

gagcgcgagc gggcggaggc aaacgtgaag ctgtccgcgg agaagatgga gccggcggcg     120

ctggcgaaga cgttcgcgag gcggtacgtc gtgatcgagg gggtggagta cgatgtgacg     180

gattttaagc acccgggagg aacggttatt ttctatgcgt tgtcaaacac cggggcggac     240

gcgacggaag cgttcaagga gtttcatcat cggtcgagaa aggcgaggaa agccttggcg     300

gcgctcccgt ctcgaccggc caagacggcc aaggtggacg acgcggagat gctccaagat     360

ttcgccaagt ggcggaaaga attggagaga gatggattct tcaagccctc tccggcgcac     420

gtggcgtatc gcttcgccga gctcgcggcg atgtacgctc tcgggacgta cctgatgtac     480

gctcgatacg tcgtctcctc ggtgctcgtg tacgcttgct ttttcggcgc ccgatgcggt      540

tgggtgcagc acgagggcgg acacagctcg ctgacgggca acatttggtg ggacaagcgc     600

atccaggcct tcacagccgg gttcggtctc gccggtagcg gcgacatgtg gaactcgatg     660

cacaacaagc atcacgcgac gcctcaaaag gttcgtcacg acatggatct ggacaccacc     720

cccgcggtgg cgttcttcaa caccgcggtg gaagacaatc gtccccgtgg ctttagcaag     780

tactggttgc gccttcaggc gtggaccttc atccccgtga cgtccggctt ggtgctcctt     840

ttctggatgt ttttcctcca cccctccaag gctttgaagg gtggcaagta cgaagagttg      900

gtgtggatgc tcgccgcgca cgtcatccgc acgtggacga tcaaggcggt gaccggattc     960

accgcgatgc agtcctacgg cttatttttg gcgacgagct gggtgagcgg ctgctatctg    1020

tttgcacact tctccacgtc gcacacgcac ctggatgtgg tgcccgcgga cgagcatctc    1080

tcctgggttc gatacgccgt cgatcacacg atcgacatcg atccgagtca aggttgggtg    1140

aactggttga tgggctacct caactgccaa gtcatccacc acctctttcc gagcatgccg    1200

cagttccgcc agcccgaggt atctcgccgc ttcgtcgcct ttgcgaaaaa gtggaacctc    1260

aactacaagg tcatgaccta cgccggtgcg tggaaggcaa cgctcggaaa cctcgacaac    1320

gtgggtaagc actactacgt gcacggccaa cactccggaa agacggcgta a             1371

<210>  4
<211>  1371
<212>  DNA
<213>  Artificial sequence

<220>
<221>  source
<223>  /note="description of artificial sequence: Ostreococcus tauri"

<400> 4
atgtgtgttg aaacagagaa caatgatggc attccaaccg tagaaatagc gtttgatggt 60

gagagagaaa gggctgaagc caacgtcaaa ctaagtgctg agaaaatgga acctgctgcg 120

ttagcaaaga cctttgctag acgttacgtg gtgattgaag gagttgagta tgatgtcacg 180

gacttcaaac atccaggtgg tacagtaatc ttctacgcct tgagtaatac tggtgctgat 240

gccacagaag cattcaagga attccaccat aggagcagaa aggcaagaaa ggctttggca 300

gctttgccct ctagaccagc taaaactgct aaagtggacg atgcagagat gctgcaagac 360

tttgccaaat ggagaaagga actggaaaga gatgggttct ttaagccttc acctgcccat 420

gttgcctacc gttttgctga attagctgca atgtatgcac taggtacata cctcatgtat 480

gcaaggtatg ttgtatcttc cgttttggtg tatgcgtgct tctttggggc aagatgtggt 540

tgggttcaac atgaaggtgg acactcatcc ctgacaggga atatatggtg ggataagagg 600

attcaggcct ttactgccgg ttttggatta gctggtagtg gtgatatgtg gaatagcatg 660

cacaataaac accatgctac tcctcaaaaa gtacgacatg acatggacct tgatactaca 720

ccagctgtcg ccttctttaa cacggcagtc gaggataatc gacctagagg cttttcgaaa 780

tattggctga gattgcaagc gtggacgttc attcccgtta ctagtggctt ggtattgttg 840

ttttggatgt tcttcttaca cccatctaaa gcacttaaag gtggcaagta tgaagaacta 900

gtgtggatgt tagcggctca tgttatcaga acctggacga ttaaggcagt tacaggcttc 960

actgcaatgc agtcttatgg gttgtttctt gccacttctt gggtatctgg ttgctacctt 1020

ttcgctcatt tttccacttc acatacacac ctagacgtag ttccagctga tgagcattta 1080

tcgtgggttc gctatgctgt cgatcatacc atcgatattg atccctccca aggttgggtc 1140

aactggttaa tgggttatct caattgtcag gtgatacatc acttatttcc gtcaatgccg 1200

caatttcgtc aaccagaagt tagcagaagg tttgttgcgt ttgccaagaa gtggaatttg 1260

aactacaaag tcatgaccta tgcaggagca tggaaagcca ctttaggaaa cctagacaat 1320

gtgggaaaac actactacgt tcatggacaa cattcaggca aaaccgctta a 1371

<210> 5
<211> 885
<212> DNA
<213> Scophthalmus maximus

<400> 5
atggaaacat tcaatcataa actgaacact tacatagact catggatggg tcccagagat 60

cagcgggtga ggggatggct gctgttggac aactacccac caacctttgc actcacagtc 120

atgtaccttc tgattgtgtg gatgggaccc aagtacatga aaaaccgaca gccgtactcc 180

tgcagaggcc tcctggtggt ctacaatctg ggcctcacgc tcctgtcttt ctacatgttc 240

tatgagcttc ttactgctgt gtggcatggc gactacaact tctactgcca ggacactcac 300

agtgtaccgg aagtcgacaa taagatcata aacgccctat ggtggtacta cttctccaag 360

ctcatcgagt tcatggacac gtttttcttc atcctacgga agaactttca tcagatcacg 420

ttcctccaca tttaccacca cgctagcatg ctgaatatct ggtggttcgt tatgaactcg 480

ataccatgcg gccactcgta cttcggcgca tccctcaaca gcttcgtcca cgtcgcgatg 540

tattcctact atggcctgtc agccatccca gccatccggc cgtacctctg gtggaagaga 600

tacatcacac agttacagct gatccaattc tttttaacca tgtcccagac aatgtgtgcg 660

gtcatatggc catgtgattt ccccagggga tggctgtact tccaaataag ttacgtggtc 720

acgctgatta tccttttctc aaacttctac attcagacgt acaagaagca caatgccact 780

ctgcagaagc agcacccgaa tggctctgct gtgtcaagaa atggacattc gaatggaaca 840

ccgtccgcgg agcacatggc gcacaagaag ctgagggtgg attga 885

<210> 6
<211> 885
<212> DNA
<213> Artificial sequence

<220>
<221> source
<223> /note="description of artificial sequence: Scophthalmus maximus"

<400> 6
atggaaactt tcaatcacaa actaaatacc tatatcgatt cttggatggg tcctagagat 60

caaagggtaa gaggctggct tttactggac aattatcctc caacatttgc cttaactgta 120

atgtacttgc tcatagtttg gatgggaccg aagtatatga aaaacagaca accgtattct 180

tgcagagggc ttttagtcgt ctataaccta ggtttgacat tgttgtcatt ttacatgttt 240

tacgagttac taactgctgt ttggcatgga gactataact tttattgcca agatactcac 300

tctgttccag aagttgacaa taagatcatt aatgcgcttt ggtggtatta cttctccaaa 360

ttgatagaat tcatggatac attcttcttc attctgagga aaatttccca ccaaatcact 420

tttctgcaca tttaccacca tgcttctatg ctcaacatat ggtggtttgt gatgaacagt 480

attccatgtg gtcattccta ttttggtgca tcgttaaata gctttgtcca tgttgctatg 540

tattcctact atggcctaag tgctattcct gccattagac cctacttatg gtggaaacgc 600

tatattactc agttgcaatt gatacagttc tttttgacaa tgagtcaaac gatgtgtgct 660

gttatttggc catgtgattt tcctagagga tggttatact tccagatttc gtatgtggtg 720

acgttaatca tcctttttag caacttttac atacaaacct acaagaagca taatgcaacc 780

ttacagaaac aacatcccaa tggttctgca gtatcacgaa atgggcattc aaacggtaca 840

ccatcagcag aacatatggc ccataaaaag ttgcgtgtag attaa 885

<210> 7
<211> 1248
<212> DNA
<213> Pichia pastoris

<400> 7
atgtcaaaag tcactgtttc gggttcagag atcctagaag ggtccaccaa gaccgtcaga 60

cgttcaggaa acgttgcttc tttcaagcaa cagaaaactg ccatcgatac gtttggtaat 120

gttttcaaag tgccagatta caccatcaaa gatatcctag acgccattcc aaaacactgc 180

tatgaaagat ctttggtcaa gtcgatgtcc tacgtcgtca gggatattgt tgctatttct 240

gctattgcct acgtgggcct aacttacatc cctctgctgc caaacgagtt tcttcgtttt 300

gctgcttgga gtgcgtatgt gttcagtatt tcttgttttg gattcggaat ttggattctt 360

ggacacgaat gcggtcactc tgccttcagc aactatggct gggttaacga cacggtcggc 420

tgggttcttc attccctggt aatggttcca tatttttctt ggaagttctc ccatgccaag 480

catcataagg caacaggtca tatgaccaga gacatggtct ttgttccata caccgctgag 540

gagttcaagg aaaaacacca agttactagt ctgcacgata ttgcagagga aactcctatt 600

tattcagttt ttgccctttt gtttcaacag ctcggaggac tcagtttgta tcttgccacg 660

aatgctactg gtcaaccgta tcccggggtg tccaagttct tcagaagtca ttactggcca 720

tcctctcctg tttttgataa gaaggactat tggtacattg ttctgagtga cctgggaatc 780

ttggcaaccc tgactagtgt ttacactgct tacaaagtgt tcggattttg gcccacattc 840

attacttggt tttgcccttg gattttggtc aaccactggc tggtatttgt caccttccta 900

caacacacag actcgtccat gcctcactac gatgcccaag aatggacttt cgccaaaggt 960

gccgccgcca caattgatag agaatttggt atcttaggaa ttatatttca cgacattatc 1020

gaaacccacg ttttgcacca ctatgttagt agaattccat tctaccatgc tagagaggct 1080

actgagtgca ttaagaaggt tatgggcgaa cattaccgtc acactgacga gaacatgtgg 1140

gtcagtctct ggaaaacctg gaggtcgtgc cagtttgttg agaaccatga tggtgtgtac 1200

atgttcagaa actgcaacaa tgttggtgtt aaacctaagg atacctaa 1248

<210> 8
<211> 1248
<212> DNA
<213> Artificial sequence

<220>
<221> source
<223> /note="description of artificial sequence: Pichia Pastoris"

<400> 8
atgagcaaag tcacagtgag tggtagcgaa attctcgaag gttcaaccaa aacggttcgt 60

agatcaggta atgtagcttc ctttaaacag caaaaaacgg ctatagacac atttggcaat 120

gtctttaagg ttccggatta tacgataaag gacattctcg atgcaatacc taaacactgt 180

tacgaaaggt ctctagtcaa gtcgatgtca tacgttgttc gagatatcgt tgccatttct 240

gcgattgcct atgtcggttt gacatacatc ccattgctac ctaatgagtt tttgcgtttt 300

gcagcttggt ctgcttatgt gttttccata tcttgcttcg gatttgggat ttggattcta 360

gggcatgaat gtggtcattc cgcattttcg aactatggtt gggtaaatga cactgttgga 420

tgggttcttc acagcttggt aatggtgcca tatttttcat ggaagttctc tcacgccaaa 480

catcacaaag ctaccggtca tatgactaga gatatggtct ttgttcctta cacagcagaa 540

gaattcaagg agaaacatca ggttacttca ttacatgaca tagcggaaga aactccgata 600

tactccgttt ttgcattgct gtttcagcaa ttaggaggtt taagtctgta tcttgctacc 660

aatgccactg gccaaccata tcctggtgtc tcaaagttct ttcgctctca ttattggcca 720

agttcaccag ttttcgacaa gaaagactac tggtacattg tcttgtctga tttgggaata 780

cttgctacct taacaagcgt atatacagcg tacaaagtat tcggcttttg gccaacgttt 840

atcacctggt tttgtccctg gattctggtg aatcattggt tagtgttcgt gaccttccta 900

caacatactg attctagtat gcctcattat gatgcacaag aatggacttt tgccaaagga 960

gctgctgcaa ctatcgatag agaatttggc attttgggca ttattttcca cgatatcatc 1020

gagacacatg tgttacacca ctatgtatcg agaattccct tctaccatgc cagagaagct 1080

acagagtgca tcaagaaggt aatgggtgaa cactacagac atactgatga gaacatgtgg 1140

gtatccttat ggaaaacttg gagaagttgt caattcgtcg aaaaccatga tggggtttat 1200

atgttcagga actgcaacaa tgttggtgtt aaaccaaagg atacataa 1248

<210> 9
<211> 1308
<212> DNA
<213> Caenorhabditis elegans

<400> 9
atggcaaaat acgactacaa tccgaagtat gggttagaaa attacagcat attccttccc 60

tttgagacat cttttgatgc atttcgatcg acaacatgga tgcaaaatca ctggtatcaa 120

tcaattacag catctgtcgt gtatgtagcc gtcattttta caggaaagaa ggtggttctc 180

atctacaaaa aatcacgagt tattactttt gagtctagcc ttcagaatgc aattaagaat 240

cgaaaccgaa aatcacttaa tagttctcaa atgtttcaga ttatggaaaa gtacaagccc 300

ttccaactgg acacaccact cttcgtctgg aattcatttt tagccatttt ctcaattctc 360

gggttcctcc gaatgacacc tgaatttgta tggagttggt cagcagaagg aaactcattc 420

aaatattcaa tttgtcattc atcttatgct caaggagtca ctggtttctg gactgaacaa 480

ttcgcaatga gcaaactttt cgagctcatc gacacaatct tcatcgttct tcgtaaacgt 540

ccactcatct tccttcactg gtatcatcat gtaactgtta tgatctacac atggcacgcg 600

tacaaggatc acactgcatc aggacggtgg ttcatttgga tgaattatgg agttcatgct 660

cttatgtatt cctactatgc tcttcgttct ctgaaattcc gtcttccaaa acaaatggca 720

atggttgtta ctactctcca acttgctcaa atggttatgg gagtaatcat cggagtcact 780

gtctaccgta tcaagtcatc gggtgaatac tgccaacaga catgggacaa tttgggatta 840

tgctttggag tttatttcac atatttcctt cttttcgcca acttcttcta ccatgcatat 900

gttaagaaaa acaaccgtac agtaaattat gaaaataatt caaaaaattt ccccgatctc 960

gttttaattt acctgagaaa aaaggtttca agaaaatcga aaaatcggca atgttcagaa 1020

aataattata aaattcaatt ttcatcaaat tttgttaatg ttgatggaaa aaaacataag 1080

aaaacatatg aacttattct tccaagaaga aaaatgacca caattttaac ttttctattt 1140

ggaaaaaatc gaattttttc gaaatatcag aaaaatcgaa aaaacatttc gattcctgtt 1200

gatttcgaaa ttctggagcc aaaagaagat atcaatgcta acatcgctga gccatccatc 1260

acaacgaggt ccgccgccgc acgaagaaaa gttcaaaaag ctgattag 1308

<210> 10
<211> 1308
<212> DNA
<213> Artificial sequence

<220>
<221> source
<223> /note="description of artificial sequence: Caenorhabditis elegans"

<400> 10
atggcaaagt atgactacaa tcccaaatac ggattggaga attacagcat tttctccct 60

ttcgaaacca gttttgatgc atttcgtagc actacatgga tgcaaaatca ctggtatcaa 120

tctattacag cttctgtagt ctatgttgca gtgatcttta ctggtaaaaa ggtcgtattg 180

atctataaaa agtccagagt tattacgttt gaatccagtt tgcaaaatgc cattaagaac 240

agaaacagga agtccctaaa ttcctctcag atgtttcaga ttatggaaaa atacaaaccc 300

tttcaactag atactccgtt gttcgtttgg aatagctttt tggcgatttt ttccattcta 360

ggttttctca gaatgactcc tgaatttgtg tggtcatggt ctgctgaagg caatagtttc 420

aagtactcaa tttgtcattc atcatatgca caaggtgtaa caggcttttg gactgaacag 480

ttcgcgatgt caaagttatt tgagttaatt gacaccatat tcatcgtttt gagaaaaaga 540

ccactgatct tcttacattg gtatcatcac gttactgtga tgatctatac atggcatgct 600

tataaggacc atacagcaag tggtagatgg ttcatatgga tgaattacgg ggttcatgcc 660

ttgatgtatt cctattacgc tttaaggtct ttgaaattca ggttaccaaa gcaaatggcc 720

atggttgtta ctacccttca attagcccag atggttatgg gtgtcatcat aggagtaaca 780

gtgtatcgca taaaatcgtc tggagaatat tgccaacaaa cttgggataa cttaggtctg 840

tgttttggtg tgtatttcac ctattttctg ctatttgcca atttcttcta ccacgcttat 900

gtcaagaaga acaatagaac cgtaaactac gagaataatt cgaaaaactt tcctgatctt 960

gtgcttatat acctaaggaa gaaagtcagt agaaaatcga aaaaccgaca atgttctgaa 1020

aataactaca agattcagtt cagttcaaac tttgtaaatg ttgatgggaa aaaacacaag 1080

aaaacatatg aactgatctt acctagacgt aaaatgacga cgattttgac cttccttttt 1140

ggcaaaaata ggattttcag caagtaccaa aagaatcgaa aaaacatttc tataccagtt 1200

gattttgaga tattagagcc aaaagaagac atcaacgcaa atatagctga accatcaatt 1260

acaactcgtt ctgctgctgc tagaagaaaa gtccaaaaag cggattaa 1308

<210> 11
<211> 1341
<212> DNA
<213> Mortierella alpina

<400> 11
atgggaacgg accaaggaaa aaccttcacc tgggaagagc tggcggccca taacaccaag 60

gacgacctac tcttggccat ccgcggcagg gtgtacgatg tcacaaagtt cttgagccgc 120

catcctggtg gagtggacac tctcctgctc ggagctggcc gagatgttac tccggtcttt 180

gagatgtatc acgcgtttgg ggctgcagat gccattatga agaagtacta tgtcggtaca 240

ctggtctcga atgagctgcc catcttcccg gagccaacgg tgttccacaa aaccatcaag 300

acgagagtcg agggctactt tacggatcgg aacattgatc ccaagaatag accagagatc 360

tggggacgat acgctcttat ctttggatcc ttgatcgctt cctactacgc gcagctcttt 420

gtgcctttcg ttgtcgaacg cacatggctt caggtggtgt ttgcaatcat catgggattt 480

gcgtgcgcac aagtcggact caaccctctt catgatgcgt ctcacttttc agtgacccac 540

aaccccactg tctggaagat tctgggagcc acgcacgact ttttcaacgg agcatcgtac 600

ctggtgtgga tgtaccaaca tatgctcggc catcacccct acaccaacat tgctggagca 660

gatcccgacg tgtcgacgtc tgagcccgat gttcgtcgta tcaagcccaa ccaaaagtgg 720

tttgtcaacc acatcaacca gcacatgttt gttcctttcc tgtacggact gctggcgttc 780

aaggtgcgca ttcaggacat caacattttg tactttgtca agaccaatga cgctattcgt 840

gtcaatccca tctcgacatg gcacactgtg atgttctggg gcggcaaggc tttctttgtc 900

tggtatcgcc tgattgttcc cctgcagtat ctgcccctgg gcaaggtgct gctcttgttc 960

acggtcgcgg acatggtgtc gtcttactgg ctggcgctga ccttccaggc gaaccacgtt 1020

gttgaggaag ttcagtggcc gttgcctgac gagaacggga tcatccaaaa ggactgggca 1080

gctatgcagg tcgagactac gcaggattac gcacacgatt cgcacctctg gaccagcatc 1140

actggcagct tgaactacca ggctgtgcac catctgttcc ccaacgtgtc gcagcaccat 1200

tatcccgata ttctggccat catcaagaac acctgcagcg agtacaaggt tccatacctt 1260

gtcaaggata cgttttggca agcatttgct tcacatttgg agcacttgcg tgttcttgga 1320

ctccgtccca aggaagagta g 1341

<210> 12
<211> 1341
<212> DNA
<213> Artificial sequence

<220>
<221> source
<223> /note="description of artificial sequence: Mortierella alpina"

<400> 12
atgggcacag atcaaggaaa aacctttaca tgggaagaat tagcagctca taacactaaa 60

gacgatctct tgttagccat aaggggtaga gtctatgatg tcactaagtt cttaagtaga 120

catcctggtg gcgttgatac gttattgcta ggagcaggaa gagacgttac accagtcttt 180

gagatgtatc acgcttttgg tgcagccgat gcaatcatga agaagtacta tgtaggcaca 240

ctagtttcca atgagttgcc aatttttccg gaaccaactg tattccacaa gactataaaa 300

acacgtgtag aaggatactt tacagaccgc aatattgatc cgaaaaatag gcctgaaatt 360

tggggtagat acgccttaat cttcggatcg ttgatagcct catactacgc tcaactcttt 420

gtgccttttg tcgtggaaag aacctggtta caggtggttt tcgccataat catgggtttt 480

gcttgtgctc aagtaggtct aaatccattg catgatgcgt cacacttctc tgtaacccat 540

aaccccacgg tttggaaaat tcttggggca actcatgact tctttaacgg tgccagctat 600

ttagtctgga tgtatcagca catgcttggt catcatccat ataccaacat tgccggtgct 660

gatcctgatg tatctacgtc tgaaccagac gtaagaagga tcaaacccaa tcaaaagtgg 720

ttcgttaacc acataaatca acacatgttc gttccatttt tgtatggctt acttgcattc 780

aaggttcgta tccaagatat caacatactg tatttcgtga aaacgaatga cgctattagg 840

gtgaatccaa tttccacttg gcatacagtt atgttttggg gaggtaaagc gttttttgtc 900

tggtatcgat tgattgtacc tttgcagtat ctgcctttgg gtaaggtgtt actgttgttt 960

acagtggcag atatggtctc ttcctactgg ttggctttaa ccttccaagc gaatcatgtt 1020

gttgaagaag ttcaatggcc attaccagat gaaaatggca tcattcagaa agattgggct 1080

gctatgcaag ttgagactac tcaagattac gcacatgact cacatctatg gacaagcata 1140

actgggagtc taaactatca agctgttcat catctgtttc cgaatgttag tcagcatcac 1200

tatcccgata ttttggcgat tattaagaac acttgctcgg agtacaaagt gccttactta 1260

gtcaaagaca ccttttggca agcattcgct tcacatctag aacacctgag agttcttggt 1320

cttagaccca aagaagagta a 1341

<210> 13
<211> 1560
<212> DNA
<213> Thraustochytrium sp.

<400> 13
atgacggtcg gctacgacga ggagatcccg ttcgagcagg tccgcgcgca caacaagccg 60

gatgacgcct ggtgcgcgat ccacgggcac gtgtacgatg tgaccaagtt cgcgagcgtg 120

cacccgggcg gcgacattat cctgctggcc gcaggcaagg aggccaccgt gctgtacgag 180

acttaccatg tgcggggcgt ctcggacgcg gtgctgcgca agtaccgcat cggcaagctg 240

ccggacggcc aaggcggcgc gaacgagaag gaaaagcgga cgctctcggg cctctcgtcg 300

gcctcgtact acacgtggaa cagcgacttt tacagggtaa tgcgcgagcg cgtcgtggct 360

cggctcaagg agcgcggcaa ggcccgccgc ggaggctacg agctctggat caaggcgttc 420

ctgctgctcg tcggcttctg gagctcgctg tactggatgt gcacgctgga cccctcgttc 480

ggggccatcc tggccgccat gtcgctgggc gtctttgccg cctttgtggg cacgtgcatc 540

cagcacgacg gcaaccacgg cgcctttgcc cagtcgcgat gggtcaacaa ggttgccggg 600

tggacgctcg acatgatcgg cgccagcggc atgacgtggg agttccagca cgtcctgggc 660

caccatccgt acacgaacct gatcgaggag gagaacggcc tgcaaaaggt gagcggcaag 720

aagatggaca ccaagctggc cgaccaggag agcgatccgg acgtcttttc cacgtacccg 780

atgatgcgcc tgcacccgtg gcaccagaag cgctggtacc accgtttcca gcacatttac 840

ggccccttca tctttggctt catgaccatc aacaaggtgg tcacgcagga cgtcggtgtg 900

gtgctccgca agcggctctt ccagattgac gccgagtgcc ggtacgcgag cccaatgtac 960

gtggcgcgtt tctggatcat gaaggcgctc acggtgctct acatggtggc cctgccgtgc 1020

tacatgcagg gcccgtggca cggcctcaag ctgttcgcga tcgcgcactt tacgtgcggc 1080

gaggtgctcg caaccatgtt cattgtgaac cacatcatcg agggcgtctc gtacgcttcc 1140

aaggacgcgg tcaagggcac gatggcgccg ccgaagacga tgcacggcgt gacgcccatg 1200

aacaacacgc gcaaggaggt ggaggcggag gcgtccaagt ctggcgccgt ggtcaagtca 1260

gtcccgctcg acgactgggc cgtcgtccag tgccagacct cggtgaactg gagcgtcggc 1320

tcgtggttct ggaatcactt ttccggcggc ctcaaccacc agattgagca ccacctgttc 1380

cccgggctca gccacgagac gtactaccac attcaggacg tctttcagtc cacctgcgcc 1440

gagtacggcg tcccgtacca gcacgagcct tcgctctgga ccgcgtactg gaagatgctc 1500

gagcacctcc gtcagctcgg caatgaggag acccacgagt cctggcagcg cgctgcctga 1560

<210> 14
<211> 1560
<212> DNA
<213> Artificial sequence

<220>
<221> source
<223> /note="description of artificial sequence: Thraustochytrium sp."

<400> 14
atgactgttg gctatgatga agagataccc tttgaacaag tacgtgcaca taacaaacct 60

gacgatgctt ggtgtgctat tcatggtcat gtctatgatg tgaccaaatt tgcttcagtt 120

catccagggg gtgacatcat actattagca gctggtaaag aagcgaccgt actatacgag 180

acgtaccatg tcaggggtgt ctctgatgcg gtactgagga aatacagaat aggcaagttg 240

cctgatggtc aaggcggtgc taatgaaaag gaaaagcgca ctttatccgg attgtcatcg 300

gcttcgtact acacatggaa cagtgacttc tacagagtca tgagagaacg tgtggttgct 360

agacttaaag aaagagggaa agcacgaaga ggtggatatg agttatggat aaaggccttt 420

ttattgctgg ttggtttttg gtctagcttg tattggatgt gcacgttaga tccgtctttc 480

ggagccattc ttgcagccat gagcttaggt gtatttgctg cctttgttgg tacttgcata 540

cagcacgatg gtaatcatgg cgcttttgct caatctagat gggtgaacaa agtggctggt 600

tggactttgg acatgatcgg agcaagtggt atgacctggg agtttcaaca tgttctagga 660

caccatccat ataccaattt gattgaggaa gaaaatggct tgcagaaggt atctggcaaa 720

aagatggaca caaaactagc ggatcaggaa tctgatccag atgtgttttc cacttatccg 780

atgatgagac ttcatccatg gcatcaaaag agatggtacc acagattcca acatatctat 840

ggtcccttca ttttcggatt catgacgatt aacaaggttg ttacccaaga tgtaggtgtt 900

gtcttgcgta agagactctt ccaaattgat gctgagtgta ggtatgccag tccaatgtat 960

gttgcaaggt tttggattat gaaagccctt acggtactct atatggtagc tttaccctgt 1020

tacatgcaag gaccttggca tgggttgaaa ctgttcgcaa ttgcacactt tacatgtggt 1080

gaagtcttag cgactatgtt catagttaat cacatcattg aaggtgtttc gtatgcctca 1140

aaagacgcag taaaagggac tatggctcct ccaaaaacaa tgcatggtgt taccccgatg 1200

aacaatacac gaaaggaagt cgaagctgaa gcatccaaat ctggggcagt tgtgaagtca 1260

gtccctttag acgattgggc agtggttcaa tgccaaacaa gcgtgaattg gtcagttggc 1320

tcatggtttt ggaatcactt tagtggaggt ttgaatcatc agattgagca tcaccttttt 1380

cctgggttat cccacgagac ttactaccat atccaggatg ttttccaatc cacttgtgct 1440

gaatatggcg tcccatatca gcacgaacca tctttgtgga cagcctactg gaaaatgctg 1500

gaacatttaa ggcaactagg taacgaagag acacacgaaa gttggcaaag agcggcctaa 1560

<210> 15
<211> 1353
<212> DNA
<213> Mortierella alpia

<400> 15
atggcccccc ctcacgttgt cgacgaacaa gttcgacgca ggatcgtcgt tgaggacgag 60

atcaagtcta agaagcaatt tgagcgcaac tatgtgccca tggactttac gattaaggag 120

attcgagatg cgatccctgc ccacctcttc atccgtgata ccacaaagtc gatcctgcat 180

gtcgtcaagg atctggtcac catcgccatc gtcttttact gtgcaacctt cattgagact 240

ctgccctcgc tcgctctgcg agttcctgcc tggatcacct actggatcat ccaaggaact 300

gtcatggtcg gcccctggat cttggctcat ggtaaggaaa cgaaaaatcc catgtgtatt 360

tctgtactac agaaggcgaa gtttgtacct gaaaagatca gcgtcgtccc ttgatttaga 420

atgtaactaa ccttgcaatc gtatgaccta aattttcttg tgtcaacgac agagtgcggc 480

cacggagctt tctcggatag caagacgatc aacaccatct ttggatgggt cctccactct 540

gctcttttgg tgccctacca ggcctgggct atgtcacact ccaagcatca caagggtact 600

ggatcgatga ccaaagatgt cgttttcatc cctgccactc gttcctacaa gggcctccca 660

gcactggaga agcctgccgt cgaagaggag gtttcggagc aggaacacca ccaccacgag 720

gagtccatct ttgccgaaac tcccatctac acgctcggag cgcttttgtt cgtcttgacc 780

ttcggatggc ccttgtactt gatcgtcaac ttttcaggac acgaggcccc tcactgggtc 840

aaccatttcc agactgtcgc tcctctctat gagcctcacc agcgcaagaa catcttctac 900

tccaactgcg gcattgtcgc catgggttcg atcttgactt acctttcgat ggtcttctcg 960

cccttgactg tcttcatgta ctatggcatc ccttacctcg gagtcaacgc ctggatcgtc 1020

tgcattacct atctccagca caccgatccc aaggtgcctc acttccgtga taacgagtgg 1080

aacttccagc gcggtgctgc ctgcactatc gaccgatcct tcggtaccat cgtgaaccac 1140

ctgcaccacc acattggcga ctctcaccag tgccaccata tgttctcgca gatgcccttc 1200

tacaatgctg tggaggctac aaagtacttg aaggccaaac ttggcaagta ctacatattt 1260

gacgacacgc ccattgccaa agccctctac cgcaattgga gagagtgcaa attcgtggag 1320

gacgagggag atgtagtgtt ttacaagcat taa 1353

<210> 16
<211> 1353
<212> DNA
<213> Artificial sequence

<220>
<221> source
<223> /note="description of artificial sequence: Mortierella alpina"

<400> 16
atggctcctc ctcatgtcgt ggatgaacaa gttagacgta ggatagtagt agaagatgag 60

attaagtcca aaaagcaatt tgagaggaat tatgtaccga tggatttcac gatcaaagag 120

attagagatg ccattccagc ccacttgttc attcgcgaca ctacgaagtc aatacttcac 180

gtggttaaag accttgtgac tatagcgata gtgttctatt gtgctacctt tatcgaaact 240

ttaccgtcat tggccttgag agtacccgca tggattacat actggatcat ccaaggaact 300

gttatggtcg gaccatggat tttggcgcat ggtaaggaga ccaaaaaccc catgtgcatt 360

tctgtcctgc aaaaggcaaa gtttgttcca gagaaaattt ccgttgtacc ctaatttcgt 420

atgtagttaa cattacagtc ctatgatctg aatttcctag tctctactac tgaatgtggt 480

catggagcat tttctgacag taaaaccata aacacgatct ttggttgggt gttacattcg 540

gcattgcttg taccatatca agcatgggct atgtcacact ctaagcacca taaaggtaca 600

ggtagcatga ccaaagatgt tgttttcatt cctgctacaa gatcctataa agggttacca 660

gctctagaga aacctgcagt tgaagaagag gtgagcgaac aagaacatca ccatcacgaa 720

gaaagcatat ttgcagaaac ccctatatac acattaggcg ctttactgtt tgttttgaca 780

tttggttggc ccttatatct aatcgtcaat tttagtggcc atgaagctcc tcattgggtt 840

aatcacttcc agacagttgc tcctctatat gaaccacatc aaaggaagaa catcttttac 900

tcgaattgtg ggattgttgc tatgggttct attctcactt acctatctat ggtcttttca 960

ccattgacag tctttatgta ctatggcatt ccgtatttgg gtgttaacgc ttggattgtg 1020

tgtatcactt atcttcagca taccgatcct aaagttccac actttagaga caacgaatgg 1080

aacttccaaa gaggagctgc atgtacaatt gacagaagtt tcggtactat agtgaatcac 1140

ctgcatcatc atattggaga tagtcatcag tgccatcaca tgttctcaca aatgccattt 1200

tacaatgccg ttgaagcgac taagtatctc aaagccaaat taggcaaata ctacatcttc 1260

gatgatacgc caatagccaa agccttgtac agaaattggc gagaatgcaa gtttgtagaa 1320

gatgaaggtg acgtagtctt ctataagcat tga 1353

<210> 17
<211> 8760
<212> DNA
<213> pFlat1

<400> 17
gaattctgaa ccagtcctaa aacgagtaaa taggaccggc aattcttcaa gcaataaaca 60

ggaataccaa ttattaaaag ataacttagt cagatcgtac aataaagctt tgaagaaaaa 120

tgcgccttat tcaatctttg ctataaaaaa tggcccaaaa tctcacattg gaagacattt 180

gatgacctca tttctttcaa tgaagggcct aacggagttg actaatgttg tgggaaattg 240

gagcgataag cgtgcttctg ccgtggccag gacaacgtat actcatcaga taacagcaat 300

acctgatcac tacttcgcac tagtttctcg gtactatgca tatgatccaa tatcaaagga 360

aatgatagca ttgaaggatg agactaatcc aattgaggag tggcagcata tagaacagct 420

aaagggtagt gctgaaggaa gcatacgata ccccgcatgg aatgggataa tatcacagga 480

ggtactagac tacctttcat cctacataaa tagacgcata taagtacgca tttaagcata 540

aacacgcact atgccgttct tctcatgtat atatatatac aggcaacacg cagatatagg 600

tgcgacgtga acagtgagct gtatgtgcgc agctcgcgtt gcattttcgg aagcgctcgt 660

tttcggaaac gctttgaagt tcctattccg aagttcctat tctctagaaa gtataggaac 720

ttcagagcgc ttttgaaaac caaaagcgct ctgaagacgc actttcaaaa aaccaaaaac 780

gcaccggact gtaacgagct actaaaatat tgcgaatacc gcttccacaa acattgctca 840

aaagtatctc tttgctatat atctctgtgc tatatcccta tataacctac ccatccacct 900

ttcgctcctt gaacttgcat ctaaactcga cctctacatt ttttatgttt atctctagta 960

ttactcttta gacaaaaaaa ttgtagtaag aactattcat agagtgaatc gaaaacaata 1020

cgaaaatgta aacatttcct atacgtagta tatagagaca aaatagaaga aaccgttcat 1080

aattttctga ccaatgaaga atcatcaacg ctatcacttt ctgttcacaa agtatgcgca 1140

atccacatcg gtatagaata taatcgggga tgcctttatc ttgaaaaaat gcacccgcag 1200

cttcgctagt aatcagtaaa cgcgggaagt ggagtcaggc tttttttatg gaagagaaaa 1260

tagacaccaa agtagccttc ttctaacctt aacggaccta cagtgcaaaa agttatcaag 1320

agactgcatt atagagcgca caaaggagaa aaaaagtaat ctaagatgct ttgttagaaa 1380

aatagcgctc tcgggatgca tttttgtaga acaaaaaaga agtatagatt ctttgttggt 1440

aaaatagcgc tctcgcgttg catttctgtt ctgtaaaaat gcagctcaga ttctttgttt 1500

gaaaaattag cgctctcgcg ttgcattttt gttttacaaa aatgaagcac agattcttcg 1560

ttggtaaaat agcgctttcg cgttgcattt ctgttctgta aaaatgcagc tcagattctt 1620

tgtttgaaaa attagcgctc tcgcgttgca tttttgttct acaaaatgaa gcacagatgc 1680

ttcgttaaca aagatatgct attgaagtgc aagatggaaa cgcagaaaat gaaccgggga 1740

tgcgacgtgc aagattacct atgcaataga tgcaatagtt tctccaggaa ccgaaataca 1800

tacattgtct tccgtaaagc gctagactat atattattat acaggttcaa atatactatc 1860

tgtttcaggg aaaactccca ggttcggatg ttcaaaattc aatgatgggt aacaagtacg 1920

atcgtaaatc tgtaaaacag tttgtcggat attaggctgt atctcctcaa agcgtattcg 1980

aatatcattg agaagctgca gcgtcacatc ggataataat gatggcagcc attgtagaag 2040

tgccttttgc atttctagtc tctttctcgg tctagctagt tttactacat cgcgaagata 2100

gaatcttaga tcacactgcc tttgctgagc tggatcaata gagtaacaaa agagtggtaa 2160

ggcctcgtta aaggacaagg acctgagcgg aagtgtatcg tacagtagac ggagtatact 2220

agtatagtct atagtccgtg gaattctcat gtttgacagc ttatcatcga taagcttttc 2280

aattcatcat ttttttttta ttcttttttt tgatttcggt ttccttgaaa tttttttgat 2340

tcggtaatct ccgaacagaa ggaagaacga aggaaggagc acagacttag attggtatat 2400

atacgcatat gtagtgttga agaaacatga aattgcccag tattcttaac ccaactgcac 2460

agaacaaaaa cctgcaggaa acgaagataa atcatgtcga aagctacata taaggaacgt 2520

gctgctactc atcctagtcc tgttgctgcc aagctattta atatcatgca cgaaaagcaa 2580

acaaacttgt gtgcttcatt ggatgttcgt accaccaagg aattactgga gttagttgaa 2640

gcattaggtc ccaaaatttg tttactaaaa acacatgtgg atatcttgac tgatttttcc 2700

atggagggca cagttaagcc gctaaaggca ttatccgcca agtacaattt tttactcttc 2760

gaagacagaa aatttgctga cattggtaat acagtcaaat tgcagtactc tgcgggtgta 2820

tacagaatag cagaatgggc agacattacg aatgcacacg gtgtggtggg cccaggtatt 2880

gttagcggtt tgaagcaggc ggcagaagaa gtaacaaagg aacctagagg ccttttgatg 2940

ttagcagaat tgtcatgcaa gggctcccta tctactggag aatatactaa gggtactgtt 3000
gacattgcga agagcgacaa agattttgtt atcggcttta ttgctcaaag agacatgggt 3060
ggaagagatg aaggttacga ttggttgatt atgacacccg gtgtgggttt agatgacaag 3120
ggagacgcat tgggtcaaca gtatagaacc gtggatgatg tggtctctac aggatctgac 3180
attattattg ttggaagagg actatttgca aagggaaggg atgctaaggt agagggtgaa 3240
cgttacagaa aagcaggctg ggaagcatat ttgagaagat gcggccagca aaactaaaaa 3300
actgtattat aagtaaatgc atgtatacta aactcacaaa ttagagcttc aatttaatta 3360
tatcagttat tacccgggaa tctcggtcgt aatgattttt ataatgacga aaaaaaaaaa 3420
attggaaaga aaaagcttaa tgcggtagtt tatcacagtt aaattgctaa cgcagtcagg 3480
caccgtgtat gaaatctaac aatgcgctca tcgtcatcct cggcaccgtc accctggatg 3540
ctgtaggcat aggcttggtt atgccggtac tgccgggcct cttgcgggat atcgtccatt 3600
ccgacagcat cgccagtcac tatggcgtgc tgctagcgct atatgcgttg atgcaatttc 3660
tatgcgcacc cgttctcgga gcactgtccg accgctttgg ccgccgccca gtcctgctcg 3720
cttcgctact tggagccact atcgactacg cgatcatggc gaccacaccc gtcctgtgga 3780
tcctctacgc cggacgcatc gtggccggca tcaccggcgc cacaggtgcg gttgctggcg 3840
cctatatcgc cgacatcacc gatggggaag atcgggctcg ccacttcggg ctcatgagcg 3900
cttgtttcgg cgtgggtatg gtggcaggcc ccgtggccgg gggactgttg ggcgccatct 3960
ccttgcatgc aacttctttt cttttttttt cttttctctc tcccccgttg ttgtctcacc 4020
atatccgcaa tgacaaaaaa atgatggaag acactaaagg aaaaaattaa cgacaaagac 4080
agcaccaaca gatgtcgttg ttccagagct gatgaggggt atctcgaagc acacgaaact 4140
ttttccttcc ttcattcacg cacactactc tctaatgagc aacggtatac ggccttcctt 4200
ccagttactt gaatttgaaa taaaaaaaag tttgctgtct tgctatcaag tataaataga 4260
cctgcaatta ttaatctttt gtttcctcgt cattgttctc gttccctttc ttccttgttt 4320
ctttttctgc acaatatttc aagctatacc aagcatacaa tcaaggaatt cgagctaagc 4380
ggccgcaaag atctaactcg agaaacgcgt tagagctaag cggccgcaaa gatctaactc 4440
gagaaacgcg ttagagctaa gcggccgcaa agatctaact cgagaaacgc gttagagctc 4500
ggtacccggg gatcctctag agtcgacgct ctcccttatg cgactcctgc attaggaagc 4560
agcccagtag taggttgagg ccgttgagca ccgccgccgc aaggaatggt gcatgcaagg 4620
agatggcgcc caacagtccc ccggccacgg ggcctgccac catacccacg ccgaaacaag 4680
cgctcatgag cccgaagtgg cgagcccgat cttccccatc ggtgatgtcg gcgatatagg 4740

cgccagcaac cgcacctgtg gcgccggtga tgccggccac gatgcgtccg gcgtagagga 4800

tcttttatgc ttgcttttca aaaggcctgc aggcaagtgc acaaacaata cttaaataaa 4860

tactactcag taataaccta tttcttagca tttttgacga aatttgctat tttgttagag 4920

tcttttacac catttgtctc cacacctccg cttacatcaa caccaataac gccatttaat 4980

ctaagcgcat caccaacatt ttctggcgtc agtccaccag ctaacataaa atgtaagctt 5040

gcatgcctgc aggtcgaccg atgcccttga gagccttcaa cccagtcagc tccttccggt 5100

gggcgcgggg catgactatc gtcgccgcac ttatgactgt cttctttatc atgcaactcg 5160

taggacaggt gccggcagcg ctctgggtca ttttcggcga ggaccgcttt cgctggagcg 5220

cgacgatgat cggcctgtcg cttgcggtat tcggaatctt gcacgccctc gctcaagcct 5280

tcgtcactgg tcccgccacc aaacgtttcg gcgagaagca ggccattatc gccggcatgg 5340

cggccgacgc gctgggctac gtcttgctgg cgttcgcgac gcgaggctgg atggccttcc 5400

ccattatgat tcttctcgct tccggcggca tcgggatgcc cgcgttgcag gccatgctgt 5460

ccaggcaggt agatgacgac catcagggac agcttcaagg atcgctcgcg gctcttacca 5520

gcctaacttc gatcattgga ccgctgatcg tcacggcgat ttatgccgcc tcggcgagca 5580

catggaacgg gttggcatgg attgtaggcg ccgccctata ccttgtctgc ctccccgcgt 5640

tgcgtcgcgg tgcatggagc cgggccacct cgacctgaat ggaagccggc ggcacctcgc 5700

taacggattc accactccaa gaattggagc caatcaattc ttgcggagaa ctgtgaatgc 5760

gcaaaccaac ccttggcaga acatatccat cgcgtccgcc atctccagca gccgcacgcg 5820

gcgcatctcg ggcagcgttg ggtcctggcc acgggtgcgc atgatcgtgc tcctgtcgtt 5880

gaggacccgg ctaggctggc ggggttgcct tactggttag cagaatgaat caccgatacg 5940

cgagcgaacg tgaagcgact gctgctgcaa aacgtctgcg acctgagcaa caacatgaat 6000

ggtcttcggt ttccgtgttt cgtaaagtct ggaaacgcgg aagtcagcgc cctgcaccat 6060

tatgttccgg atctgcatcg caggatgctg ctggctaccc tgtggaacac ctacatctgt 6120

attaacgaag cgctggcatt gaccctgagt gatttttctc tggtcccgcc gcatccatac 6180

cgccagttgt ttaccctcac aacgttccag taaccgggca tgttcatcat cagtaacccg 6240

tatcgtgagc atcctctctc gtttcatcgg tatcattacc cccatgaaca gaaatccccc 6300

ttacacggag gcatcagtga ccaaacagga aaaaaccgcc cttaacatgg cccgctttat 6360

cagaagccag acattaacgc ttctggagaa actcaacgag ctggacgcgg atgaacaggc 6420

agacatctgt gaatcgcttc acgaccacgc tgatgagctt taccgcagct gcctcgcgcg 6480

tttcggtgat gacggtgaaa acctctgaca catgcagctc ccggagacgg tcacagcttg 6540

tctgtaagcg gatgccggga gcagacaagc ccgtcagggc gcgtcagcgg gtgttggcgg 6600
gtgtcggggc gcagccatga cccagtcacg tagcgatagc ggagtgtata ctggcttaac 6660
tatgcggcat cagagcagat tgtactgaga gtgcaccata tgcggtgtga aataccgcac 6720
agatgcgtaa ggagaaaata ccgcatcagg cgctcttccg cttcctcgct cactgactcg 6780
ctgcgctcgg tcgttcggct gcggcgagcg gtatcagctc actcaaaggc ggtaatacgg 6840
ttatccacag aatcagggga taacgcagga aagaacatgt gagcaaaagg ccagcaaaag 6900
gccaggaacc gtaaaaaggc cgcgttgctg gcgtttttcc ataggctccg cccccctgac 6960
gagcatcaca aaaatcgacg ctcaagtcag aggtggcgaa acccgacagg actataaaga 7020
taccaggcgt ttccccctgg aagctccctc gtgcgctctc ctgttccgac cctgccgctt 7080
accggatacc tgtccgcctt tctcccttcg ggaagcgtgg cgctttctca tagctcacgc 7140
tgtaggtatc tcagttcggt gtaggtcgtt cgctccaagc tgggctgtgt gcacgaaccc 7200
cccgttcagc ccgaccgctg cgccttatcc ggtaactatc gtcttgagtc caacccggta 7260
agacacgact tatcgccact ggcagcagcc actggtaaca ggattagcag agcgaggtat 7320
gtaggcggtg ctacagagtt cttgaagtgg tggcctaact acggctacac tagaaggaca 7380
gtatttggta tctgcgctct gctgaagcca gttaccttcg gaaaaagagt tggtagctct 7440
tgatccggca aacaaaccac cgctggtagc ggtggttttt ttgtttgcaa gcagcagatt 7500
acgcgcagaa aaaaaggatc tcaagaagat cctttgatct tttctacggg gtctgacgct 7560
cagtggaacg aaaactcacg ttaagggatt ttggtcatga gattatcaaa aaggatcttc 7620
acctagatcc ttttaaatta aaaatgaagt tttaaatcaa tctaaagtat atatgagtaa 7680
acttggtctg acagttacca atgcttaatc agtgaggcac ctatctcagc gatctgtcta 7740
tttcgttcat ccatagttgc ctgactcccc gtcgtgtaga taactacgat acgggagggc 7800
ttaccatctg gccccagtgc tgcaatgata ccgcgagacc cacgctcacc ggctccagat 7860
ttatcagcaa taaaccagcc agccggaagg gccgagcgca gaagtggtcc tgcaacttta 7920
tccgcctcca tccagtctat taattgttgc cgggaagcta gagtaagtag ttcgccagtt 7980
aatagtttgc gcaacgttgt tgccattgct gcaggcatcg tggtgtcacg ctcgtcgttt 8040
ggtatggctt cattcagctc cggttcccaa cgatcaaggc gagttacatg atcccccatg 8100
ttgtgcaaaa aagcggttag ctccttcggt cctccgatcg ttgtcagaag taagttggcc 8160
gcagtgttat cactcatggt tatggcagca ctgcataatt ctcttactgt catgccatcc 8220
gtaagatgct tttctgtgac tggtgagtac tcaaccaagt cattctgaga atagtgtatg 8280
cggcgaccga gttgctcttg cccggcgtca acacgggata ataccgcgcc acatagcaga 8340

actttaaaag tgctcatcat tggaaaacgt tcttcggggc gaaaactctc aaggatctta 8400

ccgctgttga gatccagttc gatgtaaccc actcgtgcac ccaactgatc ttcagcatct 8460

tttactttca ccagcgtttc tgggtgagca aaaacaggaa ggcaaaatgc cgcaaaaaag 8520

ggaataaggg cgacacggaa atgttgaata ctcatactct tcctttttca atattattga 8580

agcatttatc agggttattg tctcatgagc ggatacatat ttgaatgtat ttagaaaaat 8640

aaacaaatag gggttccgcg cacatttccc cgaaaagtgc cacctgacgt ctaagaaacc 8700

attattatca tgacattaac ctataaaaat aggcgtatca cgaggccctt tcgtcttcaa 8760

<210> 18
<211> 9706
<212> DNA
<213> pFlat2

<400> 18
gaattctgaa ccagtcctaa aacgagtaaa taggaccggc aattcttcaa gcaataaaca 60

ggaataccaa ttattaaaag ataacttagt cagatcgtac aataaagctt tgaagaaaaa 120

tgcgccttat tcaatctttg ctataaaaaa tggcccaaaa tctcacattg gaagacattt 180

gatgacctca tttctttcaa tgaagggcct aacggagttg actaatgttg tgggaaattg 240

gagcgataag cgtgcttctg ccgtggccag gacaacgtat actcatcaga taacagcaat 300

acctgatcac tacttcgcac tagtttctcg gtactatgca tatgatccaa tatcaaagga 360

aatgatagca ttgaaggatg agactaatcc aattgaggag tggcagcata tagaacagct 420

aaagggtagt gctgaaggaa gcatacgata ccccgcatgg aatgggataa tatcacagga 480

ggtactagac tacctttcat cctacataaa tagacgcata taagtacgca tttaagcata 540

aacacgcact atgccgttct tctcatgtat atatatatac aggcaacacg cagatatagg 600

tgcgacgtga acagtgagct gtatgtgcgc agctcgcgtt gcattttcgg aagcgctcgt 660

tttcggaaac gctttgaagt tcctattccg aagttcctat tctctagaaa gtataggaac 720

ttcagagcgc ttttgaaaac caaaagcgct ctgaagacgc actttcaaaa aaccaaaaac 780

gcaccggact gtaacgagct actaaaatat tgcgaatacc gcttccacaa acattgctca 840

aaagtatctc tttgctatat atctctgtgc tatatcccta tataacctac ccatccacct 900

ttcgctcctt gaacttgcat ctaaactcga cctctacatt ttttatgttt atctctagta 960

ttactcttta gacaaaaaaa ttgtagtaag aactattcat agagtgaatc gaaaacaata 1020

cgaaaatgta aacatttcct atacgtagta tatagagaca aaatagaaga aaccgttcat 1080

aattttctga ccaatgaaga atcatcaacg ctatcacttt ctgttcacaa agtatgcgca 1140

atccacatcg gtatagaata taatcgggga tgcctttatc ttgaaaaaat gcacccgcag 1200

cttcgctagt aatcagtaaa cgcgggaagt ggagtcaggc tttttttatg gaagagaaaa 1260

tagacaccaa agtagccttc ttctaacctt aacggaccta cagtgcaaaa agttatcaag 1320

agactgcatt atagagcgca caaaggagaa aaaaagtaat ctaagatgct ttgttagaaa 1380

aatagcgctc tcgggatgca tttttgtaga acaaaaaaga agtatagatt ctttgttggt 1440

aaaatagcgc tctcgcgttg catttctgtt ctgtaaaaat gcagctcaga ttctttgttt 1500

gaaaaattag cgctctcgcg ttgcattttt gttttacaaa aatgaagcac agattcttcg 1560

ttggtaaaat agcgctttcg cgttgcattt ctgttctgta aaaatgcagc tcagattctt 1620

tgtttgaaaa attagcgctc tcgcgttgca tttttgttct acaaaatgaa gcacagatgc 1680

ttcgttaaca aagatatgct attgaagtgc aagatggaaa cgcagaaaat gaaccgggga 1740

tgcgacgtgc aagattacct atgcaataga tgcaatagtt tctccaggaa ccgaaataca 1800

tacattgtct tccgtaaagc gctagactat atattattat acaggttcaa atatactatc 1860

tgtttcaggg aaaactccca ggttcggatg ttcaaaattc aatgatgggt aacaagtacg 1920

atcgtaaatc tgtaaaacag tttgtcggat attaggctgt atctcctcaa agcgtattcg 1980

aatatcattg agaagctgca gcgtcacatc ggataataat gatggcagcc attgtagaag 2040

tgccttttgc atttctagtc tctttctcgg tctagctagt tttactacat cgcgaagata 2100

gaatcgatcc cccacacacc atagcttcaa aatgtttcta ctcctttttt actcttccag 2160

attttctcgg actccgcgca tcgccgtacc acttcaaaac acccaagcac agcatactaa 2220

atttcccctc tttcttcctc tagggtgtcg ttaattaccc gtactaaagg tttggaaaag 2280

aaaaaagaga ccgcctcgtt tctttttctt cgtcgaaaaa ggcaataaaa atttttatca 2340

cgtttctttt tcttgaaaat tttttttttt gatttttttc tctttcgatg acctcccatt 2400

gatatttaag ttaataaacg gtcttcaatt tctcaagttt cagtttcatt tttcttgttc 2460

tattacaact ttttttactt cttgctcatt agaaagaaag catagcaatc taatctaagg 2520

gcgagctcga attcctcgat cgaggccgcg attaaattcc aacatggatg ctgatttata 2580

tgggtataaa tgggctcgcg ataatgtcgg gcaatcaggt gcgacaatct atcgattgta 2640

tgggaagccc gatgcgccag agttgtttct gaaacatggc aaaggtagcg ttgccaatga 2700

tgttacagat gagatggtca gactaaactg gctgacggaa tttatgcctc ttccgaccat 2760

caagcatttt atccgtactc ctgatgatgc atggttactc accactgcga tccccgggaa 2820

aacagcattc caggtattag aagaatatcc tgattcaggt gaaaatattg ttgatgcgct 2880

ggcagtgttc ctgcgccggt tgcattcgat tcctgtttgt aattgtcctt ttaacagcga 2940

tcgcgtattt cgtctcgctc aggcgcaatc acgaatgaat aacggtttgg ttgatgcgag 3000

tgattttgat gacgagcgta atggctggcc tgttgaacaa gtctggaaag aaatgcataa 3060

gcttttgcca ttctcaccgg attcagtcgt cactcatggt gatttctcac ttgataacct 3120

tatttttgac gaggggaaat taataggttg tattgatgtt ggacgagtcg gaatcgcaga 3180

ccgataccag gatcttgcca tcctatggaa ctgcctcggt gagttttctc cttcattaca 3240

gaaacggctt tttcaaaaat atggtattga taatcctgat atgaataaat tgcagtttca 3300

tttgatgctc gatgagtttt tctaatcaga attggttaat tggttgtaac actggcagag 3360

cattacgctg acttgacggg acggcggctt tgttgaataa atcgaacttt tgctgagttg 3420

aaggatcaga tcacgcatct tcccgacaac gcagaccgtt ccgtggcaaa gcaaaagttc 3480

aaaatcacca actggtccac ctacaacaaa gctctcatca accgtggctc cctcactttc 3540

tggctggatg atggggcgat tcaggcctgg tatgagtcag caacaccttc ttcacgaggc 3600

agacctcagc gccccccccc ccctgcaggt cgactctaga ggatccatgg agggcacagt 3660

taagccgcta aaggcattat ccgccaagta caatttttta ctcttcgaag acagaaaatt 3720

tgctgacatt ggtaatacag tcaaattgca gtactctgcg ggtgtataca gaatagcaga 3780

atgggcagac attacgaatg cacacggtgt ggtgggccca ggtattgtta gcggtttgaa 3840

gcaggcggca gaagaagtaa caaaggaacc tagaggcctt ttgatgttag cagaattgtc 3900

atgcaagggc tccctatcta ctggagaata tactaagggt actgttgaca ttgcgaagag 3960

cgacaaagat tttgttatcg gctttattgc tcaaagagac atgggtggaa gagatgaagg 4020

ttacgattgg ttgattatga cacccggtgt gggtttagat gacaagggag acgcattggg 4080

tcaacagtat agaaccgtgg atgatgtggt ctctacagga tctgacatta ttattgttgg 4140

aagaggacta tttgcaaagg gaagggatgc taaggtagag ggtgaacgtt acagaaaagc 4200

aggctgggaa gcatatttga gaagatgcgg ccagcaaaac taaaaaactg tattataagt 4260

aaatgcatgt atactaaact cacaaattag agcttcaatt taattatatc agttattacc 4320

cgggaatctc ggtcgtaatg atttttataa tgacgaaaaa aaaaaaattg gaaagaaaaa 4380

gcttaatgcg gtagtttatc acagttaaat tgctaacgca gtcaggcacc gtgtatgaaa 4440

tctaacaatg cgctcatcgt catcctcggc accgtcaccc tggatgctgt aggcataggc 4500

ttggttatgc cggtactgcc gggcctcttg cgggatatcg tccattccga cagcatcgcc 4560

agtcactatg gcgtgctgct agcgctatat gcgttgatgc aatttctatg cgcacccgtt 4620

ctcggagcac tgtccgaccg ctttggccgc cgcccagtcc tgctcgcttc gctacttgga 4680

gccactatcg actacgcgat catggcgacc acacccgtcc tgtggatcct ctacgccgga 4740

cgcatcgtgg ccggcatcac cggcgccaca ggtgcggttg ctggcgccta tatcgccgac 4800

atcaccgatg gggaagatcg ggctcgccac ttcgggctca tgagcgcttg tttcggcgtg 4860

ggtatggtgg caggccccgt ggccggggga ctgttgggcg ccatctcctt gcatgcaact 4920

tcttttcttt ttttttcttt tctctctccc ccgttgttgt ctcaccatat ccgcaatgac 4980

aaaaaaatga tggaagacac taaaggaaaa aattaacgac aaagacagca ccaacagatg 5040

tcgttgttcc agagctgatg aggggtatct cgaagcacac gaaacttttt ccttccttca 5100

ttcacgcaca ctactctcta atgagcaacg gtatacggcc ttccttccag ttacttgaat 5160

ttgaaataaa aaaaagtttg ctgtcttgct atcaagtata aatagacctg caattattaa 5220

tcttttgttt cctcgtcatt gttctcgttc cctttcttcc ttgtttcttt ttctgcacaa 5280

tatttcaagc tataccaagc atacaatcaa ggaattcgag ctaagcggcc gcaaagatct 5340

aactcgagaa acgcgttaga gctaagcggc cgcaaagatc taactcgaga aacgcgttag 5400

agctaagcgg ccgcaaagat ctaactcgag aaacgcgtta gagctcggta cccggggatc 5460

ctctagagtc gacgctctcc cttatgcgac tcctgcatta ggaagcagcc cagtagtagg 5520

ttgaggccgt tgagcaccgc cgccgcaagg aatggtgcat gcaaggagat ggcgcccaac 5580

agtcccccgg ccacggggcc tgccaccata cccacgccga aacaagcgct catgagcccg 5640

aagtggcgag cccgatcttc cccatcggtg atgtcggcga tataggcgcc agcaaccgca 5700

cctgtggcgc cggtgatgcc ggccacgatg cgtccggcgt agaggatctt ttatgcttgc 5760

ttttcaaaag gcctgcaggc aagtgcacaa acaatactta aataaatact actcagtaat 5820

aacctatttc ttagcatttt tgacgaaatt tgctattttg ttagagtctt ttacaccatt 5880

tgtctccaca cctccgctta catcaacacc aataacgcca tttaatctaa gcgcatcacc 5940

aacattttct ggcgtcagtc caccagctaa cataaaatgt aagcttgcat gcctgcaggt 6000

cgaccgatgc ccttgagagc cttcaaccca gtcagctcct tccggtgggc gcggggcatg 6060

actatcgtcg ccgcacttat gactgtcttc tttatcatgc aactcgtagg acaggtgccg 6120

gcagcgctct gggtcatttt cggcgaggac cgctttcgct ggagcgcgac gatgatcggc 6180

ctgtcgcttg cggtattcgg aatcttgcac gccctcgctc aagccttcgt cactggtccc 6240

gccaccaaac gtttcggcga gaagcaggcc attatcgccg gcatggcggc cgacgcgctg 6300

ggctacgtct tgctggcgtt cgcgacgcga ggctggatgg ccttccccat tatgattctt 6360

ctcgcttccg gcggcatcgg gatgcccgcg ttgcaggcca tgctgtccag gcaggtagat 6420

gacgaccatc agggacagct tcaaggatcg ctcgcggctc ttaccagcct aacttcgatc 6480

attggaccgc tgatcgtcac ggcgatttat gccgcctcgg cgagcacatg gaacgggttg 6540

gcatggattg taggcgccgc cctatacctt gtctgcctcc ccgcgttgcg tcgcggtgca 6600

tggagccggg ccacctcgac ctgaatggaa gccggcggca cctcgctaac ggattcacca 6660
ctccaagaat tggagccaat caattcttgc ggagaactgt gaatgcgcaa accaaccctt 6720
ggcagaacat atccatcgcg tccgccatct ccagcagccg cacgcggcgc atctcgggca 6780
gcgttgggtc ctggccacgg gtgcgcatga tcgtgctcct gtcgttgagg acccggctag 6840
gctggcgggg ttgccttact ggttagcaga atgaatcacc gatacgcgag cgaacgtgaa 6900
gcgactgctg ctgcaaaacg tctgcgacct gagcaacaac atgaatggtc ttcggtttcc 6960
gtgtttcgta aagtctggaa acgcggaagt cagcgccctg caccattatg ttccggatct 7020
gcatcgcagg atgctgctgg ctaccctgtg gaacacctac atctgtatta acgaagcgct 7080
ggcattgacc ctgagtgatt tttctctggt cccgccgcat ccataccgcc agttgtttac 7140
cctcacaacg ttccagtaac cgggcatgtt catcatcagt aacccgtatc gtgagcatcc 7200
tctctcgttt catcggtatc attaccccca tgaacagaaa tcccccttac acggaggcat 7260
cagtgaccaa acaggaaaaa accgccctta acatggcccg ctttatcaga agccagacat 7320
taacgcttct ggagaaactc aacgagctgg acgcggatga acaggcagac atctgtgaat 7380
cgcttcacga ccacgctgat gagctttacc gcagctgcct cgcgcgtttc ggtgatgacg 7440
gtgaaaacct ctgacacatg cagctcccgg agacggtcac agcttgtctg taagcggatg 7500
ccgggagcag acaagcccgt cagggcgcgt cagcgggtgt tggcgggtgt cggggcgcag 7560
ccatgaccca gtcacgtagc gatagcggag tgtatactgg cttaactatg cggcatcaga 7620
gcagattgta ctgagagtgc accatatgcg gtgtgaaata ccgcacagat gcgtaaggag 7680
aaaataccgc atcaggcgct cttccgcttc ctcgctcact gactcgctgc gctcggtcgt 7740
tcggctgcgg cgagcggtat cagctcactc aaaggcggta atacggttat ccacagaatc 7800
aggggataac gcaggaaaga acatgtgagc aaaaggccag caaaaggcca ggaaccgtaa 7860
aaaggccgcg ttgctggcgt ttttccatag gctccgcccc cctgacgagc atcacaaaaa 7920
tcgacgctca agtcagaggt ggcgaaaccc gacaggacta taaagatacc aggcgtttcc 7980
ccctggaagc tccctcgtgc gctctcctgt tccgaccctg ccgcttaccg gatacctgtc 8040
cgcctttctc ccttcgggaa gcgtggcgct ttctcatagc tcacgctgta ggtatctcag 8100
ttcggtgtag gtcgttcgct ccaagctggg ctgtgtgcac gaaccccccg ttcagcccga 8160
ccgctgcgcc ttatccggta actatcgtct tgagtccaac ccggtaagac acgacttatc 8220
gccactggca gcagccactg gtaacaggat tagcagagcg aggtatgtag gcggtgctac 8280
agagttcttg aagtggtggc ctaactacgg ctacactaga aggacagtat ttggtatctg 8340
cgctctgctg aagccagtta ccttcggaaa aagagttggt agctcttgat ccggcaaaca 8400

aaccaccgct ggtagcggtg gtttttttgt ttgcaagcag cagattacgc gcagaaaaaa 8460

aggatctcaa gaagatcctt tgatcttttc tacggggtct gacgctcagt ggaacgaaaa 8520

ctcacgttaa gggattttgg tcatgagatt atcaaaaagg atcttcacct agatcctttt 8580

aaattaaaaa tgaagtttta aatcaatcta aagtatatat gagtaaactt ggtctgacag 8640

ttaccaatgc ttaatcagtg aggcacctat ctcagcgatc tgtctatttc gttcatccat 8700

agttgcctga ctccccgtcg tgtagataac tacgatacgg gagggcttac catctggccc 8760

cagtgctgca atgataccgc gagacccacg ctcaccggct ccagatttat cagcaataaa 8820

ccagccagcc ggaagggccg agcgcagaag tggtcctgca actttatccg cctccatcca 8880

gtctattaat tgttgccggg aagctagagt aagtagttcg ccagttaata gtttgcgcaa 8940

cgttgttgcc attgctgcag gcatcgtggt gtcacgctcg tcgtttggta tggcttcatt 9000

cagctccggt tcccaacgat caaggcgagt tacatgatcc cccatgttgt gcaaaaaagc 9060

ggttagctcc ttcggtcctc cgatcgttgt cagaagtaag ttggccgcag tgttatcact 9120

catggttatg gcagcactgc ataattctct tactgtcatg ccatccgtaa gatgcttttc 9180

tgtgactggt gagtactcaa ccaagtcatt ctgagaatag tgtatgcggc gaccgagttg 9240

ctcttgcccg gcgtcaacac gggataatac cgcgccacat agcagaactt taaaagtgct 9300

catcattgga aaacgttctt cggggcgaaa actctcaagg atcttaccgc tgttgagatc 9360

cagttcgatg taacccactc gtgcacccaa ctgatcttca gcatctttta ctttcaccag 9420

cgtttctggg tgagcaaaaa caggaaggca aaatgccgca aaaaagggaa taagggcgac 9480

acggaaatgt tgaatactca tactcttcct ttttcaatat tattgaagca tttatcaggg 9540

ttattgtctc atgagcggat acatatttga atgtatttag aaaaataaac aaataggggt 9600

tccgcgcaca tttccccgaa aagtgccacc tgacgtctaa gaaaccatta ttatcatgac 9660

attaacctat aaaaataggc gtatcacgag gccctttcgt cttcaa 9706

<210> 19
<211> 10417
<212> DNA
<213> pFlat3

<400> 19
   gaattctgaa ccagtcctaa aacgagtaaa taggaccggc aattcttcaa gcaataaaca 60
   ggaataccaa ttattaaaag ataacttagt cagatcgtac aataaagctt tgaagaaaaa 120
   tgcgccttat tcaatctttg ctataaaaaa tggcccaaaa tctcacattg gaagacattt 180
   gatgacctca tttctttcaa tgaagggcct aacggagttg actaatgttg tgggaaattg 240
   gagcgataag cgtgcttctg ccgtggccag gacaacgtat actcatcaga taacagcaat 300
   acctgatcac tacttcgcac tagtttctcg gtactatgca tatgatccaa tatcaaagga 360
   aatgatagca ttgaaggatg agactaatcc aattgaggag tggcagcata tagaacagct 420
   aaagggtagt gctgaaggaa gcatacgata ccccgcatgg aatgggataa tatcacagga 480
   ggtactagac tacctttcat cctacataaa tagacgcata taagtacgca tttaagcata 540
   aacacgcact atgccgttct tctcatgtat atatatatac aggcaacacg cagatatagg 600
   tgcgacgtga acagtgagct gtatgtgcgc agctcgcgtt gcattttcgg aagcgctcgt 660
   tttcggaaac gctttgaagt tcctattccg aagttcctat tctctagaaa gtataggaac 720
   ttcagagcgc ttttgaaaac caaaagcgct ctgaagacgc actttcaaaa aaccaaaaac 780
   gcaccggact gtaacgagct actaaatatt tgcgaatacc gcttccacaa acattgctca 840
   aaagtatctc tttgctatat atctctgtgc tatatcccta tataacctac ccatccacct 900
   ttcgctcctt gaacttgcat ctaaactcga cctctacatt ttttatgttt atctctagta 960
   ttactcttta gacaaaaaaa ttgtagtaag aactattcat agagtgaatc gaaaacaata 1020
   cgaaaatgta aacatttcct atacgtagta tatagagaca aaatagaaga aaccgttcat 1080
   aattttctga ccaatgaaga atcatcaacg ctatcacttt ctgttcacaa agtatgcgca 1140
   atccacatcg gtatagaata taatcgggga tgcctttatc ttgaaaaaat gcacccgcag 1200
   cttcgctagt aatcagtaaa cgcgggaagt ggagtcaggc tttttttatg gaagagaaaa 1260
   tagacaccaa agtagccttc ttctaacctt aacggaccta cagtgcaaaa agttatcaag 1320
   agactgcatt atagagcgca caaaggagaa aaaaagtaat ctaagatgct ttgttagaaa 1380
   aatagcgctc tcgggatgca tttttgtaga acaaaaaaga agtatagatt ctttgttggt 1440
   aaaatagcgc tctcgcgttg catttctgtt ctgtaaaaat gcagctcaga ttctttgttt 1500
   gaaaaattag cgctctcgcg ttgcattttt gttttacaaa aatgaagcac agattcttcg 1560
   ttggtaaaat agcgctttcg cgttgcattt ctgttctgta aaaatgcagc tcagattctt 1620
   tgtttgaaaa attagcgctc tcgcgttgca tttttgttct acaaaatgaa gcacagatgc 1680
   ttcgttaaca aagatatgct attgaagtgc aagatggaaa cgcagaaaat gaaccgggga 1740
   tgcgacgtgc aagattacct atgcaataga tgcaatagtt tctccaggaa ccgaaataca 1800
   tacattgtct tccgtaaagc gctagactat atattattat acaggttcaa atatactatc 1860
   tgtttcaggg aaaactccca ggttcggatg ttcaaaattc aatgatgggt aacaagtacg 1920
   atcgtaaatc tgtaaaacag tttgtcggat attaggctgt atctcctcaa agcgtattcg 1980
   aatatcattg agaagctgca gcgtcacatc ggataataat gatggcagcc attgtagaag 2040
   tgccttttgc atttctagtc tctttctcgg tctagctagt tttactacat cgcgaagata 2100
   gaatcttaga tcacactgcc tttgctgagc tggatcaata gagtaacaaa agagtggtaa 2160
   ggcctcgtta aaggacaagg acctgagcgg aagtgtatcg tacagtagac ggagtatact 2220
   agtatagtct atagtccgtg gaattctcat gtttgacagc ttatcatcga taagcttttc 2280
   aattcatcat ttttttttta ttcttttttt tgatttcggt ttccttgaaa tttttttgat 2340
   tcggtaatct ccgaacagaa ggaagaacga aggaaggagc acagacttag attggtatat 2400
   atacgcatat gtagtgttga agaaacatga aattgcccag tattcttaac ccaactgcac 2460
   agaacaaaaa cctgcaggaa acgaagataa atcatgtcga aagctacata taaggaacgt 2520
   gctgctactc atcctagtcc tgttgctgcc aagctattta atatcatgca cgaaaagcaa 2580
   acaaacttgt gtgcttcatt ggatgttcgt accaccaagg aattactgga gttagttgaa 2640
   gcattaggtc ccaaaatttg tttactaaaa acacatgtgg atatcttgac tgatttttcc 2700
   atggatccgg tgattgattg agcaagctaa ctgtgggaat actcaggtat cgtaagatgc 2760
   aagagttcga atctcttagc aaccattatt tttttcctca acataacgag aacacacagg 2820
   ggcgctatcg cacagaatca aattcgatga ctggaaattt tttgttaatt tcagaggtcg 2880
   cctgacgcat ataccttttt caactgaaaa attgggagaa aaaggaaagg tgagagcgcc 2940
   ggaaccggct tttcatatag aatagagaag cgttcatgac taaatgcttg catcacaata 3000
   cttgaagttg acaatattat ttaaggacct attgtttttt ccaataggtg gttagcaatc 3060
   gtcttacttt ctaacttttc ttacctttta catttcagca atatatatat atatttcaag 3120
   gatataccat tctaatgtct gcccctaaga agatcgtcgt tttgccaggt gaccacgttg 3180
   gtcaagaaat cacagccgaa gccattaagg ttcttaaagc tatttctgat gttcgttcca 3240
   atgtcaagtt cgatttcgaa aatcatttaa ttggtggtgc tgctatcgat gctacaggtg 3300
   ttccacttcc agatgaggcg ctggaagcct ccaagaaggc tgatgccgtt ttgttaggtg 3360
   ctgtgggtgg tcctaaatgg ggtaccggta gtgttagacc tgaacaaggt ttactaaaaa 3420
   atccgtaaag aacttcaatt gtacgccaac ttaagaccat gtaactttgc atccgactct 3480
   cttttagact tatctccaat caagccacaa tttgctaaag gtactgactt cgttgttgtc 3540
   agagaattag tgggaggtat ttactttggt aagagaaagg aagacgatgg tgatggtgtc 3600
   gcttgggata gtgaacaata caccgttcca gaagtgcaaa gaatcacaag aatggccgct 3660
   ttcatggccc tacaacatga gccaccattg cctatttggt ccttggataa agctaatgtt 3720
   ttggcctctt caagattatg gagaaaaact gtggaggaaa ccatcaagaa cgaattccct 3780
   acattgaagg ttcaacatca attgattgat tctgccgcca tgatcctagt taagaaccca 3840
   acccacctaa atggtattat aatcaccagc aacatgtttg gtgatatcat ctccgatgaa 3900
   gcctccgtta tcccaggttc cttgggtttg ttgccatctg cgtccttggc ctctttgcca 3960
   gacaagaaca ccgcatttgg tttgtacgaa ccatgccacg gttctgctcc agatttgcca 4020
   aagaataagg tcaaccctat cgccactatc ttgtctgctg caatgatgtt gaaattgtca 4080
   ttgaacttgc ctgaagaagg taaggccatt gaagatgcag ttaaaaaggt tttggatgca 4140
   ggtatcagaa ctggtgattt aggtggttcc aacagtacca ccgaagtcgg tgatgctgtc 4200
   gccgaagaag ttaagaaaat ccttgcttaa aaagattctc tttttttatg atatttgtac 4260
   ataaacttta taaatgaaat tcataataga aacgacacga aattacaaaa tggaatatgt 4320
   tcatagggta gagcttgctc aatcaatcac cggatccatg gagggcacag ttaagccgct 4380
   aaaggcatta tccgccaagt acaatttttt actcttcgaa gacagaaaat ttgctgacat 4440
   tggtaataca gtcaaattgc agtactctgc gggtgtatac agaatagcag aatgggcaga 4500
   cattacgaat gcacacggtg tggtgggccc aggtattgtt agcggtttga agcaggcggc 4560
   agaagaagta acaaaggaac ctagaggcct tttgatgtta gcagaattgt catgcaaggg 4620
   ctccctatct actggagaat atactaaggg tactgttgac attgcgaaga gcgacaaaga 4680
   ttttgttatc ggctttattg ctcaaagaga catgggtgga agagatgaag gttacgattg 4740
   gttgattatg acacccggtg tgggtttaga tgacaaggga gacgcattgg gtcaacagta 4800
   tagaaccgtg gatgatgtgg tctctacagg atctgacatt attattgttg gaagaggact 4860
   atttgcaaag ggaagggatg ctaaggtaga gggtgaacgt tacagaaaag caggctggga 4920
   agcatatttg agaagatgcg gccagcaaaa ctaaaaaact gtattataag taaatgcatg 4980
   tatactaaac tcacaaatta gagcttcaat ttaattatat cagttattac ccgggaatct 5040
   cggtcgtaat gatttttata atgacgaaaa aaaaaaaatt ggaaagaaaa agcttaatgc 5100
   ggtagtttat cacagttaaa ttgctaacgc agtcaggcac cgtgtatgaa atctaacaat 5160
   gcgctcatcg tcatcctcgg caccgtcacc ctggatgctg taggcatagg cttggttatg 5220
   ccggtactgc cgggcctctt gcgggatatc gtccattccg acagcatcgc cagtcactat 5280
   ggcgtgctgc tagcgctata tgcgttgatg caatttctat gcgcacccgt tctcggagca 5340
   ctgtccgacc gctttggccg ccgcccagtc ctgctcgctt cgctacttgg agccactatc 5400
   gactacgcga tcatggcgac cacacccgtc ctgtggatcc tctacgccgg acgcatcgtg 5460
   gccggcatca ccggcgccac aggtgcggtt gctggcgcct atatcgccga catcaccgat 5520
   ggggaagatc gggctcgcca cttcgggctc atgagcgctt gtttcggcgt gggtatggtg 5580
   gcaggccccg tggccggggg actgttgggc gccatctcct tgcatgcaac ttcttttctt 5640
   tttttttctt ttctctctcc cccgttgttg tctcaccata tccgcaatga caaaaaaatg 5700
   atggaagaca ctaaaggaaa aaattaacga caaagacagc accaacagat gtcgttgttc 5760
   cagagctgat gaggggtatc tcgaagcaca cgaaactttt tccttccttc attcacgcac 5820
   actactctct aatgagcaac ggtatacggc cttccttcca gttacttgaa tttgaaataa 5880
   aaaaaagttt gctgtcttgc tatcaagtat aaatagacct gcaattatta atcttttgtt 5940
   tcctcgtcat tgttctcgtt ccctttcttc cttgtttctt tttctgcaca atatttcaag 6000
   ctataccaag catacaatca aggaattcga gctaagcggc cgcaaagatc taactcgaga 6060
   aacgcgttag agctaagcgg ccgcaaagat ctaactcgag aaacgcgtta gagctaagcg 6120
   gccgcaaaga tctaactcga gaaacgcgtt agagctcggt acccggggat cctctagagt 6180
   cgacgctctc ccttatgcga ctcctgcatt aggaagcagc ccagtagtag gttgaggccg 6240
   ttgagcaccg ccgccgcaag gaatggtgca tgcaaggaga tggcgcccaa cagtcccccg 6300
   gccacggggc ctgccaccat acccacgccg aaacaagcgc tcatgagccc gaagtggcga 6360
   gcccgatctt ccccatcggt gatgtcggcg atataggcgc cagcaaccgc acctgtggcg 6420
   ccggtgatgc cggccacgat gcgtccggcg tagaggatct tttatgcttg cttttcaaaa 6480
   ggcctgcagg caagtgcaca aacaatactt aaataaatac tactcagtaa taacctattt 6540
   cttagcattt ttgacgaaat ttgctatttt gttagagtct tttacaccat ttgtctccac 6600
   acctccgctt acatcaacac caataacgcc atttaatcta agcgcatcac caacattttc 6660
   tggcgtcagt ccaccagcta acataaaatg taagcttgca tgcctgcagg tcgaccgatg 6720
   cccttgagag ccttcaaccc agtcagctcc ttccggtggg cgcggggcat gactatcgtc 6780
   gccgcactta tgactgtctt ctttatcatg caactcgtag gacaggtgcc ggcagcgctc 6840
   tgggtcattt tcggcgagga ccgctttcgc tggagcgcga cgatgatcgg cctgtcgctt 6900
   gcggtattcg gaatcttgca cgccctcgct caagccttcg tcactggtcc cgccaccaaa 6960
   cgtttcggcg agaagcaggc cattatcgcc ggcatggcgg ccgacgcgct gggctacgtc 7020
   ttgctggcgt tcgcgacgcg aggctggatg gccttcccca ttatgattct tctcgcttcc 7080
   ggcggcatcg ggatgcccgc gttgcaggcc atgctgtcca ggcaggtaga tgacgaccat 7140
   cagggacagc ttcaaggatc gctcgcggct cttaccagcc taacttcgat cattggaccg 7200
   ctgatcgtca cggcgattta tgccgcctcg gcgagcacat ggaacgggtt ggcatggatt 7260
   gtaggcgccg ccctatacct tgtctgcctc cccgcgttgc gtcgcggtgc atggagccgg 7320
   gccacctcga cctgaatgga agccggcggc acctcgctaa cggattcacc actccaagaa 7380
   ttggagccaa tcaattcttg cggagaactg tgaatgcgca aaccaaccct tggcagaaca 7440
   tatccatcgc gtccgccatc tccagcagcc gcacgcggcg catctcgggc agcgttgggt 7500
   cctggccacg ggtgcgcatg atcgtgctcc tgtcgttgag gacccggcta ggctggcggg 7560
   gttgccttac tggttagcag aatgaatcac cgatacgcga gcgaacgtga agcgactgct 7620
   gctgcaaaac gtctgcgacc tgagcaacaa catgaatggt cttcggtttc cgtgtttcgt 7680
   aaagtctgga aacgcggaag tcagcgccct gcaccattat gttccggatc tgcatcgcag 7740
   gatgctgctg gctaccctgt ggaacaccta catctgtatt aacgaagcgc tggcattgac 7800
   cctgagtgat ttttctctgg tcccgccgca tccataccgc cagttgttta ccctcacaac 7860
   gttccagtaa ccgggcatgt tcatcatcag taacccgtat cgtgagcatc ctctctcgtt 7920
   tcatcggtat cattaccccc atgaacagaa atccccctta cacggaggca tcagtgacca 7980
   aacaggaaaa aaccgccctt aacatggccc gctttatcag aagccagaca ttaacgcttc 8040
   tggagaaact caacgagctg gacgcggatg aacaggcaga catctgtgaa tcgcttcacg 8100
   accacgctga tgagctttac cgcagctgcc tcgcgcgttt cggtgatgac ggtgaaaacc 8160
   tctgacacat gcagctcccg gagacggtca cagcttgtct gtaagcggat gccgggagca 8220
   gacaagcccg tcagggcgcg tcagcgggtg ttggcgggtg tcggggcgca gccatgaccc 8280
   agtcacgtag cgatagcgga gtgtatactg gcttaactat gcggcatcag agcagattgt 8340
   actgagagtg caccatatgc ggtgtgaaat accgcacaga tgcgtaagga gaaaataccg 8400
   catcaggcgc tcttccgctt cctcgctcac tgactcgctg cgctcggtcg ttcggctgcg 8460
   gcgagcggta tcagctcact caaaggcggt aatacggtta tccacagaat caggggataa 8520
   cgcaggaaag aacatgtgag caaaaggcca gcaaaaggcc aggaaccgta aaaaggccgc 8580
   gttgctggcg tttttccata ggctccgccc ccctgacgag catcacaaaa atcgacgctc 8640
   aagtcagagg tggcgaaacc cgacaggact ataaagatac caggcgtttc cccctggaag 8700
   ctccctcgtg cgctctcctg ttccgaccct gccgcttacc ggatacctgt ccgcctttct 8760
   cccttcggga agcgtggcgc tttctcatag ctcacgctgt aggtatctca gttcggtgta 8820
   ggtcgttcgc tccaagctgg gctgtgtgca cgaacccccc gttcagcccg accgctgcgc 8880
   cttatccggt aactatcgtc ttgagtccaa cccggtaaga cacgacttat cgccactggc 8940
   agcagccact ggtaacagga ttagcagagc gaggtatgta ggcggtgcta cagagttctt 9000
   gaagtggtgg cctaactacg gctacactag aaggacagta tttggtatct gcgctctgct 9060
   gaagccagtt accttcggaa aaagagttgg tagctcttga tccggcaaac aaaccaccgc 9120
   tggtagcggt ggtttttttg tttgcaagca gcagattacg cgcagaaaaa aaggatctca 9180
   agaagatcct ttgatctttt ctacggggtc tgacgctcag tggaacgaaa actcacgtta 9240
   agggattttg gtcatgagat tatcaaaaag gatcttcacc tagatccttt taaattaaaa 9300
   atgaagtttt aaatcaatct aaagtatata tgagtaaact tggtctgaca gttaccaatg 9360
   cttaatcagt gaggcaccta tctcagcgat ctgtctattt cgttcatcca tagttgcctg 9420
   actccccgtc gtgtagataa ctacgatacg ggagggctta ccatctggcc ccagtgctgc 9480
   aatgataccg cgagacccac gctcaccggc tccagattta tcagcaataa accagccagc 9540
   cggaagggcc gagcgcagaa gtggtcctgc aactttatcc gcctccatcc agtctattaa 9600
   ttgttgccgg gaagctagag taagtagttc gccagttaat agtttgcgca acgttgttgc 9660
   cattgctgca ggcatcgtgg tgtcacgctc gtcgtttggt atggcttcat tcagctccgg 9720
   ttcccaacga tcaaggcgag ttacatgatc ccccatgttg tgcaaaaaag cggttagctc 9780
   cttcggtcct ccgatcgttg tcagaagtaa gttggccgca gtgttatcac tcatggttat 9840
   ggcagcactg cataattctc ttactgtcat gccatccgta agatgctttt ctgtgactgg 9900
   tgagtactca accaagtcat tctgagaata gtgtatgcgg cgaccgagtt gctcttgccc 9960
   ggcgtcaaca cgggataata ccgcgccaca tagcagaact ttaaaagtgc tcatcattgg 10020
   aaaacgttct tcggggcgaa aactctcaag gatcttaccg ctgttgagat ccagttcgat 10080
   gtaacccact cgtgcaccca actgatcttc agcatctttt actttcacca gcgtttctgg 10140
   gtgagcaaaa acaggaaggc aaaatgccgc aaaaaaggga ataagggcga cacggaaatg 10200
   ttgaatactc atactcttcc tttttcaata ttattgaagc atttatcagg gttattgtct 10260
   catgagcgga tacatatttg aatgtattta gaaaaataaa caaatagggg ttccgcgcac 10320
   atttccccga aaagtgccac ctgacgtcta agaaaccatt attatcatga cattaaccta 10380
   taaaaatagg cgtatcacga ggccctttcg tcttcaa 10417

<210> 20
<211> 9966
<212> DNA
<213> pFlat4

<400> 20
gaattctgaa ccagtcctaa aacgagtaaa taggaccggc aattcttcaa gcaataaaca 60

ggaataccaa ttattaaaag ataacttagt cagatcgtac aataaagctt tgaagaaaaa 120

tgcgccttat tcaatctttg ctataaaaaa tggcccaaaa tctcacattg gaagacattt 180

gatgacctca tttctttcaa tgaagggcct aacggagttg actaatgttg tgggaaattg 240

gagcgataag cgtgcttctg ccgtggccag gacaacgtat actcatcaga taacagcaat 300

acctgatcac tacttcgcac tagtttctcg gtactatgca tatgatccaa tatcaaagga 360

aatgatagca ttgaaggatg agactaatcc aattgaggag tggcagcata tagaacagct 420

aaagggtagt gctgaaggaa gcatacgata ccccgcatgg aatgggataa tatcacagga 480

ggtactagac tacctttcat cctacataaa tagacgcata taagtacgca tttaagcata 540

aacacgcact atgccgttct tctcatgtat atatatatac aggcaacacg cagatatagg 600

tgcgacgtga acagtgagct gtatgtgcgc agctcgcgtt gcattttcgg aagcgctcgt 660

tttcggaaac gctttgaagt tcctattccg aagttcctat tctctagaaa gtataggaac 720

ttcagagcgc ttttgaaaac caaaagcgct ctgaagacgc actttcaaaa aaccaaaaac 780

gcaccggact gtaacgagct actaaaatat tgcgaatacc gcttccacaa acattgctca 840

aaagtatctc tttgctatat atctctgtgc tatatcccta tataacctac ccatccacct 900

ttcgctcctt gaacttgcat ctaaactcga cctctacatt ttttatgttt atctctagta 960

ttactcttta gacaaaaaaa ttgtagtaag aactattcat agagtgaatc gaaaacaata 1020

cgaaaatgta aacatttcct atacgtagta tatagagaca aaatagaaga aaccgttcat 1080

aattttctga ccaatgaaga atcatcaacg ctatcacttt ctgttcacaa agtatgcgca 1140

atccacatcg gtatagaata taatcgggga tgcctttatc ttgaaaaaat gcacccgcag 1200

cttcgctagt aatcagtaaa cgcgggaagt ggagtcaggc tttttttatg gaagagaaaa 1260

tagacaccaa agtagccttc ttctaacctt aacggaccta cagtgcaaaa agttatcaag 1320

agactgcatt atagagcgca caaaggagaa aaaaagtaat ctaagatgct ttgttagaaa 1380

aatagcgctc tcgggatgca tttttgtaga acaaaaaaga agtatagatt ctttgttggt 1440

aaaatagcgc tctcgcgttg catttctgtt ctgtaaaaat gcagctcaga ttctttgttt 1500

gaaaaattag cgctctcgcg ttgcattttt gttttacaaa aatgaagcac agattcttcg 1560

ttggtaaaat agcgctttcg cgttgcattt ctgttctgta aaaatgcagc tcagattctt 1620

tgtttgaaaa attagcgctc tcgcgttgca tttttgttct acaaaatgaa gcacagatgc 1680

ttcgttaaca aagatatgct attgaagtgc aagatggaaa cgcagaaaat gaaccgggga 1740

tgcgacgtgc aagattacct atgcaataga tgcaatagtt tctccaggaa ccgaaataca 1800

tacattgtct tccgtaaagc gctagactat atattattat acaggttcaa atatactatc 1860

tgtttcaggg aaaactccca ggttcggatg ttcaaaattc aatgatgggt aacaagtacg 1920

atcgtaaatc tgtaaaacag tttgtcggat attaggctgt atctcctcaa agcgtattcg 1980

aatatcattg agaagctgca gcgtcacatc ggataataat gatggcagcc attgtagaag 2040

tgccttttgc atttctagtc tctttctcgg tctagctagt tttactacat cgcgaagata 2100

gaatcttaga tcacactgcc tttgctgagc tggatcaata gagtaacaaa agagtggtaa 2160

ggcctcgtta aaggacaagg acctgagcgg aagtgtatcg tacagtagac ggagtatact 2220

agtatagtct atagtccgtg gaattctcat gtttgacagc ttatcatcga taagcttttc 2280

aattcatcat ttttttttta ttcttttttt tgatttcggt ttccttgaaa tttttttgat 2340

tcggtaatct ccgaacagaa ggaagaacga aggaaggagc acagacttag attggtatat 2400

atacgcatat gtagtgttga agaaacatga aattgcccag tattcttaac ccaactgcac 2460

agaacaaaaa cctgcaggaa acgaagataa atcatgtcga aagctacata taaggaacgt 2520

gctgctactc atcctagtcc tgttgctgcc aagctattta atatcatgca cgaaaagcaa 2580

acaaacttgt gtgcttcatt ggatgttcgt accaccaagg aattactgga gttagttgaa 2640

gcattaggtc ccaaaatttg tttactaaaa acacatgtgg atatcttgac tgatttttcc 2700

atggatccgg tgattgattg agcaagcttc gagttcaaga gaaaaaaaag aaaaagcaaa 2760

aagaaaaaag gaaagcgcgc ctcgttcaga atgacacgta tagaatgatg cattaccttg 2820

tcatcttcag tatcatactg ttcgtataca tacttactga cattcatagg tatacatata 2880

tacacatgta tatatatcgt atgctgcagc tttaaataat cggtgtcact acataagaac 2940

acctttggtg gagggaacat cgttggtacc attgggcgag gtggcttctc ttatggcaac 3000

cgcaagagcc ttgaacgcac tctcactacg gtgatgatca ttcttgcctc gcagacaatc 3060

aacgtggagg gtaattctgc tagcctctgc aaagctttca agaaaatgcg ggatcatctc 3120

gcaagagaga tctcctactt tctccctttg caaaccaagt tcgacaactg cgtacggcct 3180

gttcgaaaga tctaccaccg ctctggaaag tgcctcatcc aaaggcgcaa atcctgatcc 3240

aaaccttttt actccacgca cggcccctag ggcctcttta aaagcttgac cgagagcaat 3300

cccgcagtct tcagtggtgt gatggtcgtc tatgtgtaag tcaccaatgc actcaacgat 3360

tagcgaccag ccggaatgct tggccagagc atgtatcata tggtccagaa accctatacc 3420

tgtgtggacg ttaatcactt gcgattgtgt ggcctgttct gctactgctt ctgcctcttt 3480

ttctgggaag atcgagtgct ctatcgctag gggaccaccc tttaaagaga tcgcaatctg 3540

aatcttggtt tcatttgtaa tacgctttac tagggctttc tgctctgtca tctttgcctt 3600

cgtttatctt gcctgctcat tttttagtat attcttcgaa gaaatcacat tactttatat 3660

aatgtataat tcattatgtg ataatgccaa tcgctaagaa aaaaaaagag tcatccgcta 3720

ggtggaaaaa aaaaaatgaa aatcattacc gaggcataaa aaaatataga gtgtactaga 3780

ggaggccaag agtaatagaa aaagaaaatt gcgggaaagg actgtgttat gacttccctg 3840

actaatgccg tgttcaaacg atacctggca gtgactccta agcttgctca atcaatcacc 3900

ggatccatgg agggcacagt taagccgcta aaggcattat ccgccaagta caatttttta 3960

ctcttcgaag acagaaaatt tgctgacatt ggtaatacag tcaaattgca gtactctgcg 4020

ggtgtataca gaatagcaga atgggcagac attacgaatg cacacggtgt ggtgggccca 4080

ggtattgtta gcggtttgaa gcaggcggca gaagaagtaa caaaggaacc tagaggcctt 4140

ttgatgttag cagaattgtc atgcaagggc tccctatcta ctggagaata tactaagggt 4200

actgttgaca ttgcgaagag cgacaaagat tttgttatcg gctttattgc tcaaagagac 4260

atgggtggaa gagatgaagg ttacgattgg ttgattatga cacccggtgt gggtttagat 4320

gacaagggag acgcattggg tcaacagtat agaaccgtgg atgatgtggt ctctacagga 4380

tctgacatta ttattgttgg aagaggacta tttgcaaagg gaagggatgc taaggtagag 4440

ggtgaacgtt acagaaaagc aggctgggaa gcatatttga gaagatgcgg ccagcaaaac 4500

taaaaaactg tattataagt aaatgcatgt atactaaact cacaaattag agcttcaatt 4560

taattatatc agttattacc cgggaatctc ggtcgtaatg atttttataa tgacgaaaaa 4620

aaaaaaattg gaaagaaaaa gcttaatgcg gtagtttatc acagttaaat tgctaacgca 4680

gtcaggcacc gtgtatgaaa tctaacaatg cgctcatcgt catcctcggc accgtcaccc 4740

tggatgctgt aggcataggc ttggttatgc cggtactgcc gggcctcttg cgggatatcg 4800

tccattccga cagcatcgcc agtcactatg gcgtgctgct agcgctatat gcgttgatgc 4860

aatttctatg cgcacccgtt ctcggagcac tgtccgaccg ctttggccgc cgcccagtcc 4920

tgctcgcttc gctacttgga gccactatcg actacgcgat catggcgacc acacccgtcc 4980

tgtggatcct ctacgccgga cgcatcgtgg ccggcatcac cggcgccaca ggtgcggttg 5040

ctggcgccta tatcgccgac atcaccgatg gggaagatcg ggctcgccac ttcgggctca 5100

tgagcgcttg tttcggcgtg ggtatggtgg caggccccgt ggccggggga ctgttgggcg 5160

ccatctcctt gcatgcaact tcttttcttt tttttctttt tctctctccc ccgttgttgt 5220

ctcaccatat ccgcaatgac aaaaaaatga tggaagacac taaaggaaaa aattaacgac 5280

aaagacagca ccaacagatg tcgttgttcc agagctgatg aggggtatct cgaagcacac 5340

gaaacttttt ccttccttca ttcacgcaca ctactctcta atgagcaacg gtatacggcc 5400

ttccttccag ttacttgaat ttgaaataaa aaaaagtttg ctgtcttgct atcaagtata 5460

aatagacctg caattattaa tcttttgttt cctcgtcatt gttctcgttc cctttcttcc 5520

ttgtttcttt ttctgcacaa tatttcaagc tataccaagc atacaatcaa ggaattcgag 5580

ctaagcggcc gcaaagatct aactcgagaa acgcgttaga gctaagcggc cgcaaagatc 5640

taactcgaga aacgcgttag agctaagcgg ccgcaaagat ctaactcgag aaacgcgtta 5700

gagctcggta cccggggatc ctctagagtc gacgctctcc cttatgcgac tcctgcatta 5760

ggaagcagcc cagtagtagg ttgaggccgt tgagcaccgc cgccgcaagg aatggtgcat 5820

gcaaggagat ggcgcccaac agtcccccgg ccacggggcc tgccaccata cccacgccga 5880

aacaagcgct catgagcccg aagtggcgag cccgatcttc cccatcggtg atgtcggcga 5940

tataggcgcc agcaaccgca cctgtggcgc cggtgatgcc ggccacgatg cgtccggcgt 6000

agaggatctt ttatgcttgc ttttcaaaag gcctgcaggc aagtgcacaa acaatactta 6060

aataaatact actcagtaat aacctatttc ttagcatttt tgacgaaatt tgctattttg 6120

ttagagtctt ttacaccatt tgtctccaca cctccgctta catcaacacc aataacgcca 6180

tttaatctaa gcgcatcacc aacattttct ggcgtcagtc caccagctaa cataaaatgt 6240

aagcttgcat gcctgcaggt cgaccgatgc ccttgagagc cttcaaccca gtcagctcct 6300

tccggtgggc gcggggcatg actatcgtcg ccgcacttat gactgtcttc tttatcatgc 6360

aactcgtagg acaggtgccg gcagcgctct gggtcatttt cggcgaggac cgctttcgct 6420

ggagcgcgac gatgatcggc ctgtcgcttg cggtattcgg aatcttgcac gccctcgctc 6480

aagccttcgt cactggtccc gccaccaaac gtttcggcga gaagcaggcc attatcgccg 6540

gcatggcggc cgacgcgctg ggctacgtct tgctggcgtt cgcgacgcga ggctggatgg 6600

ccttccccat tatgattctt ctcgcttccg gcggcatcgg gatgcccgcg ttgcaggcca 6660

tgctgtccag gcaggtagat gacgaccatc agggacagct tcaaggatcg ctcgcggctc 6720

ttaccagcct aacttcgatc attggaccgc tgatcgtcac ggcgatttat gccgcctcgg 6780

cgagcacatg gaacgggttg gcatggattg taggcgccgc cctatacctt gtctgcctcc 6840

ccgcgttgcg tcgcggtgca tggagccggg ccacctcgac ctgaatggaa gccggcggca 6900

cctcgctaac ggattcacca ctccaagaat tggagccaat caattcttgc ggagaactgt 6960

gaatgcgcaa accaaccctt ggcagaacat atccatcgcg tccgccatct ccagcagccg 7020

cacgcggcgc atctcgggca gcgttgggtc ctggccacgg gtgcgcatga tcgtgctcct 7080

gtcgttgagg acccggctag gctggcgggg ttgccttact ggttagcaga atgaatcacc 7140

gatacgcgag cgaacgtgaa gcgactgctg ctgcaaaacg tctgcgacct gagcaacaac 7200

atgaatggtc ttcggtttcc gtgtttcgta aagtctggaa acgcggaagt cagcgccctg 7260

caccattatg ttccggatct gcatcgcagg atgctgctgg ctaccctgtg gaacacctac 7320

atctgtatta acgaagcgct ggcattgacc ctgagtgatt tttctctggt cccgccgcat 7380

ccataccgcc agttgtttac cctcacaacg ttccagtaac cgggcatgtt catcatcagt 7440

aacccgtatc gtgagcatcc tctctcgttt catcggtatc attaccccca tgaacagaaa 7500

tcccccttac acggaggcat cagtgaccaa acaggaaaaa accgccctta acatggcccg 7560

ctttatcaga agccagacat taacgcttct ggagaaactc aacgagctgg acgcggatga 7620

acaggcagac atctgtgaat cgcttcacga ccacgctgat gagctttacc gcagctgcct 7680

cgcgcgtttc ggtgatgacg gtgaaaacct ctgacacatg cagctcccgg agacggtcac 7740

agcttgtctg taagcggatg ccgggagcag acaagcccgt cagggcgcgt cagcgggtgt 7800

tggcgggtgt cggggcgcag ccatgaccca gtcacgtagc gatagcggag tgtatactgg 7860

cttaactatg cggcatcaga gcagattgta ctgagagtgc accatatgcg gtgtgaaata 7920

ccgcacagat gcgtaaggag aaaataccgc atcaggcgct cttccgcttc ctcgctcact 7980

gactcgctgc gctcggtcgt tcggctgcgg cgagcggtat cagctcactc aaaggcggta 8040

atacggttat ccacagaatc aggggataac gcaggaaaga acatgtgagc aaaaggccag 8100

caaaaggcca ggaaccgtaa aaaggccgcg ttgctggcgt ttttccatag gctccgcccc 8160

cctgacgagc atcacaaaaa tcgacgctca agtcagaggt ggcgaaaccc gacaggacta 8220

taaagatacc aggcgtttcc ccctggaagc tccctcgtgc gctctcctgt tccgaccctg 8280

ccgcttaccg gatacctgtc cgcctttctc ccttcgggaa gcgtggcgct ttctcatagc 8340

tcacgctgta ggtatctcag ttcggtgtag gtcgttcgct ccaagctggg ctgtgtgcac 8400

gaaccccccg ttcagcccga ccgctgcgcc ttatccggta actatcgtct tgagtccaac 8460

ccggtaagac acgacttatc gccactggca gcagccactg gtaacaggat tagcagagcg 8520

aggtatgtag gcggtgctac agagttcttg aagtggtggc ctaactacgg ctacactaga 8580

aggacagtat ttggtatctg cgctctgctg aagccagtta ccttcggaaa aagagttggt 8640

agctcttgat ccggcaaaca aaccaccgct ggtagcggtg gtttttttgt ttgcaagcag 8700

cagattacgc gcagaaaaaa aggatctcaa gaagatcctt tgatcttttc tacggggtct 8760

gacgctcagt ggaacgaaaa ctcacgttaa gggattttgg tcatgagatt atcaaaaagg 8820

atcttcacct agatcctttt aaattaaaaa tgaagtttta aatcaatcta aagtatatat 8880

gagtaaactt ggtctgacag ttaccaatgc ttaatcagtg aggcacctat ctcagcgatc 8940

tgtctatttc gttcatccat agttgcctga ctccccgtcg tgtagataac tacgatacgg 9000

gagggcttac catctggccc cagtgctgca atgataccgc gagacccacg ctcaccggct 9060

ccagatttat cagcaataaa ccagccagcc ggaagggccg agcgcagaag tggtcctgca 9120

actttatccg cctccatcca gtctattaat tgttgccggg aagctagagt aagtagttcg 9180

ccagttaata gtttgcgcaa cgttgttgcc attgctgcag gcatcgtggt gtcacgctcg 9240

tcgtttggta tggcttcatt cagctccggt tcccaacgat caaggcgagt tacatgatcc 9300

cccatgttgt gcaaaaaagc ggttagctcc ttcggtcctc cgatcgttgt cagaagtaag 9360

ttggccgcag tgttatcact catggttatg gcagcactgc ataattctct tactgtcatg 9420

ccatccgtaa gatgcttttc tgtgactggt gagtactcaa ccaagtcatt ctgagaatag 9480

tgtatgcggc gaccgagttg ctcttgcccg gcgtcaacac gggataatac cgcgccacat 9540

agcagaactt taaaagtgct catcattgga aaacgttctt cggggcgaaa actctcaagg 9600

atcttaccgc tgttgagatc cagttcgatg taacccactc gtgcacccaa ctgatcttca 9660

gcatctttta ctttcaccag cgtttctggg tgagcaaaaa caggaaggca aaatgccgca 9720

aaaaagggaa taagggcgac acggaaatgt tgaatactca tactcttcct ttttcaatat 9780

tattgaagca tttatcaggg ttattgtctc atgagcggat acatatttga atgtatttag 9840

aaaaataaac aaataggggt tccgcgcaca tttccccgaa aagtgccacc tgacgtctaa 9900

gaaaccatta ttatcatgac attaacctat aaaaataggc gtatcacgag gccctttcgt 9960

cttcaa 9966

## Description

The present invention relates to a method of providing an organism belonging to the genus Saccharomyces, which is capable of producing polyunsaturated fatty acids (PUFAs) comprising the steps of subjecting such an organism, which does not produce PUFAs, to a mutagenesis step and selecting a Saccharomyces organism, which is capable of accumulating at least 15% of the cellular dry weight as lipids; and genetically modifying the Saccharomyces organism selected in step (a) so that it is capable of synthesizing PUFAs, in particular eicosapentaenoic acid and/or docosahexaenoic acid. The invention also relates to organisms obtained by such a method, as well as a method for the synthesis of PUFAs, in particular eicosapentaenoic acid and/or docosahexaenoic acid comprising culturing such an organism.

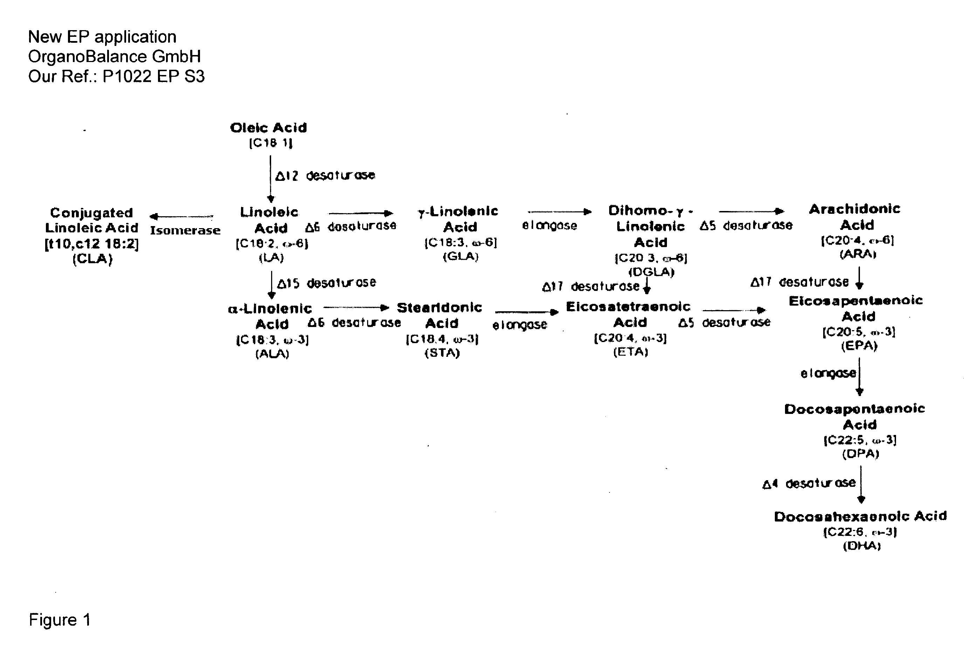

A mammal, and in particular a human can produce all but two of the fatty acids it needs. These two, linoleic acid (LA) and alpha-linolenic acid (ALA), are widely distributed in plant oils. In addition, fish oils contain the longer-chain omega-3 fatty acids eicosapentaenoic acid (EPA) and docosahexaenoic acid (DHA). Other marine oils, such as from seal, also contain significant amounts of docosapentaenoic acid (DPA), which is also an omega-3 fatty acid. These fatty acids are typically designated as polyunsaturated fatty acids (PUFAs), i.e. as fatty acids comprising at least 18 C atoms and at least 2 double bonds. The group of biologically important PUFAs comprises linoleic acid, conjugated linoleic acid (CLA), gamma-linolenic acid (GLA), dihomo-gamma-linolenic acid (DGLA), arachidonic acid (ARA), alpha-linolenic acid, stearidonic acid (STA), eicosatetraenoic acid (ETA), eicosapentaenoic acid, docosapentaenoic acid (DPA) and docosahexaenoic acid. Linoleic acid, conjugated linoleic acid, alpha-linolenic acid, stearidonic acid, eicosatetraenoic acid, eicosapentaenoic acid, docosapentaenoic acid and docosahexaenoic acid are examples of omega-3 fatty acids; wheras linoleic acid, gamma-linolenic acid, dihomo-gamma-linolenic acid and arachidonic acid are examples of omega-6 fatty acids (see also Fig. 1). As omega-3 or omega-6 bond is understood a double bond located on the 3^{rd} or 6^{th} carbon-carbon bond, respectively, counting from the ω, (methyl carbon) end of the chain.

Since the mammal body, when provided with LA or ALA, to some extent can convert these fatty acids into longer-chain omega-3 or omega-6 fatty acids, linoleic acid and alpha-linolenic acid are considered, in senso strictu, as essential fatty acids and therefore must be obtained from the diet.

In the recent past it has become evident that PUFAs are important in several human body systems, including the immune system and in blood pressure regulation. PUFAs are mainly present in cellular plasmamembranes in the form of phospholipids or triglycerides. They are also important during the development of the brain as well as during synthesis and repair processes of tisssue. For example, the brain shows increased amounts of linolenic and alpha-linoleic acid derivatives. Changes in the levels and balance of these fatty acids due to a typical Western diet rich in omega-6 and poor in omega-3 fatty acids is alleged to be associated with depression and behavioral change, including violence. Further, changing to a diet richer in omega-3 fatty acids, or consumption of supplements to compensate for a dietary imbalance, has been associated with reduced violent behavior and increased attention span. In addition PUFAS constitute starting points for the synthesis of further biologically important compounds like prostaglandins, prostacylins, thromboxanes or leukotrienes and they play an important role in the life and death of cardiac cells since they are essential fuels for mechanical and electrical activities of the heart.
In modern medical treatments PUFAs are used for the prevention and treatment of cardiovascular diseases, asthma, hypertension, elevated cholesterol levels, cancer or diabetes. Recently, well documented research has further indicated the importance of DHA in normal neurological development of infants. Based on these data the FAO/WHO has recommended the inclusion of supplemental DHA (and ARA) in both preterm and full term infant formulas.
PUFAs are currently extracted from plant seeds, algae or fish. However, although oils derived from fatty fish like herring, mackerel, sardine or salmon contain a large amount of PUFAs, in particular DHA and EPA, these oils are often unsuitable for human consumption or for inclusion in infant formula. Furthermore, such extraction approaches are generally associated with complex purification procedures, which, especially if carried out on an industrial scale, are expensive, laborious and tend to pollute the environment.

Thus, there is a need for means and methods allowing to produce PUFAs in a pure and cost neutral form, which also allow large scale and automated process formats.

The present invention addresses this need and presents a method of providing an organism belonging to the genus Saccharomyces, which is capable of producing PUFAs as well as organisms obtained by such a method. The use of Saccharomyces cells for the production of PUFAs allows for cheap, large scale and automated synthesis formats of these compounds. In particular, the present invention provides the embodiments as characterized in the claims.

Accordingly, the present invention in a first aspect relates to a method of providing an organism belonging to the genus Saccharomyces, which is capable of producing polyunsaturated fatty acids (PUFAs) comprising the steps of: (a) subjecting a Saccharomyces organism, which does not produce PUFAs, to a mutagenesis step and selecting a Saccharomyces organism, which is capable of accumulating at least 15% of the cellular dry weight as lipids; and (b) optionally genetically modifying the Saccharomyces organism selected in step (a) so that it is capable of synthesizing PUFAs. In a preferred embodiment the method comprises both steps (a) and (b) as defined above. The present invention also relates to a Saccharomyces organism obtainable or obtained by the method according to the invention.

The inventors surprisingly found that yeast cells of the genus Saccharomyces can be provided, which are capable of accumulating at least 15% of the cellular dry weight as lipids and which are further capable synthesizing and accumulating polyunsaturated fatty acids (PUFAS), which are not naturally produced by such organisms, if the organism is genetically modified.

The term "an organism belonging to the genus "Saccharomyces" means any organism belonging to the genus Saccharomyces. This includes organisms of the species *Saccharomyces cerevisiae, Saccharomyces delbrückii, Saccharomyces italicus, Saccharomyces ellipsoideus, Saccharomyces fermentati, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum or Saccharomycodes ludwigii.* In a preferred embodiment the organism is of the species Saccharomyces cerevisiae.

The term "polyunsaturated fatty acids (PUFAs)" relates to fatty acids comprising at least 18 C atoms and at least 2 double bonds, more preferably the term relates to omega-3 and omega-6 fatty acids comprising at least 18 C atoms, even more preferably the term relates to linoleic acid, conjugated linoleic acid (CLA), gamma-linolenic acid (GLA), dihomo-gamma-linolenic acid (DGLA), arachidonic acid (ARA), alpha-linolenic acid, stearidonic acid (STA), eicosatetraenoic acid (ETA), eicosapentaenoic acid, docosapentaenoic acid (DPA) or docosahexaenoic acid (DHA). The terms "omega-3" and "omega-6" relates to double bonds in a fatty acid, which are located on the 3^{rd} or 6^{th} carbon-carbon bond, respectively, counting from the ω, (methyl carbon) end of the fatty acid molecule.

The term "Saccharomyces organism, which does not produce PUFAs" relates to a Saccharomyces organism, which does not synthesize or accumulate fatty acids comprising at least 18 C atoms and at least 2 double bonds, as defined herein above. More preferably, the term relates to a Saccharomyces organism, which does not synthesize or accumulate omega-3 and omega-6 fatty acids comprising at least 18 C atoms and at least 2 double bonds, even more preferably, the term relates to a Saccharomyces organism, which does not synthesize or accumulate any of linoleic acid, conjugated linoleic acid (CLA), gamma-linolenic acid (GLA), dihomo-gamma-linolenic acid (DGLA), arachidonic acid (ARA), alpha-linolenic acid, stearidonic acid (STA), eicosatetraenoic acid (ETA), eicosapentaenoic acid, docosapentaenoic acid (DPA) or docosahexaenoic acid (DHA). In a further embodiment, the term also relates to a Saccharomyces organism, which is solely capable of synthesizing oleic acid, but is not capable of further derivatizing said oleic acid into polyunsaturated fatty acids by the introduction of additional double bonds.

The term "a Saccharomyces organism, which does not produce PUFAs" preferably means a Saccharomyces organism which does not produce more PUFAs than a naturally occurring Saccharomyces organism, more preferably a Saccharomyces organism which accumulates less than 3%, preferably less than 2%, even more preferably less than 1% and most preferably 0% of its cellular dry weight as PUFAs. Thus, in a preferred embodiment the Saccharomyces organism used in step (a) of the method according to the invention is a naturally occurring Saccharomyces organism which is not capable of producing PUFAs, i.e. does not contain the enzymatic activities for synthesizing PUFAs.

The term "subjecting a Saccharomyces organism which does not produce PUFAs to a mutagenesis step" means that a Saccharomyces strain as defined herein above is mutagenised according to any suitable means known to the person skilled in the art. In principle any known mutagenesis method can be employed in step (a) of the method according to the invention, e.g. directed or undirected mutagenesis methods. Preferred are undirected mutagenesis methods. Examples for suitable mutagenesis methods are transposon mutagenesis, mutagenesis methods using physical means, such as irradiation, in particular ionising irradiation, e.g. x-rays, alpha-, beta-, or gamma-rays, and UV light; mutagenesis methods using DNA damaging compounds, e.g. chemical mutagens, such as ethylnitrosourea (ENS), ethyl-methane-sulfonate (EMS), 5-bromo uracil, HNO₂, N-methyl-N'-nitro-N-nitrosoguanidin (MNNG), nitrosamines, mutagenesis methods using viral mutagens, such as viruses, e.g. retroviruses. Preferably, an EMS (ethyl-methane-sulfonate) mutagenesis as known to the person skilled in the art is performed. More preferably, the term relates to a mutagenesis, e.g. an EMS mutagenesis, combined with the treatment of the cells with any suitable compound that inhibits the biosynthesis of sterol and/or fatty acids known to the person skilled in the art. Even more preferably, the term relates to mutagenesis, e.g. an EMS mutagenesis, combined with the treatment of the cells with an agent, e.g. an antifungal antibiotic, that inhibits the biosynthesis of sterol and/or fatty acids.

Most preferably, the term relates to an EMS mutagenesis combined with a treatment with cerulenin which is an antifungal antibiotic inhibiting steroid and fatty acid biosynthesis. In a preferred embodiment, an EMS mutagenesis may be carried out by preculturing Saccharomyces cells in any suitable medium known to the skilled person, e.g. in a WMVIII medium according to Lang and Looman, 1995 (1 liter of medium comprising 50 g sucrose; 250 mg NH₄H₂PO₄; 2.8 NH₄Cl; 250 mg MgCl₂ x 6H₂O; 100 mg CaCl₂ x 2H₂O; 2 g KH₂PO₄; 550 mg MgSO₄ x 7H₂O; 75 mg meso-Inositol; 10 g Na-Glutamate; 1 ml 1000 x trace elements-solution; 4 ml 250 x vitamin-solution), in any suitable volume, e.g. in 20 ml, for any suitable period of time, preferably for 3 days, and at any suitable temperature known to the skilled person, preferably at 28°C. Subsequently, the Saccharomyces cells may be inoculated in a mainculture, e.g. in a WMVIII medium, preferably, an amount of 50 µl of the preculture may be inoculated in 50 µl of a a WMVIII medium. The mainculture solution may then be incubation by any suitable means known to the person skilled in the art, preferably for 18 h at a temperature of 28°C. Subsequently, Saccharomyces cells may be harvested, e.g. by centrifugation, preferably by centrifugation for 8 min, at a velocity of 5000 rpm. Harvested cells may then be resuspended in a suitable buffer, e.g. in phosphate buffer, preferably in a 0.1M Na₂HPO₄ buffer (pH=7). More preferably, 1x10⁸ harvested cells are resuspended in 400µl of a 0.1M Na₂HPO₄ buffer (pH=7).
Subsequently, ethyl-methane-sulfonate (EMS) is added in any suitable amount and concentration known to the person skilled in the art, preferably 10µl EMS is added. The Saccharomyces cells are then mixed with EMS, e.g. by vortexing and incubated under any suitable conditions known to the person skilled in the art, e.g. for 30 min to 12 hours, preferably for 45 min to 6 hours, more preferably for 50 min to 3 hours or 2 hours and most preferably for 1 hour. The incubation may take place at any suitable temperature known to the person skilled in the art, preferably the incubation is carried out at 24 °C.
Subsequently a compound is added which neutralizes EMS. An example of such a compound is thiosulfate, preferably sodium thiosulfate. Thus, in a preferred embodiment sodium thiosulfate solution is added, preferably a 5% sodium thiosulfate solution, more preferably, 4.6 ml of a 5% sodium thiosulfate solution is added to the incubated solution.
Subsequently, the cells may be harvested, e.g. by centrifugation, preferably by centrifugation for 8 min, at a velocity of 5000 rpm. As a further step, the cells may be resuspended in any suitable solution known to the person skilled in the art, e.g. in a sucrose-solution. Preferably, the cells may be resuspended in 8.5% sucrose-solution, more preferably, in 1ml of 8.5% sucrose-solution.

Is a mutagenesis to be combined with a treatment with a compound that inhibits the biosynthesis of sterol and/or fatty acids, e.g. with an antifungal antibiotic that inhibits the biosynthesis of sterol and/or fatty acids, like cerulenin, additional steps may be performed. Such additional steps may, in a preferred embodiment, comprise the inoculation of cells obtained from the mutagenesis step, e.g. from the EMS mutagenesis, preferably of EMS mutagenized cells resuspended in a sucrose-solution in any suitable medium known to the person skilled in the art, preferably in WMVIII liquid-medium, wherein said medium comprises a compound that inhibits the biosynthesis of sterol and/or fatty acids. Preferably, the medium comprises cerulenin, more preferably, the medium comprises 2µM cerulenin. Most preferably, to 50 ml WMVIII liquid-medium are added 2µM cerulenin and 200µl of a sucrose-solution, which contains EMS mutagenized Saccharomyces cells.
Subsequently, the solution may be incubated under any suitable conditions known to the person skilled in the art, e.g. at a temperature of 28°C and for a time of 48 to 120 hours, preferably for a time of 72 to 108 hours, most preferably for 96 hours. Cells treated in the above described manner may be used for selection procedures in order to detect any mutagenesis effect.

The term "selecting a Saccharomyces organism, which is capable of accumulating at least 15% of the cellular dry weight as lipids" means that a Saccharomyces organism which has been subjected to a mutagenesis, preferably to a mutagenesis as described herein above, is analyzed for the amount of lipids that it is capable of accumulating and that a Saccharomyces organism is selected that accumulates at least 15% of the cellular dry weight as lipids. The term "lipid" refers to any member of the group of fats and fatlike substances characterised by being water insoluble and being extractable by nonpolar solvents and containing as a major constituent aliphatic hydrocarbons. More preferably, the term relates to fatty acids, acylglycerides (such as mono-, di- and triacylglycerols), waxes, phospholipids, sphingolipids, lipopolysaccharides and isoprenoides (such as sterols and sterylesters). More preferably, the term "lipid" in the context of the selection of a Saccharomyces organism subsequently of a mutagenesis step means that no polyunsatured fatty acids (PUFAs) are comprised.
The term "selecting" refers to any suitable technique or procedure known to the person skilled in the art which allows the identification of Saccharomyces cells with an elevated lipid content as indicated above. The determination of the lipid content of the mutagenized cells may be carried out by any suitable method known to the person skilled in the art. Preferably, it is determined by GC/MS and more preferably by a method as described in the appended Examples. Thus, preferably, the lipid content is determined by the steps of harvesting mutagenized cells and separating them by gradient centrifugation, preferably density gradient centrifugation. The centrifugation is preferably carried out in an isotonic solution and it is preferably carried out in a Percoll-solution or a Ficoll-solution. Thus, the cells may be harvested, e.g. by centrifugation, preferably by centrifugation for 8 min, at a velocity of 5000 rpm. Subsequently, the cells may be resuspended in any suitable solution known to the person skilled in the art, e.g. in a Percoll-solution or in a Ficoll-solution. Preferably, 1x10⁸ harvested cells may be resuspended in a Percoll-solution or in a Ficoll-solution. After this step, the cells may be separated according to their specific weight, e.g. by gradient centrifugation, preferably by centrifugation for 1 hour, at 30000 x g. Selection of cells with an elevated lipid content may be carried out by retrieving cells, which are located in the top-layer of the centrifugation product, i.e. in the top layer of the centrifugation gradient. The term "elevated lipid content" means that the amount of lipids in the selected Saccharomyces organism is higher than in an isogenic control strain, which has not been subjected to a mutagenesis approach as defined herein above. More preferably, the term means that the amount of lipids in the selected Saccharomyces cells is at least 15%. The lipid content can be determined as described above, i.e. by GC/MS and most preferably as described in the appended Examples.

The term "at least 15%" means that the portion of lipids of the dried cells of a selected Saccharomyces organism which has been subjected to a mutagenesis is 15% or higher, preferably, said amount is between 15% and 50%, more preferably between 15% and 30%, even more preferably between 15% and 20% and most preferably between 15% and 17.5%. Preferably, the term also means that the portion of lipids of the dried cells of a selected Saccharomyces organism which has been subjected to a mutagenesis, is 15% or higher, wherein said lipids do not comprise lipids of the group of polyunsaturated fatty acids as defined herein above, preferably said lipids do not comprise any of linoleic acid, conjugated linoleic acid (CLA), gamma-linolenic acid (GLA), dihomo-gamma-linolenic acid (DGLA), arachidonic acid (ARA), alpha-linolenic acid, stearidonic acid (STA), eicosatetraenoic acid (ETA), eicosapentaenoic acid, docosapentaenoic acid (DPA) or docosahexaenoic acid (DHA). The term "cellular dry weight" means the weight of the cell mass after applying a method which reduces the amount of residual water to 5% or less, more preferred between 3% and 5%. Methods for reducing the water content of the cell mass to 5% or less are known in the art and include, e.g., vacuum drying. An example is vacuum-drying (e.g. of a cell pellet obtained by centrifugation) for 12h at 80°C in a cabinet desiccator and cooling to room temperature in a dehydrator.
The determination of the amount of lipids in relation to the dry weight of the cells may be carried out by any suitable means known to the person skilled in the art. Generally, such methods comprise extracting the lipids from cell preparations and determining the quantity (and optionally the nature) of the lipids by known and available methods. One such method is GC/MS. The skilled person can, by these methods, determine the total amount of lipids present in the sample and can put it in relation to the cellular dry weight thereby arriving at the percentage of lipids in relation to the dry weight.

The term "genetically modifying the Saccharomyces organism selected in step (a) so that it is capable of synthesizing PUFAs" means that a Saccharomyces organism, which is selected according to step (a) of the method according to the invention after a mutagenesis approach as defined herein above according to its capability to accumulate at least 15% of the cellular dry weight as lipids, is altered by any suitable genetic means and methods known to the skilled person in order to synthesize PUFAs. Preferably, the term means that such a Saccharomyces organism is provided with genetic elements which, upon their expression in the Saccharomyces organism, allow for the production of PUFAs. More preferably, the term means that such a Saccharomyces organism is transformed with genetic elements encoding one, more or all enzymes of a PUFA synthesis pathway of a heterologous organism, e.g. with genetic elements encoding fungal or plant enzymes of a PUFA synthesis pathway present in fungi or plants. Even more preferably, the genetic modification leads to the expression of one, more or all enzymes of a PUFA synthesis pathway of a heterologous organisms. Such an expression of enzymes of a PUFA synthesis pathway results in the synthesis of PUFAs as described herein above, preferably of any one or any combination of linoleic acid, conjugated linoleic acid, gamma-linolenic acid, dihomo-gamma-linolenic acid, arachidonic acid, alpha-linolenic acid, stearidonic acid, eicosatetraenoic acid, eicosapentaenoic acid, docosapentaenoic acid or docosahexaenoic acid. In a preferred alternative, the term also means that a Saccharomyces organism, which is selected in step (a) of the method according to the invention after a mutagenesis approach as defined herein above according to its capability to accumulate at least 15% of the cellular dry weight as lipids, is altered by genetic means and methods in order to synthesize PUFAs wherein said lipids do not comprise lipids of the group of polyunsaturated fatty acids as defined herein above, preferably said lipids do not comprise any of linoleic acid, conjugated linoleic acid (CLA), gamma-linolenic acid (GLA), dihomo-gamma-linolenic acid (DGLA), arachidonic acid (ARA), alpha-linolenic acid, stearidonic acid (STA), eicosatetraenoic acid (ETA), eicosapentaenoic acid, docosapentaenoic acid (DPA) or docosahexaenoic acid (DHA).
The term "genetic element" means any molecular unit which is able to transport genetic information, preferably it relates to a gene, more preferably to a native gene, a chimeric gene, a foreign gene, a transgene or a codon-optimized gene. The term "gene" refers to a nucleic acid molecule or fragment that expresses a specific protein, preferably it refers to nucleic acid molecule including regulatory sequences preceding (5' non-coding sequences) and following (3' non-coding sequences) the coding sequence. The term "native gene" refers to a gene as found in nature with its own regulatory sequences. The term "chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. According to the present invention a "foreign" gene refers to a gene not normally found in the host organism, but that is introduced into the host organism by gene transfer. Foreign genes can comprise native genes inserted into a non-native organism, or chimeric genes. The term "transgene" is a gene that has been introduced into the genome by a transformation procedure. A "codon-optimized gene" is a gene having its frequency of codon usage designed to mimic the frequency of preferred codon usage of the host cell, preferably the codon usage has been adapted to the codon usage of an organism belonging to the genus Saccharomyces, more preferably to the codon usage of Saccharomyces cerevisiae. The term "coding sequence" refers to a DNA sequence that codes for a specific amino acid sequence. The term "regulatory sequence" refer to a nucleotide sequence located upstream (5' non-coding sequences), within, or downstream (3' non-coding sequences) of a coding sequence, and which influences the transcription, RNA processing or stability, or translation of the associated coding sequence. Regulatory sequences may include promoters, enhancers, translation leader sequences, introns, polyadenylation recognition sequences, RNA processing sites, effector binding sites and stem-loop structures.
The term "promoter" refers to a DNA sequence capable of controlling the expression of a coding sequence or functional RNA. Typically, a coding sequence is located 3' to a promoter sequence. Promoters may be derived in their entirety from a native gene, or be composed of different elements derived from different promoters found in nature, or even comprise synthetic DNA segments. It is understood by a person skilled in the art that different promoters may direct the expression of a gene at different stages of development, or in response to different environmental or physiological conditions. Promoters that cause a gene to be expressed in most cell types at most times are commonly referred to as constitutive promoters. Typically, since the exact boundaries of regulatory sequences have not been completely defined, DNA fragments of different lengths may have identical promoter activity. A promoter may be operably linked with a coding sequence. In a preferred embodiment, the term "promoter" refers to DNA sequence capable of controlling the expression of a coding sequence, which is active in Saccharomyces, more preferably in Saccharomyces cerevisiae.

The term "3' non-coding sequences" refers to DNA sequences located downstream of a coding sequence. This includes polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The 3' region can influence the transcription, i.e. the presence of RNA transcripts, the RNA processing or stability, or translation of the associated coding sequence. The term "RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from post-transcriptional processing of the primary transcript and is referred to as the mature RNA. The term "mRNA" refers to messenger RNA, i.e. RNA that is without introns and that can be translated into protein by the cell.
The term "operably linked" refers to the association of nucleic acid sequences on a single nucleic acid fragment so that the function of one is affected by the other. In the context of a promoter the term means that a coding sequence is rendered capable of affecting the expression of that coding sequence, i.e., the coding sequence is under the transcriptional control of the promoter. Regulatory elements for driving expression of genes in organisms of the genus Saccharomyces are well-known to the person skilled in the art and are widely described in the literature (see, e.g., Guthrie and Fink, "Guide to yeast genetics and molecular biology", Academic Press 2002). In a preferred embodiment, an ADH1, PGK1, TDH1 or USP promoter is used.

Methods for genetically modifying organisms belonging to the genus Saccharomyces are well-known to the person skilled in the art and are widely described in the literature. They comprise the commonly used methods for introducing genetic material into Saccharomyces so as to be contained in the Saccharomyces cells integrated into the chromosome or extrachromosomally (see, e.g., Guthrie and Fink, "Guide to yeast genetics and molecular biology", Academic Press 2002).

The term "synthesis of PUFAs" or "synthesizing PUFAs" means that at least one PUFA as described herein above is produced, more preferably it means that the organism produces and accumlates at least 3%, more preferably at least 3.5%, even more preferably at least 4%, and most preferably at least 4.5% of the cellular dry weight as at least one PUFA, even more preferably as omega-3 and/or omega-6 fatty acids comprising at least 18 C atoms and most preferably as any of or any combination of linoleic acid, conjugated linoleic acid (CLA), gamma-linolenic acid (GLA), dihomo-gamma-linolenic acid (DGLA), arachidonic acid (ARA), alpha-linolenic acid, stearidonic acid (STA), eicosatetraenoic acid (ETA), eicosapentaenoic acid, docosapentaenoic acid (DPA) and docosahexaenoic acid (DHA). In a particularly preferred embodiment the Saccharomyces organism produces and accumulates at least 5%, even more preferred at least 7.5% and most preferred at least 10% of the cellular dry weight as at least one PUFA.

In another preferred embodiment the term "synthesis of PUFAs" or "synthesizing PUFAs" means that the Saccharomyces organism produces and accumulates PUFAs to a level of at least 10%, more preferably of at least 15%, even more preferably of at least 20% and most preferably of at least 30% in relation to the total lipid content of the Saccharomyces organism.
The amount of PUFAs can be determined by the skilled person by methods known in the art and as described, e.g., in the appended Examples. Thus, the amount of PUFAs can, e.g., be determined by extracting the lipids from the cells and by determining the quantity (and if desired the quality, i.e. nature) of the PUFAs, e.g. by GC/MS.
The percentage of PUFAs in relation to the total lipid content can be determined by determining the amount of PUFAs and the amount of total lipids and putting the determined values in relation to each other.

In another aspect the present invention relates to an organism belonging to the genus Saccharomyces, which is capable of accumulating at least 15% of the cellular dry weight as lipids, wherein said organism is genetically modified so that polyunsatured fatty acids (PUFAs) are synthesized. The term "capable of accumulating at least 15% of the cellular dry weight as lipids" has been defined herein above and means that a Saccharomyces organism is able to accumulate lipid (as defined hereinabove) to an extent of 15% or higher of the cellular dry weight. More preferably, it means that said amount is between 15% and 50%, between 15% and 30%, between 15% and 20% and most preferably between 15% and 17.5%. In a preferred alternative said term also means that the portion of lipids of the cellular dry weight of a Saccharomyces organism is 15% or higher, wherein said lipids do not comprise lipids of the group of polyunsaturated fatty acids as defined herein above, preferably said lipids do not comprise any of linoleic acid, conjugated linoleic acid (CLA), gamma-linolenic acid (GLA), dihomo-gamma-linolenic acid (DGLA), arachidonic acid (ARA), alpha-linolenic acid, stearidonic acid (STA), eicosatetraenoic acid (ETA), eicosapentaenoic acid, docosapentaenoic acid (DPA) or docosahexaenoic acid (DHA). In a preferred embodiment, such an organism can be obtained by performing a mutagenesis and selection approach as defined herein above, or as described in the examples.
The term "genetically modified so that polyunsatured fatty acids (PUFAs) are synthesized" has also been defined herein above and means in the context of such an organism that a Saccharomyces organism is altered by any suitable genetic means and methods known to the skilled person in order to synthesize PUFAs, i.e. it is provided with genetic elements as described herein above, which allow the production of PUFAs. More preferably, the term means that such a Saccharomyces organism is transformed with genetic elements encoding one, more or all enzymes of a PUFA synthesis pathway of a heterologous organism, e.g. with genetic elements encoding fungal or plant enzymes of a PUFA synthesis pathway present in fungi or plants. Even more preferably, the genetic modification leads to the expression of one, more or all enzymes of a PUFA synthesis pathway of a heterologous organisms. Such an expression of enzymes of a PUFA synthesis pathway results in the synthesis of PUFAs as described herein above, preferably of any one or any combination of linoleic acid, conjugated linoleic acid, gamma-linolenic acid, dihomo-gamma-linolenic acid, arachidonic acid, alpha-linolenic acid, stearidonic acid, eicosatetraenoic acid, eicosapentaenoic acid, docosapentaenoic acid or docosahexaenoic acid.

In a preferred embodiment of the present invention, the genetic modification of step (a) of the method according to the invention or of an organism as defined herein above results in the expression of enzymes of the omega-3/omega-6 fatty acid biosynthetic pathway. The term "omega-3/omega-6 fatty acid biosynthetic pathway" refers to a set of genes which encode enzymes that catalyze the production of either or both omega-3 and omega-6 polyunsaturated fatty acids. Preferably, the genes involved in the omega-3/omega-6 fatty acid biosynthetic pathway encode some or all of the following enzymes: Δ12 desaturase, isomerase, Δ6 desaturase, C_{18/20} elongase, C_{20/22} elongase, Δ5 desaturase, Δ17 desaturase, Δ15 desaturase, Δ9 desaturase and Δ4 desaturase. A representative pathway is illustrated in Fig. 1, which demonstrates how both omega-3 and omega-6 fatty acids may be produced from a common source. It should be understood that "omega-3/omega-6 fatty acid biosynthetic pathway" does not imply that all the genes listed in this paragraph are required as a number of fatty acid products will only require the expression of a subset of the genes of this pathway, as the person skilled in the art would know and as can be derived from Fig. 1.
The term "expression", as used herein, refers to the transcription and accumulation of sense (mRNA) derived from nucleic acid molecules or genes involved in the omega-3/omega-6 fatty acid biosynthetic pathway. More preferably, the term also refers to the translation of mRNA into a polypeptide or proteins and the corresponding provision of such polypeptides or proteins within the cell.

In another preferred embodiment of the present invention, the genetic modification of step (a) of the method according to the invention or of an organism as defined herein above, which results in the expression of enzymes of the omega-3/omega-6 fatty acid biosynthetic pathway, is a transformation of said organism with genes encoding enzymes of the omega-3/omega-6 fatty acid biosynthetic pathway.
The term "transformation" refers to the transfer of a genetic element, typically of a nucleic acid molecule into a host organism, i.e. into an organism of the genus Saccharomyces as defined herein above, wherein said transfer results in a genetically stable inheritance. Conditions for a transformation of Saccharomyces cells and corresponding techniques are known to the person skilled in the art. These techniques include chemical transformation, preferably a lithium acetate transformation, as, e.g., derivable from Methods in Enzymology, 194:186-187 (1991), protoplast fusion, bolistic impact transformation, electroporation, microinjection, or any other method that introduces the gene or nucleic acid molecule of interest into the host cell.
A transformed cell may have at least one copy of the introduced genetic element and may have two or more, depending upon whether the genetic element is integrated into the genome, amplified or is present on an extrachromosomal element having multiple copy numbers. Preferably, the transformed cell can be identified by selection for a marker contained on the introduced genetic element. Alternatively, a separate marker construct may be co-transformed with the desired genetic element, as many transformation techniques introduce many DNA molecules into host cells. Typically, transformed cells are selected for their ability to grow on selective media. Selective media may incorporate an antibiotic or lack a factor necessary for growth of the untransformed cell, such as a nutrient or growth factor. An introduced marker gene may confer antibiotic resistance, or encode an essential growth factor or enzyme, thereby permitting growth on selective media when expressed in the transformed host. Selection of a transformed cell can also occur when the expressed marker protein can be detected, either directly or indirectly. The marker protein may be expressed alone or as a fusion to another protein. The marker protein may be detected, for example, by its enzymatic activity. Alternatively, antibodies may be used to detect the marker protein or a molecular tag on, for example, a protein of interest. Cells expressing the marker protein or tag can be selected, for example, visually, or by techniques such as FACS or panning using antibodies. Preferably, any suitable marker that functions in cells of the genus Saccharomyces, as known to the person skilled in the art, may be used. More preferably markers which provide resistance to kanamycin, hygromycin or the amino glycoside G418, as well as the ability to grow on media lacking uracil, leucine, histidine, methionine, lysine or tryptophane may be employed.

In a preferred embodiment of the present invention, the polyunsaturated fatty acid synthesized by the organism according to the present invention or provided in a method according to the present invention is any one of, or any combination of: linoleic acid, conjugated linoleic acid, gamma-linolenic acid, dihomo-gamma-linolenic acid, arachidonic acid, alpha-linolenic acid, stearidonic acid, eicosatetraenoic acid, eicosapentaenoic acid, docosapentaenoic acid or docosahexaenoic acid. The term "any combination of" relates preferably to a combination of linoleic acid and conjugated linoleic acid, conjugated linoleic acid and gamma-linolenic acid, gamma-linolenic acid and dihomo-gamma-linolenic acid, dihomo-gamma-linolenic acid and arachidonic acid, arachidonic acid and alpha-linolenic acid, alpha-linolenic acid and stearidonic acid, stearidonic acid and eicosatetraenoic acid, eicosatetraenoic acid and eicosapentaenoic acid, eicosapentaenoic acid and docosapentaenoic acid, docosapentaenoic acid and docosahexaenoic acid, linoleic acid and gamma-linolenic acid, linoleic acid and dihomo-gamma-linolenic acid, linoleic acid and arachidonic acid, linoleic acid and alpha-linolenic acid, linoleic acid and stearidonic acid, linoleic acid and eicosatetraenoic acid, linoleic acid and eicosapentaenoic acid, linoleic acid and docosapentaenoic acid, linoleic acid and docosapentaenoic acid, linoleic acid and docosahexaenoic acid, conjugated linoleic acid and dihomo-gamma-linolenic acid, conjugated linoleic acid and arachidonic acid, conjugated linoleic acid and alpha-linolenic acid, conjugated linoleic acid and stearidonic acid, conjugated linoleic acid and eicosatetraenoic acid, conjugated linoleic acid and eicosapentaenoic acid, conjugated linoleic acid and docosapentaenoic acid, conjugated linoleic acid and docosapentaenoic acid, conjugated linoleic acid and docosahexaenoic acid, gamma-linolenic acid and arachidonic acid, gamma-linolenic acid and alpha-linolenic acid, gamma-linolenic acid and stearidonic acid, gamma-linolenic acid and eicosatetraenoic acid, gamma-linolenic acid and eicosapentaenoic acid, gamma-linolenic acid and docosapentaenoic acid, gamma-linolenic acid and docosapentaenoic acid, dihomo-gamma-linolenic acid and alpha-linolenic acid, dihomo-gamma-linolenic acid and stearidonic acid, dihomo-gamma-linolenic acid and eicosatetraenoic acid, dihomo-gamma-linolenic acid and eicosapentaenoic acid, dihomo-gamma-linolenic acid and docosapentaenoic acid, dihomo-gamma-linolenic acid and docosapentaenoic acid, arachidonic acid and stearidonic acid, arachidonic acid and eicosatetraenoic acid, arachidonic acid and eicosapentaenoic acid, arachidonic acid and docosapentaenoic acid, arachidonic acid and docosapentaenoic acid, alpha-linolenic acid and eicosatetraenoic acid, alpha-linolenic acid and eicosapentaenoic acid, alpha-linolenic acid and docosapentaenoic acid, alpha-linolenic acid and docosapentaenoic acid, stearidonic acid and eicosapentaenoic acid, stearidonic acid and docosapentaenoic acid, stearidonic acid and docosapentaenoic acid, eicosatetraenoic acid and docosapentaenoic acid, eicosatetraenoic acid and docosapentaenoic acid, as well as eicosapentaenoic acid and docosahexaenoic acid.
When more than one PUFA is synthesized at the same time, e.g. when a combination of different PUFAs is synthesized, the produced PUFAs may be present in any possible ratio, ranging from 1 to 99 for PUFA A to PUFA B, up to 99 to1 for PUFA A to PUFA B. The terms "A" and "B" refer to and represent any of the PUFAs mentioned herein above in the context of the present invention, preferably they refer to and represent linoleic acid, conjugated linoleic acid, gamma-linolenic acid, dihomo-gamma-linolenic acid, arachidonic acid, alpha-linolenic acid, stearidonic acid, eicosatetraenoic acid, eicosapentaenoic acid, docosapentaenoic acid or docosahexaenoic acid. More preferably, the ratio between PUFA A and PUFA B is 25 to 75, 35 to 65, 40 to 60, 45 to 55 and most preferably 50 to 50. The term "ratio" refers to a ratio of amounts of PUFAs, preferably to a ratio of weight of PUFAs in cells in a dry state, more preferably it relates to a ratio of percentage of PUFAs of the cellular dry weight.
The PUFAs linoleic acid, conjugated linoleic acid, gamma-linolenic acid, dihomo-gamma-linolenic acid, arachidonic acid, alpha-linolenic acid, stearidonic acid, eicosatetraenoic acid, eicosapentaenoic acid, docosapentaenoic acid and docosahexaenoic acid may be synthesized by the expression of at least one or a combination of the enzymes desaturase, elongase and isomerase. The term "desaturase" refers to a polypeptide component of a multi-enzyme complex that can desaturate one or more fatty acids to produce a mono- or polyunsaturated fatty acid or precursor of interest. The term "elongase" refers to a polypeptide component of a multi-enzyme complex that can elongate a fatty acid carbon chain to produce a mono- or polyunsaturated fatty acid that is 2 carbons longer than the fatty acid substrate that the elongase acts upon. This process of elongation occurs in a multi-step mechanism in association with fatty acid synthase, whereby CoA is the acyl carrier (Lassner et al., The Plant Cell 8:281-292 (1996)). Briefly, malonyl-CoA is condensed with a long-chain acyl-CoA to yield CO2 and a β-ketoacyl-CoA (where the acyl moiety has been elongated by two carbon atoms). Subsequent reactions include reduction to β - hydroxyacyl-CoA, dehydration to an enoyl-CoA and a second reduction to yield the elongated acyl-CoA. Examples of reactions catalyzed by elongases are the conversion of GLA to DGLA, STA to ETA and EPA to DPA. Accordingly, elongases may have different specificities (e.g., a C16/18 elongase will prefer a C16 substrate, a C18/20 elongase will prefer a C18 substrate and a C20/22 elongase will prefer a C20 substrate).
More preferably the PUFAs according to the present invention may be synthesized by the expression of at least one or a combination of the enzymes Δ12 desaturase, Δ 6 desaturase, Δ5 desaturase, Δ4 desaturase, C_{18/20} elongase, C_{20/22} elongase, Δ15 desaturase, Δ17 desaturase and isomerase. Preferably, nucleic acid molecules encoding said enzymes are expressed, as defined herein above. The term "Δ12 desaturase" refers to any known desaturase, which desaturates a fatty acid at position 12, preferably it refers to a Δ12 desaturase derived from Pichia, more preferably it refers to a Δ12 desaturase derived from Pichia pastoris. In the most preferred embodiment, the term refers to a protein encoded by the sequence of SEQ ID NO: 1 or 2. The term "Δ6 desaturase" refers to any known desaturase, which desaturates a fatty acid at position 6, preferably it refers to a Δ6 desaturase derived from Ostreococcus, more preferably it refers to a Δ6 desaturase derived from Ostreococcus tauri. In the most preferred embodiment, the term refers to a protein encoded by the sequence of SEQ ID NO: 3 or 4. The term "Δ5 desaturase" refers to any known desaturase, which desaturates a fatty acid at position 5, preferably it refers to a Δ5 desaturase derived from Mortierella, more preferably it refers to a Δ5 desaturase derived from Mortierella alpina. In the most preferred embodiment, the term refers to a protein encoded by the sequence of SEQ ID NO: 11 or 12. The term "Δ4 desaturase" refers to any known desaturase, which desaturates a fatty acid at position 4, preferably it refers to a Δ4 desaturase derived from Thraustochytrium sp. In the most preferred embodiment, the term refers to a protein encoded by the sequence of SEQ ID NO: 13 or 14.
The term "Δ15 desaturase" refers to any known desaturase, which desaturates a fatty acid at position 15, preferably it refers to a Δ15 desaturase derived from Pichia, more preferably it refers to a Δ15 desaturase derived from Pichia pastoris In the most preferred embodiment, the term refers to a protein encoded by the sequence of SEQ ID NO: 7 or 8.

The term "Δ17 desaturase" refers to any known desaturase, which desaturates a fatty acid at position 17, preferably it refers to a Δ17 desaturase, derived from Mortierella, more preferably it refers to a Δ17 desaturase derived from Mortierella alpina. In the most preferred embodiment, the term refers to a protein encoded by the sequence of SEQ ID NO: 15 or 16.

The term "C_{18/20} elongase" refers to any known elongase, which elongates a fatty acid comprising 18 C atoms to a fatty acid comprising 20 C atoms, preferably it refers to a C_{18/20} elongase derived from Caenorhabditis, more preferably it refers to a C_{18/20} elongase derived from Caenorhabditis elegans. In the most preferred embodiment, the term refers to a protein encoded by the sequence of SEQ ID NO: 5 or 6. The term "C_{20/22} elongase" refers to any known elongase, which elongates a fatty acid comprising 20 C atoms to a fatty acid comprising 22 C atoms, preferably it refers to a C_{20/22} elongase derived from Scophthalmus, more preferably it refers to a C_{18/20} elongase derived from Scophthalmus maximus. In the most preferred embodiment, the term refers to a protein encoded by the sequence of SEQ ID NO: 9 or 10.
The term "isomerase" refers to any known isomerase which is capable of converting linoleic acid into conjugated linoleic acid, in particular into conjugated linoleic acid (t10, c12 18:2). An example for such an isomerase of Propionibacterium acnes is described in Liavonchanka and Hornung (Proc. Natl. Acad. Sci. USA 103 (2005), 2576-2581).
In a preferred embodiment the term "nucleic acid" as used in the context of the expression of the enzymes of a PUFA biosynthesis pathway, in particular of Δ12 desaturase, Δ 6 desaturase, Δ5 desaturase, Δ4 desaturase, C_{18/20} elongase, C_{20/22} elongase, Δ15 desaturase, Δ17 desaturase and isomerase, refers to any type of nucleic acid molecule which results, upon transcription and translation, in the mentioned polypeptides, including nucleic acids comprising degenerated codons. The term "degenerated codons" refers to the nature in the genetic code permitting variation of the nucleotide sequence without affecting the amino acid sequence of an encoded polypeptide.
As the person skilled in the art would know, there is a codon-bias exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.
Thus, in a preferred embodiment the present invention also contemplates the use of nucleic acid sequences encoding enzymes of the PUFA biosynthesis pathway, in particular Δ12 desaturase, Δ 6 desaturase, Δ5 desaturase, Δ4 desaturase, C_{18/20} elongase, C_{20/22} elongase, Δ15 desaturase, Δ17 desaturase and isomerase, which have been modified in a portion or all of the cordons such that the modified polypeptide uses codons that are preferred by the host cell, i.e. that are preferred by Saccharomyces, preferably by Saccharomyces cerevisiae. Typically, host-preferred codons may be determined within a particular host species of interest by examining codon usage in proteins (preferably those expressed in the largest amount) and determining which codons are used with highest frequency. Then, the coding sequence for a polypeptide of interest having desaturase or elongase activity may be synthesized in whole or in part using the codons preferred in the host species. All (or portions) of the DNA also can be synthesized to remove any destabilizing sequences or regions of secondary structure that would be present in the transcribed mRNA. All (or portions) of the DNA also can be synthesized to alter the base composition to one more preferable in the desired host cell, preferably to those preferred in Saccharomyces, more preferably in Saccharomyces cerevisiae. More preferably codon-optimized sequences of Δ12 desaturase, Δ 6 desaturase, Δ5 desaturase, Δ4 desaturase, C_{18/20} elongase, C_{20/22} elongase, Δ15 desaturase, Δ17 desaturase are used, most preferably the codon-optimized sequences of SEQ ID NOs: 2, 4, 6, 8, 10, 12, 14 and 16 encoding Δ12 desaturase, Δ6 desaturase, C_{18/20} elongase, Δ15 desaturase, C_{20/22} elongase, Δ5 desaturase, Δ4 desaturase and Δ17 desaturase, respectively, may be used for the synthesis of PUFAs as defined herein above.

In a further preferred embodiment of the present invention, conjugated linoleic acid is synthesized by the expression of the enzymes Δ12 desaturase and isomerase in a Saccharomyces organism according to the present invention as defined herein above. More preferably conjugated linoleic acid may be synthesized by the expression of a Δ12 desaturase derived from Pichia, even more preferably a Δ12 desaturase derived from Pichia pastoris and most preferably by the expression of a protein encoded by the sequence of SEQ ID NO: 1 or 2 and by the expression of an isomerase, in particular an isomerase which is capable of converting linoleic acid into conjugated linoleic acid preferably into conjugated linoleic acid (t10, c12 18:2).

In further preferred embodiment of the present invention, arachidonic acid is synthesized by the expression of the enzymes Δ12 desaturase, Δ6 desaturase, Δ5 desaturase, and C_{18/20} elongase in a Saccharomyces organism according to the present invention as defined herein above. More preferably arachidonic acid may be synthesized by the expression of a Δ12 desaturase derived from Pichia, even more preferably a Δ12 desaturase derived from Pichia pastoris and most preferably by the expression of a protein encoded by the sequence of SEQ ID NO: 1 or 2;
and by the expression of Δ6 desaturase derived from Ostreococcus, even more preferably derived from Ostreococcus tauri and most preferably by the expression of a protein encoded by the sequence of SEQ ID NO: 3 or 4;
and by the expression of Δ5 desaturase derived from Mortierella, even more preferably derived from Mortierella alpina and most preferably by the expression of a protein encoded by the sequence of SEQ ID NO: 11 or 12;
and by the expression of C_{18/20} elongase derived from Caenorhabditis, even more preferably derived from Caenorhabditis elegans and most preferably by the expression of a protein encoded by the sequence of SEQ ID NO: 5 or 6.

In a further preferred embodiment of the present invention, eicosapentaenoic acid is synthesized by the expression of the enzymes Δ12 desaturase, Δ6 desaturase, Δ5 desaturase, C_{18/20} elongase and further Δ15 desaturase and/or Δ17 desaturase in a Saccharomyces organism according to the present invention as defined herein above. More preferably eicosapentaenoic acid may be synthesized by the expression of a Δ12 desaturase derived from Pichia, even more preferably a Δ12 desaturase derived from Pichia pastoris and most preferably by the expression of a protein encoded by the sequence of SEQ ID NO: 1 or 2;
and by the expression of Δ6 desaturase derived from Ostreococcus, even more preferably derived from Ostreococcus tauri and most preferably by the expression of a protein encoded by the sequence of SEQ ID NO: 3 or 4;
and by the expression of Δ5 desaturase derived from Mortierella, even more preferably derived from Mortierella alpina and most preferably by the expression of a protein encoded by the sequence of SEQ ID NO: 11 or 12;
and by the expression of C_{18/20} elongase derived from Caenorhabditis, even more preferably derived from Caenorhabditis elegans and most preferably by the expression of a protein encoded by the sequence of SEQ ID NO: 5 or 6;
and by the expression of Δ15 desaturase derived from Pichia, even more preferably derived from Pichia pastoris and most preferably by the expression of a protein encoded by the sequence of SEQ ID NO: 7 or 8;
and/or by the expression of Δ17 desaturase derived from Mortierella, even more preferably derived from Mortierella alpina, and most preferably by the expression of a protein encoded by the sequence of SEQ ID NO: 15 or 16. The term "and/or" means that either a Δ15 desaturase or Δ17 desaturase or both activities may be used in order to synthesize eicosapentaenoic acid. In a preferred embodiment both activities are used in an activity ratio of 50 to 50. The term "activity ratio" means that Δ15 desaturase and Δ17 desaturase are both equally active and equally contribute to the synthesis of eicosapentaenoic acid.

In further preferred embodiment of the present invention, docosapentaenoic acid is synthesized by the expression of the enzymes Δ12 desaturase, Δ6 desaturase, Δ5 desaturase, C_{18/20} elongase and C_{20/22} elongase and further Δ15 desaturase and/or Δ17 desaturase in a Saccharomyces organism according to the present invention as defined herein above. More preferably docosapentaenoic acid may be synthesized by the expression of a Δ12 desaturase derived from Pichia, even more preferably a Δ12 desaturase derived from Pichia pastoris and most preferably by the expression of a protein encoded by the sequence of SEQ ID NO: 1 or 2;
and by the expression of Δ6 desaturase derived from Ostreococcus, even more preferably derived from Ostreococcus tauri and most preferably by the expression of a protein encoded by the sequence of SEQ ID NO: 3 or 4;
and by the expression of Δ5 desaturase derived from Mortierella, even more preferably derived from Mortierella alpina and most preferably by the expression of a protein encoded by the sequence of SEQ ID NO: 11 or 12;
and by the expression of C_{18/20} elongase derived from Caenorhabditis, even more preferably derived from Caenorhabditis elegans and most preferably by the expression of a protein encoded by the sequence of SEQ ID NO: 5 or 6;
and by the expression of C_{20/22} elongase derived from Scophthalmus, even more preferably derived from Scophthalmus maximus and most preferably by the expression of a protein encoded by the sequence of SEQ ID NO: 9 or 10;
and by the expression of Δ15 desaturase derived from Pichia, even more preferably derived from Pichia pastoris and most preferably by the expression of a protein encoded by the sequence of SEQ ID NO: 7 or 8;
and/or by the expression of Δ17 desaturase derived from Mortierella, even more preferably derived from Mortierella alpina, and most preferably by the expression of a protein encoded by the sequence of SEQ ID NO: 15 or 16. The term "and/or" means that either a Δ15 desaturase or Δ17 desaturase or both activities may be used in order to synthesize eicosapentaenoic acid. In a preferred embodiment both activities are used in an activity ratio of 50 to 50. The term "activity ratio" means that Δ15 desaturase and Δ17 desaturase are both equally active and equally contribute to the synthesis of eicosapentaenoic acid.

In further preferred embodiment of the present invention, docosahexaenoic acid is synthesized by the expression of the enzymes Δ12 desaturase, Δ6 desaturase, Δ5 desaturase, Δ4 desaturase, C_{18/20} elongase and C_{20/22} elongase and further Δ15 desaturase and/or Δ17 desaturase in a Saccharomyces organism according to the present invention as defined herein above. More preferably docosahexaenoic acid may be synthesized by the expression of a Δ12 desaturase derived from Pichia, even more preferably a Δ12 desaturase derived from Pichia pastoris and most preferably by the expression of a protein encoded by the sequence of SEQ ID NO: 1 or 2;
and by the expression of Δ6 desaturase derived from Ostreococcus, even more preferably derived from Ostreococcus tauri and most preferably by the expression of a protein encoded by the sequence of SEQ ID NO: 3 or 4;
and by the expression of Δ5 desaturase derived from Mortierella, even more preferably derived from Mortierella alpina and most preferably by the expression of a protein encoded by the sequence of SEQ ID NO: 11 or 12;
and by the expression of Δ4 desaturase derived from Thraustochytrium sp., most preferably by the expression of a protein encoded by the sequence of SEQ ID NO: 13 or 14;
and by the expression of C_{18/20} elongase derived from Caenorhabditis, even more preferably derived from Caenorhabditis elegans and most preferably by the expression of a protein encoded by the sequence of SEQ ID NO: 5 or 6;
and by the expression of C_{20/22} elongase derived from Scophthalmus, even more preferably derived from Scophthalmus maximus and most preferably by the expression of a protein encoded by the sequence of SEQ ID NO: 9 or 10;
and by the expression of Δ15 desaturase derived from Pichia, even more preferably derived from Pichia pastoris and most preferably by the expression of a protein encoded by the sequence of SEQ ID NO: 7 or 8;
and/or by the expression of Δ17 desaturase derived from Mortierella, even more preferably derived from Mortierella alpina, and most preferably by the expression of a protein encoded by the sequence of SEQ ID NO: 15 or 16. The term "and/or" means that either a Δ15 desaturase or Δ17 desaturase or both activities may be used in order to synthesize eicosapentaenoic acid. In a preferred embodiment both activities are used in an activity ratio of 50 to 50. The term "activity ratio" means that Δ15 desaturase and Δ17 desaturase are both equally active and equally contribute to the synthesis of eicosapentaenoic acid.

In further preferred embodiment of the present invention, docosahexaenoic acid and eicosapentaenoic acid are synthesized by the expression of the enzymes Δ12 desaturase, Δ6 desaturase, Δ5 desaturase, Δ4 desaturase, C_{18/20} elongase and C_{20/22} elongase and further Δ15 desaturase and/or Δ17 desaturase in a Saccharomyces organism according to the present invention as defined herein above. More preferably docosahexaenoic acid and eicosapentaenoic acid may be synthesized by the expression of a Δ12 desaturase derived from Pichia, even more preferably a Δ12 desaturase derived from Pichia pastoris and most preferably by the expression of a protein encoded by the sequence of SEQ ID NO: 1 or 2;
and by the expression of Δ6 desaturase derived from Ostreococcus, even more preferably derived from Ostreococcus tauri and most preferably by the expression of a protein encoded by the sequence of SEQ ID NO: 3 or 4;
and by the expression of Δ5 desaturase derived from Mortierella, even more preferably derived from Mortierella alpina and most preferably by the expression of a protein encoded by the sequence of SEQ ID NO: 11 or 12;
and by the expression of Δ4 desaturase derived from Thraustochytrium sp., most preferably by the expression of a protein encoded by the sequence of SEQ ID NO: 13 or 14;
and by the expression of C_{18/20} elongase derived from Caenorhabditis, even more preferably derived from Caenorhabditis elegans and most preferably by the expression of a protein encoded by the sequence of SEQ ID NO: 5 or 6;
and by the expression of C_{20/22} elongase derived from Scophthalmus, even more preferably derived from Scophthalmus maximus and most preferably by the expression of a protein encoded by the sequence of SEQ ID NO: 9 or 10;
and by the expression of Δ15 desaturase derived from Pichia, even more preferably derived from Pichia pastoris and most preferably by the expression of a protein encoded by the sequence of SEQ ID NO: 7 or 8;
and/or by the expression of Δ17 desaturase derived from Mortierella, even more preferably derived from Mortierella alpina, and most preferably by the expression of a protein encoded by the sequence of SEQ ID NO: 15 or 16. The term "and/or" means that either a Δ15 desaturase or Δ17 desaturase or both activities may be used in order to synthesize eicosapentaenoic acid. In a preferred embodiment both activities are used in an activity ratio of 50 to 50. The term "activity ratio" means that Δ15 desaturase and Δ17 desaturase are both equally active and equally contribute to the synthesis of eicosapentaenoic acid. The term "docosahexaenoic acid and eicosapentaenoic acid" means that both compounds are synthesized at the same time.

In further preferred embodiment of the present invention, eicosapentaenoic acid and docosahexaenoic acid are synthesized in a ratio of approximately 40 to 60. The term "ratio" refers to a ratio of amounts of docosahexaenoic acid and eicosapentaenoic acid, preferably to a ratio of weight of docosahexaenoic acid and eicosapentaenoic acid in cells in a dry state, more preferably it relates to a ratio of percentage of docosahexaenoic acid and eicosapentaenoic acid of the cellular dry weight. The term "approximately" means that the exact value can differ by any integer of 1, 2, 3, 4, 5, 6, 7, 8, 9 or 10 from 40 or 60, respectively. More preferably the term relates to ratios between eicosapentaenoic acid and docosahexaenoic acid ranging from 30 : 70 to 50 : 50 with any integer in between these values.

In further preferred embodiment of the present invention, the expression of enzymes of the omega-3/omega-6 fatty acid biosynthetic pathway in a Saccharomyces organism in accordance with the present invention and as described herein above is based on genetic elements integrated in the genome of said organism or present on plasmids. The term "genetic elements integrated in the genome of said organism or present on plasmids" means that nucleic acid molecules, preferably DNA encoding enzymes of the omega-3/omega-6 fatty acid biosynthetic pathway, more preferably enzymes encoding Δ12 desaturase, Δ 6 desaturase, Δ5 desaturase, Δ4 desaturase, C_{18/20} elongase, C_{20/22} elongase, Δ15 desaturase, Δ17 desaturase or isomerase, as defined herein above, are either stabely integrated into the chromosome of a Saccharomyces cell as defined herein above, or placed on a plasmid or vector, which is preferably capable of autonomous replication in a Saccharomyces cell. The terms "plasmid" and "vector" refer to an extra chromosomal element often carrying genes that are not part of the central metabolism of the cell, and usually in the form of circular double-stranded DNA fragments. More preferably, the term plasmid refers to any plasmid suitable for transformation of Saccharomyces known to the person skilled in the art and in particular to any plasmid suitable for expression of proteins in Saccharomyces. Such plasmids are extensively described in the art (see, e.g., Guthrie and Fink, "Guide to yeast genetics and molecular biology", Academic Press 2002). Even more preferably, the term relates to plasmids of the type pFlat (Veen, M. (2002), "Identifizierung von Regulationspunkten des Ergosterolbiosyntheseweges und Etablierung des 7-Dehydrocholesterolbiosyntheseweges in der Hefe Saccharomyces cerevisiae", PhD thesis, Technische Universität Berlin, Germany). Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell. The term also refers to transformation cassettes and expression cassettes. In accordance with the present invention the term "transformation cassette" refers to a specific vector containing a foreign gene and having elements in addition to the foreign gene that facilitates transformation of a particular host cell, in particular a cell of the genus Saccharomyces, more preferably a Saccharomyces cerevisiae cell. The term "expression cassette" refers to a specific vector containing a foreign gene and having elements in addition to the foreign gene that allow for enhanced expression of that gene in a foreign host, in particular in a cell of the genus Saccharomyces, more preferably in Saccharomyces cerevisiae.
Integration of genetic elements, e.g. of expression cassettes can occur randomly within the host genome or can be targeted through the use of constructs containing regions of homology with the host genome sufficient to target recombination within the host locus. Where constructs are targeted to an endogenous locus, all or some of the transcriptional and translational regulatory regions can be provided by the endogenous locus. Where two or more genes are expressed from separate replicating vectors, it is desirable that each vector has a different means of selection and should lack homology to the other constructs to maintain stable expression and prevent reassortment of elements among constructs. Judicious choice of regulatory regions, selection means and method of propagation of the introduced construct can be experimentally determined so that all introduced genes are expressed at the necessary levels to provide for synthesis of the desired products.

In a preferred embodiment the genetic elements comprise microbial expression systems. Such expression systems and expression vectors containing regulatory sequences that direct high level expression of foreign proteins are well known to those skilled in the art. Any of these could be used to construct chimeric genes for production of any of the gene products of the desaturase, elongase and/or isomerase sequences mentioned herein above. These chimeric genes could be introduced into Saccharomyces via transformation, as described herein above, to provide high-level expression of the encoded enzymes.
Accordingly, it is expected that introduction of chimeric genes encoding a PUFA biosynthetic pathway under the control of an appropriate promoter will result in (increased) production of omega-3 and/or omega-6 fatty acids. Preferably, various combinations of these PUFA desaturase and elongase genes may be expressed together in a host microorganism from specific expression cassette. Preferably, genes encoding Δ12 desaturase and Δ6 desaturase may be present in one expression cassette, genes encoding C_{20/22} elongase and Δ15 desaturase may be present in one expression cassette, genes encoding C_{18/20} elongase and Δ5 desaturase may be present in one expression cassette; and genes encoding Δ4 desaturase and Δ17 desaturase may be present in one expression cassette. Such expression cassettes may be used independently of each other or in combination. The use will depend on the PUFA to be synthesized, as illustrated by the PUFA biosynthesis pathway illustrated in Fig. 1. The combined expression of Δ12 desaturase and Δ6 desaturase will result in the synthesis of γ-linolenic acid (GLA). The combined expression of Δ12 desaturase, Δ6 desaturase, C_{18/20} elongase and Δ5 desaturase will result in the synthesis of arachidonic acid (ARA). The combined expression of Δ12 desaturase, Δ6 desaturase, C_{18/20} elongase, Δ5 desaturase, Δ4 desaturase and Δ17 desaturase results in the synthesis of eicosapentaenoic acid (EPA). The combined expression of Δ12 desaturase, Δ6 desaturase, C_{20/22} elongase, Δ15 desaturase, C_{18/20} elongase and Δ5 desaturase leads to the synthesis of docosapentaenoic acid (DPA). The combined expression of Δ12 desaturase, Δ6 desaturase, C_{20/22} elongase, Δ15 desaturase, C_{18/20} elongase, Δ5 desaturase, Δ4 desaturase and Δ17 desaturase leads to the synthesis of docosahexanoic acid (DHA). They may either be present on plasmids as described herein above or be integrated in the genome of a Saccharomyces cell. In a preferred embodiment expression cassettes comprising genes encoding Δ12 desaturase and Δ6 desaturase, C_{20/22} elongase and Δ15 desaturase, C_{18/20} elongase and Δ5 desaturase as well as encoding Δ4 desaturase and Δ17 desaturase may be present on a plasmid, preferably on a pFlat plasmid. More preferably the sequences of any of SEQ ID NOs: 1 to 16 may be present in an expression cassette. In a further preferred embodiment, a plasmid comprising an expression cassette in accordance with the present invention comprises the sequence of any one of SEQ ID NOs: 17 to 20, i.e. sequences encoding Δ12 desaturase and Δ6 desaturase, C_{20/22} elongase and Δ15 desaturase, C_{18/20} elongase and Δ5 desaturase as well as Δ4 desaturase and Δ17 desaturase, respectively, on a pFlat plasmid. Should more than one expression cassette or plasmid be used at a time, different markers for each expression cassette or plasmid have to be used. Preferably, the markers URA3, KanMX, LEU2 and HIS3 may be used.

In a further preferred embodiment of the invention, the expression of enzymes of the omega-3/omega-6 fatty acid biosynthetic pathway in a Saccharomyces organism in accordance with the present invention and as described herein above can be regulated. The term "can be regulated" means that the transcription and/or translation of genes or gene products can be controlled by any suitable means known to the person skilled in the art. Preferably, the term relates to the presence of genetic elements, e.g. DNA sequences, and more preferably a promoter, which is capable of controlling the expression of a coding sequence. Most preferably such a promoter is regulatable in Saccharomyces, particularly in Saccharomyces cerevisiae. Preferably, the term "regulatable" means that activity of the promoter can be regulated by an exogenous factor, e.g. that the promoter can be switched on or off by the use of a stimulus or of a compound, preferably of an exogenous compound which is provided, for example, in the medium. An example of a regulatable promoter is the Tetracyline-promoter, which is regulated by the presence or absence of Tetracycline.
Transcriptional initiation regulatory regions may be obtained, for example, from genes in the glycolytic pathway, such as alcohol dehydrogenase, glyceraldehyde-3-phosphate-dehydrogenase, phosphoglycerate mutase, fructose-bisphosphate aldolase, phosphoglucose-isomerase or phosphoglycerate kinase, or from regulatable genes such as acid phosphatase, lactase, metallothionein, glucoamylase, translation elongation factor EF1-α (TEF) protein or ribosomal protein S7 Any one of a number of regulatory sequences can be used, depending upon whether constitutive or induced transcription is desired, the efficiency of the promoter in expressing the ORF of interest, the ease of construction and the like.

Nucleotide sequences surrounding the translational initiation codon 'ATG' have been found to affect expression in Saccharomyces. Thus, in a further embodiment, If the desired polypeptide is poorly expressed in Saccharomyces, the nucleotide sequences of exogenous genes may be modified to include an efficient yeast translation initiation sequence to obtain optimal gene expression. For expression in yeast, this can be done by site-directed mutagenesis of an inefficiently expressed gene by fusing it in-frame to an endogenous Saccharomyces gene, preferably a highly expressed gene. Alternatively one can determine the consensus translation initiation sequence in the host and engineer this sequence into heterologous genes for their optimal expression in the host of interest, as has already been described herein above in the context of codon-optimization.

A further control elements, which can contribute to the regulation of expression, is a termination region, which may be derived, for example, from the 3' region of the gene from which the initiation region was obtained or from a different gene. A large number of termination regions are known and function satisfactorily in a variety of hosts (when utilized both in the same and different genera and species from where they were derived). The termination region usually is selected more as a matter of convenience rather than because of any particular property. Preferably, the termination region may be derived from a Saccharomyces gene, or from a Schizosaccharomyces, Candida, Yarrowia or Kluyveromyces gene. 3'-regions of mammalian genes encoding gamma-intererferon or alpha-2 interferon, which are also known to function in yeast, may alternatively be used. Termination control regions may also be derived from various genes native to the preferred hosts. Optionally, a termination site may be unnecessary; however, it is preferred if included.

In further preferred embodiment of the present invention, a Saccharomyces organism according to the present invention or obtainable according the method of the present invention as described herein above, may in addition to PUFAs as described herein above synthesizes at least one antioxidant. The term "antioxidant" refers to any molecule capable of slowing or preventing the oxidation of other molecules, as known to the person skilled in the art. More preferably, the term relates to any compound selected from the group consisting of ascorbic acid, glutathione, lipoic acid, uric acid, carotenes, α-tocopherol or ubiquinol. The term "at least" means that any one or any combination of compounds selected from the group consisting of ascorbic acid, glutathione, lipoic acid, uric acid, carotenes, α-tocopherol or ubiquinol is synthesized by a Saccharomyces organism according to the present invention. The term "synthesizes" means that a Saccharomyces organism according to the present invention produces an antioxidant as mentioned herein above, preferably by expressing enzymatic activities necessary for the generation of the antioxidant. Corresponding proteins and sequences are known to the person skilled in the art or derivable, e.g., from EP patent EP-B1 300 168. In a preferred embodiment the antioxidant synthesized by a Saccharomyces organism according to the present invention is glutathion.

The organism of the genus Saccharomyces according to the present invention or employed in a method according to the invention can be any species of Saccharomyces, in particular those known to the person skilled in the art, e.g. from taxonomy book or taxonomy information databases as provided by NCBI, NIH, USA. Preferably, the term "an organism of the genus Saccharomyces" comprises any of the species *Saccharomyces cerevisiae, Saccharomyces delbrückii, Saccharomyces italicus, Saccharomyces ellipsoideus, Saccharomyces fermentati, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum or Saccharomycodes ludwigii.*
More preferably, the organism of the genus Saccharomyces is of the species *Saccharomyces cerevisiae.*

In a preferred embodiment the organism belonging to the genus Saccharomyces is an organism in which one or more genes are deleted which code for enzymes using the same substrate as one of the above-described enzymes of a PUFA synthesis pathway and which would therefore reduce, by their activity, the flux of metabolites into the PUFA pathway. Thus, by deleting the genes encoding such enzymes the Saccharomyces organism is optimized with regard to its PUFA synthesis.
Genes which may be deleted in the Saccharomyces organism include genes encoding regulatory proteins such as OPI1 (negative regulator of phospholipid biosynthetic genes), genes coding for acetyltransferases, such as diacylglycerolacetyl transferase (DGAT), e.g. DGAT 1 and 2, and phospholipiddiacylglycerol acetyltransferase (PDAT).

Means and methods for deleting genes in the genome of organisms belonging to the genus Saccharomyces, in particular S. cerevisiae, are well-known to the person skilled in the art and are extensively described in the literature (see, e.g. Guthrie and Fink, "Guide to yeast genetics and molecular biology", Academic Press, 2002). The term "deleting" a gene also comprises approaches for inhibiting expression of the gene like gene disruption, RNAi approaches, cosuppression approaches, antisense approaches and the like.

In another preferred embodiment the organism belonging to the genus Saccharomyces is an organism which is further modified so as to lead to (over)expression of certain genes. Examples of such genes are genes coding for transcription factors being endogenous to Saccharomyces, such as HAP (transcriptional activator and global regulator of respiratory gene expression), INO2 (transcriptional activator) and INO4 (transcriptional activator). Other examples are genes which occur in organisms which naturally produce PUFAs, such as genes coding for acylCoA: lysophospholipid acyltransferase (LPLAT) which mediates the transport of acyl groups from the location of desaturation (sn2 position of phosphatidylcholine) to the location of elongation (CoA pool).
Further examples of genes which may be (over)expressed in the Saccharomyces organism are diacylglycerol acyltransferase (DGAT1), diacylglycerol acyltransferase (DGAT2), phospholipids:diacylglycerol acyltransferase (PDAT); glycerol-3-phosphate acyltransferase (GPAT), lysophosphatidic acid acyltransferase (LPAAT), fatty acid synthetase FAS1, fatty acid synthetase FAS2, phospholipid:diacylglycerol acyltransferase LRO1, phospholipaseD SPO14, cytochrome-b5 reductase MCR1 and cytochrome-b5 CYB5, phosphatidylinositol phosphatase Sac1.
In a further aspect the present invention relates to a method for synthesizing a polyunsatured fatty acid (PUFA), comprising culturing an organism of the genus Saccharomyces in accordance with the present invention, preferably as described herein above. The term "culturing" means the use of any suitable means and methods known to the person skilled in the art, which allows the growth of the organisms and the synthesis and/or accumulation of PUFAs.

The present invention also relates to a method for synthesizing a polyunsaturated fatty acid (PUFA) comprising steps (a) and (b) of the method according to the invention as described herein above and further comprising the step of culturing the organism resulting from step (b). The term "culturing" means the use of any suitable means and methods known to the person skilled in the art, which allows the growth of the organism and the synthesis and/or accumulation of PUFAs. The above described methods for synthesizing PUFAs may also additionally comprise the step of recovering the synthesized PUFAs from the cultured organism and/or the culture medium. Corresponding methods are known to the person skilled in the art.

In a preferred embodiment of a method for synthesizing a PUFA the method also comprises providing the organism additionally with PUFA precursors and/or intermediates.
The term "PUFA precursor" in this context means any intermediate of the PUFA synthesis pathway as present in the Saccharomyces organism (including oleic acid), such as oleic acid, linoleic acid, gamma-linoleic acid, dihomo-gamma-linoleic acid, arachidonic acid, alpha-linoleic acid, stearidonic acid, eicosatetraenoic acid, eicosapentaenoic acid and docosapentaenoic acid. The term "PUFA intermediate" in this context means any intermediate of the PUFA synthesis pathway as present in the Saccharomyces organism (including oleic acid), such as oleic acid, linoleic acid, gamma-linoleic acid, dihomo-gamma-linoleic acid, arachidonic acid, alpha-linoleic acid, stearidonic acid, eicosatetraenoic acid, eicosapentaenoic acid and docosapentaenoic acid.

In another preferred embodiment of the methods for synthesizing a PUFA the growth phase of the organism is independent of the synthesis of said PUFA. This means generally that a culture of the Saccharomyces organism is first grown without allowing the PUFA synthesis to occur (this can be achieved, e.g., by using regulatable/inducible promoters for expression of the corresponding enzymes), preferably until it reaches the stationary phase, and that then the production phase of PUFAs is initiated, e.g. by switching on the genes expressing the corresponding enzymes for the PUFA synthesis.

In a further preferred embodiment of the method for synthesizing a PUFA, the synthesis of the PUFA is carried out within a certain temperature range, e.g., between 15°C and 45°C, preferably between 20°C and 40°C or 15°C and 30°C, more preferably between 20°C and 30°C.
In another preferred embodiment the synthesis of the PUFA is carried out in a broad pH range, e.g., between pH 6 and pH 9, preferably between pH 6.5 and 8.5.
In a further preferred embodiment the synthesis of the PUFA is carried out under conditions of nitrogen and/or phosphate and/or inositol limitation. Such limitations may lead to an increase in the total lipid content. A limitation of inositol preferably means that the culture medium contains less than 1%, even more preferred 0% inositol. Nitrogen limitation means that the C/N ration is 15 or higher.

In a further preferred embodiment of the method for synthesizing a PUFA the method is a continuous process. Methods for carrying out continuous fermentation processes are well known to the person skilled in the art and are described in the literature (see, e.g., Chmiel, H., "Bioprozesstechnik", Spektrum Akademischer Verlag, 2005).
In another preferred embodiment of the method for synthesizing a PUFA the method also comprises the step of separating the synthesized PUFA from the cultured organism by (a) perforating the cell wall of the organism, e.g., by electroporation and (b) mechanically separating an oil fraction from the cell walls and cell plasma aqueous fractions.

The present invention also relates to an edible product containing PUFAs from at least one Saccharomyces organism according to the present invention. The product may be any edible product suitable as feed for animal or as a human food product, as a dietary supplement or a medical preparation.
Examples are any kinds of bakery products, in particular those containing bakery yeast, tablets, and food products for babies and children.

### Figure 1: provides an overview of the PUFA synthetic pathway

### Examples

### Materials

### Strains

Saccharomyces cerevisiae AH22 [MATa leu2-3 leu2-112 his4-519 can1 ura3Δ] (Polakowski (1998), "Molekularbiologische Beeinflussung des Ergosterolstoffwechsels der Hefe Saccharomyces cerevisiae"; PhD thesis at the Technische Universität Berlin, Germany)

*Saccharomyces cerevisiae GRF18 [MATα leu2-3 leu2-112 his3-11 his3-15 can1 ura3*Δ*]*
Various *Saccharomyces cerevisiae* deletion strains

### Media

- WMVIII:: (Lang and Looman, Appl. Micorobiol. Biotechnol. 44 (1995), 147-156) For 1 liter of medium: 50 g sucrose; 250 mg NH₄H₂PO₄; 2.8 NH₄Cl; 250 mg MgCl₂ x 6H₂O; 100 mg CaCl₂ x 2H₂O; 2 g KH₂PO₄; 550 mg MgSO₄ x 7H₂O; 75 mg meso-Inositol; 10 g Na glutamate; 1 ml 1000 x trace elements-solution; 4 ml 250 x vitamine solution;
- Trace-elements:: 1000x concentrated: 1.75 g ZnSO₄ x 7H₂O; 0.5 g FeSO₄ x 7 H₂O; 0.1 g CuSO₄x 5 H₂O; 0.1 g MnCl₂ x 4 H₂O; 0.1 g NaMoO₄ x 2 H₂O for 1 liter
- Vitamine solution:: 250x concentrated: 2.5 g nicotinic acid; 6.25 g pyridoxine; 2.5 g thiamine; 0.625 g biotine; 12.5 g Ca pantothenate for 1 liter.
- Supplements:: leucine (0.4 g/l); histidine HCl (strain-dependent 20 mg/l - 1 g/l); uracil (0.1 g/l); haemin (Sigma, München) (0.8 mg/ml). FeSO₄ 5 mg/l or 50 mg/l; 5-FOA (5-fluoroorotic acid) 1g/l.
- YE-Medium:: 0.5% Yeast extract; 2% glucose; pH 6.3
- YNB-Medium:: 0.67 % YNB ("yeast nitrogen base", Difco, Augsburg); 2% glucose

### Example 1: Mutagenesis of S.cerevisiae

To establish the synthesis of significant amounts of PUFAs in the yeast *Saccharomyces cerevisiae,* the total lipid content of the yeast has to be increased. Therefore, the yeast cells are treated with ethyl-methane-sulfonate (EMS). EMS is an DNA-alkylating reagent, which causes mutations. The treatment with this mutagene causes an enhanced lipid content in some cells. These cells are separated and provide a basis for further genetic manipulations. In addition to the application of EMS, the cells are partly treated with Cerulenin. Cerulenin is an antifungal antibiotic, which inhibits the biosynthesis of sterols and fatty acids. The application of this reagent allows the selection of yeast cells, which have an enhanced fatty acids biosynthetic system. After the mutagenesis, the cells are further separated by means of their lipid content. This is achieved by the centrifugation of the cells in a Percoll density gradient. The spontaneously developing density gradient causes the separation of the cells according to their lipid content, because the lipid content of the cells has an influence on their density. The selected yeast cells with an enhanced lipid content provide the basis for the further transformation of the cells with genes for the PUFA synthetic pathway.

### Mutagenesis

The mutagenesis was carried out as follows:
- Preculturing of yeast cells in 20ml of WMVIII Medium, 3 days, 28°C
- For the main culture, 50ml of WMVIII Medium were inoculated with 50µl of the preculture; incubation for 18h in 250µl shake flasks at 28°C
- 1x10⁸ cells are harvested: 8min, 5000 rpm in 15ml Greiner-tubes
- Cells are resuspended in 400µl of 0.1M Na₂HPO₄ buffer (pH 7)
- Addition of 10µl ethyl methane sulfonate (EMS); vortexing; incubation at 24°C for 1 hour
- Addition of 4.6 ml of 5% sodium thiosulfate solution
- Harvesting at 5000rpm, 8min
- Resuspension in 1ml of 8.5% Sucrose-solution
- Inoculation of 50ml of WMVIII liquid medium containing 2µM Cerulenin with 200µl of cell-containing sucrose solution; cultivation at 28°C for 96h
- Harvesting of 1 x 10⁸ cells (5000 rpm, 8min) and resuspension in 1ml of Percoll solution
- Centrifugation at 30000 x g for 1h
- For further experiments, cells from the top-layer of the gradient are collected (Selection of cells with highest lipid-content)
   The lipid content of the cells is determined with GC/MS as described further below.

### Example 2: Genetic manipulation of S.cerevisiae

Since the yeast *S.cerevisiae* has only the ability to synthesize oleic acid as the sole unsaturated fatty acid, the relevant enzymatic steps for synthesis of PUFAs have to be identified. Therefore the biosynthetic pathways of organisms are examined, which have the ability to synthesize PUFAs naturally. These are e.g. algae, fish, mammals or plant. Figure 1 exhibits a general overview of the PUFA-synthesis starting with oleic acid. The selection of essential enzymatic reactions for the synthesis of PUFAs is based on figure 1. The corresponding gene sequences are selected from a gene database. The genes are chosen with regard to a good expression in yeast *S.cerevisiae.* The selected genes are displayed in Table 1.

**Table 1: Selected genes for PUFA-synthesis**

| Gene | Origin | Reaction | Accession No. |
|---|---|---|---|
| Δ12 desaturase | Pichia pastoris | 18:1 → 18:2 ω6 | AY863218 |
| Δ6 desaturase | Ostreococcus tauri | 18:2 ω6 → 18:3 w6 ; 18:3 ω3 → 18:4 ω3 | AY746357 |
| Δ5 desaturase | Mortirella alpina | 20:3 ω6 → 20:4 ω6 ; 20:4 ω3 → 20:5 w3 | AF067654 |
| Δ17 desaturase | Mortirella alpina | 20:3 ω6 → 20:4 ω3 ; 20:4 ω6 → 20:5 w3 | AB182163 |
| Δ4 desaturase | Thraustochytrium sp. | 22:5 ω3 → 22:6 w3 | AF489589 |
| Δ15 desaturase | Pichia pastoris | 18:2 ω6 → 18:3 ω3 | EF116884 |
| C_{18/20} elongase | Caenorhabditis elegans | 18:3 ω6 → 20:3 w6 ; 18:4 ω3 → 20:4 w3 | BD250026 |
| C_{20/22} elongase | Scophthalmus maximus | 20:5 ω3 → 22:5 w3 | AF465520 |

Besides the genes displayed in Table 1, further genes from various organisms could be used instead (Sequences not shown). For the transformation of S.cerevisiae, the pFlat-plasmids are chosen. The application of these plasmids provides the possibility of using 4 different markers for selection. The plasmids can be expressed separately or together in yeast. A construct based on 2 genes, a promoter sequence and a terminator is integrated into each plasmid before transformation. The selection of inducible promoters or the introduction of regulatory elements offers the possibility of producing a certain lipid pattern in the host strain. A suitable promoter is the constitutive *ADH1*-promotor. The designed plasmids including the PUFA-genes are shown in Table 2.

**Table 2:Designed plasmids for yeast transformation**

| Plasmid | Marker | Insert |
|---|---|---|
| pFlat1 | URA3 | Δ12 desaturase, Δ6 desaturase |
| pFlat2 | KanMX (G418) | C_{20/22} elongase, Δ15 desaturase |
| pFlat3 | LEU2 | C_{18/20} elongase, Δ5 desaturase |
| pFlat4 | HIS3 | Δ4 desaturase, Δ17 desaturase |

After the construction of the plasmids, the yeast is transformed. The selection of the plasmids provides the opportunity to synthesize different fatty acids. For the synthesis of e.g. DHA, the yeast has to be transformed with all 4 plasmids, since all genes are necessary for production of this fatty acid. If the yeast is transformed only with plasmid 1, resulting in the expressing of delta12-desaturase and delta 6-desaturase, γ-linolenic acid will be synthesized. For the synthesis of arachidonic acid, plasmids pFlat1 and 3 should be introduced into the yeast. Besides wildtype strains, deletion strains will also be used as hosts for the transformation. These deletion strains are optimized with regard to their PUFA synthesis, e.g. genes might be deleted, which use the same substrate as the PUFA-genes, thereby causing reduced flux into the PUFA-pathway.

### Transformation of S.cerevisiae

A preculture of *S.cerevisiae* cells was cultivated for 48 h in 100 ml flasks containing 20 ml of YE-Medium. The main culture was inoculated with the preculture to an OD₆₀₀ of 0.3 and cultivated for 4 h, resulting in an OD₆₀₀ of 0.8-1.0. Then the cells were harvested by centrifugation at 4000 x g for 5 minutes and washed subsequently with 10 ml of H₂O. Afterwards, the cells were resuspended in 1.5 ml of H₂O and transferred to an 1.5 ml Eppendorf tube. After another centrifugation step (4000 x g, 2 min), 1.5 ml of a lithium-acetate solution (100 mM LiAc in 1x TE-buffer) were added. Then the mixture was centrifuged again at 4000 x g for 2 min. The pellet was resuspended in 200 µl lithium-acetate solution. The cells were thereby made competent. Each transformation preparation consisted of 50 µl of competent cells, 1-10 µg plasmid-DNA, 10 µl of herringsperm-DNA (10 mg/ml) and 230 µl PEG-solution (40% PEG 4000 in 0.1 M lithium-acetate in 1x TE-buffer, pH 8.0). The preparation was firstly incubated at 30°C for 30 min and then for 8 min at 42°C. After addition of 800 µl H₂O, a centrifugation at 10000 x g for 20 sec followed. The cells were finally plated on the corresponding selective-medium, and incubated for 3 to 5 days at 28°C. If a dominant marker like G418 is used, a 24h period for regeneration is necessary. After treatment at 42°C, cells are harvested (2min, 5000rpm) and resuspended in 3-4 ml of medium. Then the cells are plated on selective media and cultivated for 3-5 days at 28°C.

### Example 3: Analytics

### Cultivation of of S.cerevisiae for analysis of lipids

For the analysis of lipids, 20 ml WMVIII-medium plus supplements (Lang and Loomann, 1995; loc. cit) were inoculated with 200 µl of a freeze culture or directly from an agar plate. After two days at 28°C on a shaker spinning at 160 rpm, 50 ml WMVIII medium in baffled-flasks was inoculated with 1% of the preculture and grown for 3 days at 20°C. In the case of auxotrophic clones, the medium was supplemented with 0.1 g/l of uracil, 0.02 - 1 g/l of histidine, 0.4 g/l of leucine and/or 0.3 g/l geneticine. If necessary, the content of nitrogen, phosphate and/or inositol is altered to enhance the lipid content of the yeast.

### Biomass dry weight determination

The cell dry weight is determined by harvesting 3 x 6 ml of culture after the desired cultivation time. The culture is centrifuged at 3500 x g for 5min. Afterwards, the cells are washed once with H₂O and the pellet is weighted by a scale after vacuum-drying for 12h at 80°C in a cabinet desiccator and cooling to room temperature in a dehydrator.

### Sample preparation for the Lipid Analysis

For the analysis of lipids, 3 x 30 OD₆₀₀ of cells are harvested by centrifugation at 3500 x g for 5min (triplicate analysis). The yeast pellet is resuspended in 2ml of a 3 N HCL and incubated at 100°C for 20min. Then, the cells are cooled on ice and 4ml of methanol are added. The samples are incubated at 85°C for 1 hour for derivatization. After cooling to room temperature, the fatty acid methyl esters are extracted with 2 x 20ml of n-hexane. The 2 n-hexane-layers are combined and concentrated in a rotary evaporator at 40°C. Subsequently, the residue is resuspended in 2 x 1ml off n-hexane and pooled. The extracts are vortexed and incubated for 5min at room temperature. To ensure a complete derivatization of fatty acids, TMSH (Trimethylsulfoniumhydroxid) is applied. Therefore, 450µl of extract are combined with 50µl of TMSH. After 15min of incubation, the analysis is performed using a gas chromatograph coupled to a mass selective detector (GC/MS). The GC is an Agilent 6890N VL MSD with a 5975B quadruple mass selective detector. The column used, is an Agilent DB23 (60m 0.25µm film thickness). The MS is operated in Scan-modus. The initial oven temperature is 130°C, rising at 6.5°C/min to 170°C, rising 2.75°C/min to 215°C, holding 215°C for 12min, rising at 40°C/min to 250°C and holding this final temperature for 8min. The flow through the column is 1.1 ml /min of helium. Injection volume was 1µl using a Agilent 7683B Series injector. The injector is driven at splitless modus. The temperature of the inlet is 270°C and 150°C of the MS-quadruple. Detected fatty acid methylesters are confirmed using mass spectral database, and retention times are compared to standard fatty acid methyl esters. The amount of fatty acids in unknown samples is calculated by comparing responses of mass detector with fatty acid methyl ester standards.

The amount of fatty acids per dry weight is determined by relating the data from GC/MS-analysis to data from dry weight determination.

## Claims

1. A method of providing an organism belonging to the genus Saccharomyces, which is capable of producing polyunsaturated fatty acids (PUFAs) comprising the steps of:
(a) subjecting a Saccharomyces organism, which does not produce PUFAs, to a mutagenesis step and selecting a Saccharomyces organism, which is capable of accumulating at least 15% of the cellular dry weight as lipids; and
(b) optionally genetically modifying the Saccharomyces organism selected in step (a) so that it is capable of synthesizing PUFAs.

2. An organism belonging to the genus Saccharomyces, which is capable of accumulating at least 15% of the cellular dry weight as lipids, wherein said organism is genetically modified so that polyunsaturated fatty acids (PUFAs) are synthesized.

3. The method of claim 1 or the organism of claim 2, wherein said genetic modification results in the expression of enzymes of the omega-3/omega-6 fatty acid biosynthetic pathway.

4. The method or organism of claim 3, wherein said genetic modification is a transformation of the organism with genes encoding enzymes of the omega-3/omega-6 fatty acid biosynthetic pathway.

5. The method of any one of claims 1 to 4 or the organism of any one of claims 2 to 4, wherein said polyunsaturated fatty acid is any one of, or any combination of: linoleic acid, conjugated linoleic acid, gamma-linolenic acid, dihomo-gamma-linolenic acid, arachidonic acid, alpha-linolenic acid, stearidonic acid, eicosatetraenoic acid, eicosapentaenoic acid, docosapentaenoic acid or docosahexaenoic acid.

6. The method or organism of claim 5, wherein conjugated linoleic acid is synthesized by the expression of the enzymes Δ12 desaturase and isomerase.

7. The method or organism of claim 5, wherein arachidonic acid is synthesized by the expression of the enzymes Δ12 desaturase, Δ6 desaturase, Δ5 desaturase, and C_{18/20} elongase

8. The method or organism of claim 5, wherein eicosapentaenoic acid is synthesized by the expression of the enzymes Δ12 desaturase, Δ6 desaturase, Δ5 desaturase and C_{18/20} elongase, and further Δ15 desaturase and/or Δ17 desaturase.

9. The method or organism of claim 5, wherein docosapentaenoic acid is synthesized by the expression of the enzymes Δ12 desaturase, Δ6 desaturase, Δ5 desaturase, C_{18/20} elongase and C_{20/22} elongase, and further Δ15 desaturase and/or Δ17 desaturase.

10. The method or organism of claim 5, wherein docosahexaenoic acid is synthesized by the expression of the enzymes Δ12 desaturase, Δ 6 desaturase, Δ5 desaturase, Δ4 desaturase, C_{18/20} elongase and C_{20/22} elongase, and further Δ15 desaturase and/or Δ17 desaturase.

11. The method or organism of claim 5, wherein eicosapentaenoic acid and docosahexaenoic acid are synthesized by the expression of the enzymes Δ12 desaturase, Δ6 desaturase, Δ5 desaturase, Δ4 desaturase, C_{18/20} elongase and C_{20/22} elongase, and further Δ15 desaturase and/or Δ17 desaturase.

12. The method or organism of claim 11, wherein eicosapentaenoic acid and docosahexaenoic acid are synthesized in a ratio of approximately 40 to 60.

13. The method of any one of claims 1 to 12 or the organism of any one of claims 2 to 11, wherein said expression of enzymes of the omega-3/omega-6 fatty acid biosynthetic pathway is based on genetic elements integrated in the genome of said organism or present on plasmids.

14. The method or organism of claim 13, wherein said expression can be regulated.

15. The method of any one of claims 1 to 14 or the organism of any one of claims 2 to 13, wherein said organism further synthesizes at least one antioxidant.

16. The method or organism of claim 15, wherein said antioxidant is glutathione.

17. The method of any one of claims 1 to 16 or the organism of any one of claims 2 to 15, wherein said organism is of the species *Saccharomyces cerevisiae, Saccharomyces delbrückii, Saccharomyces italicus, Saccharomyces ellipsoideus, Saccharomyces fermentati, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum or Saccharomycodes ludwigii.*

18. A method for synthesizing a polyunsaturated fatty acid (PUFA), comprising culturing the organism of any one of claims 2 to 17.

19. A method for synthesizing a polyunsaturated fatty acid (PUFA) comprising the steps of (a) and (b) of the method of any one of claims 1 to 17 and further comprising the step of cultivating the organism resulting from step (b).

20. The method of claim 18 or 19, wherein said organism is additionally provided with PUFA precursors or intermediates.

21. The method of claim 20, wherein said PUFA precursors or intermediates are selected from the group comprising oleic acid, linoleic acid, gamma-linoleic acid, dihomo-gamma-linoleic acid, arachidonic acid, alpha-linoleic acid, stearidonic acid, eicosatetraenoic acid, eicosapentaenoic acid and docosapentaenoic acid.

22. The method of any one of claims 18 to 21, wherein the growth phase of the organism is independent of the synthesis of said PUFA.

23. The method of any one of claims 18 to 22, wherein said synthesis of a PUFA is carried out within a temperature range of 15-45°C, a pH range of 6 - 9 and/or under conditions of nitrogen and/or phospate and/or inositol limitation.

24. The method of any one of claims 18 to 23, wherein said method is a continuous synthesis process.

25. The method of any of claims 18 to 24, wherein said method additionally comprises the step of separating said PUFA from said organism by (a) perforating the cell wall of said organism by electroporation and (b) mechanically separating an oil fraction from cell walls and cell plasma aqueous fractions.

26. An edible product containing PUFAs from at least one organism as defined in any one of claims 1 to 17.

27. The edible product of claim 26, wherein said product is suitable for consumption as a human food product.

28. The edible product of claim 26, wherein said product is suitable for consumption as an animal food product, a dietary supplement, or a medical preparation.
